# EUROPEAN PATENT SPECIFICATION

(11) **EP 1 996 217 B1**
(45) Date of publication and mention of the grant of the patent: **10.11.2010**
(21) Application number: 07752701.8
(22) Date of filing: 09.03.2007
(51) Int. Cl.: A61K 38/16

(54) **NOVEL ANTIBIOTIC COMPOSITIONS**
NEUARTIGE ANTIBIOTISCHE ZUSAMMENSETZUNGEN
NOUVELLES PRÉPARATIONS D'ANTIBIOTIQUES

(30) Priority: 09.03.2006 US 780782 P
(43) Date of publication of application: 03.12.2008
(73) Proprietor: Cambridge Enterprise Limited, Trinity Lane Cambridge Cambridgeshire CB2 1TN (GB)
(72) Inventor: COLEMAN, John, Richmond, British Columbia V6V 2M2 (CA); HAN, Kang, Richmond, British Columbia V6V 2M2 (CA); COOPER, Matthew Allister, Cambridge, CB1 3PT (GB)
(74) Representative: Sutcliffe, Nicholas Robert
(86) International application number: PCT/US2007/006019
(87) International publication number: WO 2007/103548

(56) References cited:
- US-A- 5 910 300
- US-A- 6 153 405
- US-A1- 2004 106 544
- CHAKICHERLA ANU ET AL: "Role of the leader and structural regions of prelantibiotic peptides as assessed by expressing nisin-subtilin chimeras in Bacillus subtilis 168, and characterization of their physical, chemical, and antimicrobial properties" JOURNAL OF BIOLOGICAL CHEMISTRY, vol. 270, no. 40, 1995, pages 23533-23539, XP002534929 ISSN: 0021-9258
- WALSH E M ET AL: "Use of antibiotics to inhibit non-starter lactic acid bacteria in cheddar cheese" INTERNATIONAL DAIRY JOURNAL, vol. 6, no. 4, 1996, pages 425-431, XP002534968 ISSN: 0958-6946
- HSU ET AL.: 'The nisin-lipid II complex reveals a pyrophosphate cage that provides a blueprint for novel antibiotics' NATURE STRUCTURAL AND MOLECULAR vol. 11, no. 10, September 2004, pages 963 - 967
- BONEV ET AL.: 'Targeting extracellular pyrophosphates underpins the high selectivity of nisin' FASEB J. vol. 18, no. 15, December 2004, pages 1862 - 1869

## Description

### FIELD OF THE INVENTION

This invention relates to antibiotic compositions with anti-bacterial activity and methods and intermediates for their production. The invention further concerns the use of such antibiotic compositions for the treatment of bacterial infections in mammals, including humans.

### BACKGROUND OF THE INVENTION

Diseases caused by bacterial infections have significant morbidity and mortality in humans and other mammals. The infection process consists of three stages: bacterial entry and colonization of the host; bacterial invasion and growth in host tissues along with.the appearance of toxic substances; and the host response.

Bacterial infections can be classified broadly into those caused by Gram positive bacteria, such as the *Staphylococci* and *Streptococci,* and those caused by Gram negative bacteria, such as *Escherichia coli.* Gram positive bacteria have a typical lipid bilayer cytoplasmic membrane surrounded by a rigid cell wall. The cell wall is composed mainly of peptidoglycan, a polymer of N-acetylglucosamine and N-acetyl muramic acid crosslinked by a pentapeptide comprising alternating D- and L- amino acids. In addition, the outer cell wall of Gram-positive bacteria comprises a complex of polysaccharides, proteins, teichoic acids, and lipoteichoic acids. By contrast, Gram-negative bacteria have a much smaller peptidoglycan layer, an outer membrane that contains lipopolysaccharide which lacks the complex layer of carbohydrate and teichoic acids.

Antibiotics are substances produced by various species of microorganisms (bacteria, fungi) that suppress the growth of other microorganisms and may eventually destroy them. In addition, common usage extends the term antibiotic to include antibacterial agents which are not products of microbes, such as synthetic antibacterial agents (*e.g*., sulphonamides) and various peptides found in host defense systems which are produced locally in response to colonization by or invasion of microorganisms (*e.g*., magainin). Hundreds of antibiotics have been identified, and many have been developed to a point where they are of value in the therapy of infectious diseases.

Several schemes have been proposed to classify and group antimicrobial agents. The most common classification has been based on chemical structure and proposed mechanism of action, as follows: (1) agents that act directly on the cell membrane of the microorganism, affecting permeability and leading to leakage of the intracellular compounds, such as detergents, cationic peptides, gramicidin A, and polymyxin; (2) agents that inhibit synthesis of bacterial cell walls, including the beta-lactams and glycopeptides; (3) agents that affect bacterial protein synthesis, including tetracycline and chloramphenicol; (4) agents that act as antimetabolites and interfere with the bacterial synthesis of folic acid, such as the sulphonamides; and (5) agents that inhibit nucleic acid synthesis or activity, such as quinolones.

Anti-bacterial agents which act directly on the bacterial membrane cause a general permeabilization or modification of the bacterial cytoplasmic membrane. This results from the binding of peptides to components of the outer membrane surface, causing reorganization of membrane structure and the creation of pores through which the intracellular contents may leak. Generally, these features are associated with a peptide that is amphiphilic in nature, often including helical secondary structure and a net positive charge. Peptide antibiotics having broadly this mode of action include the magainins, defensins, and lantibiotics. The activity of this class of antibiotics is directed towards bacteria rather than mammalian cells because the positive charged residues of the antibiotic interact with negatively charged lipids which are found predominantly in bacterial rather than mammalian cell membranes.

A further group of antibiotics that has received widespread attention due to their clinical efficacy is the glycopeptide group of antibiotics. These agents consist of a rigid, cyclized heptapeptide backbone which may be substituted with a variety of amino and non-amino sugars. The amino sugar moieties of some members of this class contain *N*-acyl, *N*-alkyl, or *N*-aryl substitutions. Two antibiotics in this class are vancomycin and teicoplanin.

Resistance to antibiotics is well documented and the resistant strains are a potential major threat to the well-being of humankind (*e.g*., D'Costa et al., 2006 *Science* 311:374). Bacteria often become resistant to an antimicrobial agent because the drug fails to reach its target; for instance, the drug may be inactivated, and/or the target may be structurally altered and/or altered in its availability or accessibility to the drug. For example, some bacteria produce enzymes that reside in or within the cell surface and inactivate the drug, while others possess impermeable cell membranes that prevent influx of the drug.

The emergence and dissemination of high-level resistance to members of the glycopeptide group of antibiotics in *enterococci* in the past decade has resulted in clinical isolates resistant to all antibiotics of proven efficacy. The incidence of glycopeptide resistance among clinical isolates is increasing and *enterococci* have become important as nosocomial pathogens and as a reservoir of resistance genes. Nosocomial infections with multidrug resistant strains are potentially catastrophic and there is a need to identify novel anti-bacterial agents or methods of controlling bacterial infections. Methicillin-resistant *staphylococcus aureus* (MRSA) is now a serious nosocomial pathogen, and vancomycin is used in treatment of MRSA infections often at relatively high doses. The MRSA problem has prompted development of a number of novel antibiotics including vancomycin analogues.

Approaches that have been used to combat the emergence of antibiotic resistant strains include the modification of existing antibiotics to improve their potency against resistant organisms, and the discovery of new peptide antibiotics which kill their targets by permeabilizing the bacterial plasma membrane. Examples of the first approach have recently focussed on creating derivatives of glycopeptides such as vancomycin. Functionalization of the carboxyl terminal of vancomycin using the coupling agent 2-(1-hydroxybenzotriazol-1-yl)-1,1,3,3-tetramethyluronium hexafluorophosphate (HBTU) has been successful in attaching short peptide sequences, both in solution and solid phases. The amino sugar and terminal amine moieties of Vancomycin and related antibiotics have also been derivatized. In a reductive alkylation approach, a series of compounds alkylated on the vancosamine sugar was created, some of which showed greatly improved activity when compared to vancomycin against vancomycin resistant bacterial strains.

U.S. Patent No. 6,713,606 describes polypeptide derivatives in which a therapeutic soluble protein or peptide is modified with an entity of the following general structure:

-{L-[W]}ₙ-X (1)

in which each **L** is independently a flexible linker group, each **W** is independently a peptidic membrane-binding element, n is an integer greater than or equal to one, and **X** is a peptidic or non-peptidic membrane-binding or insertive element.

Structures of type **(1)** represent a combinatorial array of membrane-interactive elements whose attachment to soluble polypeptides was found to mediate binding of those polypeptides to the outer cell membrane of mammalian cells. This approach gives rise to therapeutic benefits, particularly in the case of regulators of complement activation acting as cytoprotectants and anti-inflammatory agents. It was subsequently hypothesized that structures of type **(1)** would display stronger binding to bacterial membranes, which have a higher proportion of acidic phospholipids than do eukaryotic organisms, and which have a higher proportion of membrane-associated proteins. In agreement with this hypothesis, it was shown in U.S. Patent Application Publication No. 2004/0106544 that agents comprising antibacterial peptides, pertaining to the general structure (1) and derivatized further with the glycopeptide antibiotic Vancomycin, had antibacterial activities greater than those displayed by either component alone.

According to such agents as described in U.S. Publication No. 2004/0106544, however, certain such anti-bacterial peptides conferred enhanced anti-bacterial activity by physically inserting into the bacterial membrane and disrupting it, thus requiring the anti-bacterial peptide to have both (i) a functionally active membrane-binding domain and (ii) a functionally active membrane-inserting domain. Consequently, these anti-bacterial agents necessarily involve a relatively large and conformationally correct anti-bacterial peptide, with attendant challenges presenting themselves in the contexts of time, cost and efficiency of synthesis of such agents, including assurance of proper folding. Clearly it would be desirable to avoid such problems, if antibiotic compositions could be designed using smaller and therefore more efficient anti-bacterial targeting moieties. As described herein, the present invention fulfills this need and offers other related advantages.

The production and expression of nisin-subtilin chimeras is described in Chakicheria et al J. Biol. Chem (1995) 270 23533-23539 and US6153606 and US6153405.

Walsh et al (1996) Int. Dairy J. 6 425-431 describe the use of nisin to inhibit non-starter lactic acid bacteria in Cheddar cheese.

### RELATED REFERENCES

The following references provide additional background to the invention:
U.S. Published Patent Applicaition No. 20040106544 (Cooper et al.);
U.S. Patent No. 6,713,606 (Smith et al.);
U.S. Patent No. 6,794,181 (Coughlin et al.);
McCormick, M.H. et al., "Vancomycin, a new antibiotic. I. Chemical and biologic properties", Antibiot. Annu.1955; 3:606-11;
Sheldrick, G.M. et al., "Structure of vancomycin and its complex with acetyl-D-alanyl-D-alanine", Nature 1978; 271(5642):223-5;
Dutka-Malen, S. et al., "The VANA glycopeptide resistance protein is related to D-alanyl-D-alanine ligase cell wall biosynthesis enzymes", Mol. Gen. Genet. 1990; 224(3):364-72;
Murray, B.E., "The life and times of the Enterococcus" Clin. Microbiol. Rev. 1990; 3(1):46-65;
Bugg, T.D. et al., "Identification of vancomycin resistance protein VanA as a D-alanine: D-alanine ligase of altered substrate specificity" Biochemistry 1991; 30(8):2017-21;
Thuault, D. et al., "Inhibition of Clostridium tyrobutyricum by bacteriocin-like substances produced by lactic acid bacteria." J. Dairy Sci. 1991; 74(4):1145-50;
Handwerger, S. et al., "Concomitant high-level vancomycin and penicillin resistance in clinical isolates of enterococci" Clin. Infect. Dis. 1992; 14(3):655-61;
Arthur, M. et al., "Characterization of Tn1546, a Tn3-related transposon conferring glycopeptide resistance by synthesis of depsipeptide peptidoglycan precursors in Enterococcus faecium BM4147", J. Bacteriol. 1993; 175(1):117-27;
Handwerger, S. et al., "Nosocomial outbreak due to Enterococcus faecium highly resistant to vancomycin, penicillin, and gentamicin", Clin. Infect. Dis. 1993; 16(6):750-5;
Billot-Klein, D. et al., "Association constants for the binding of vancomycin and teicoplanin to N-acetyl-D-alanyl-D-alanine and N-acetyl-D-alanyl-D-serine" Biochem. J. 1994;.304 ( Pt 3):1021-2;
Reynolds, P.E. et al., "Analysis of peptidoglycan precursors in vancomycin-resistant Enterococcus gallinarum BM4174", Biochem. J. 1994; 301 ( Pt 1):5-8;
Sundram,U.N. et al., "General and Efficient Method for the Solution- and Solid-Phase Synthesis of Vancomycin Carboxamide Derivatives", J. Org. Chem. 1995; 60(5):1102-1103;
Woodford, N. et al., "Linkage of vancomycin and high-level gentamicin resistance genes on the same plasmid in a clinical isolate of Enterococcus faecalis" J. Antimicrob. Chemother. 1995; 35(1):179-84;
Arthur, M. et al., "Quantitative analysis of the metabolism of soluble cytoplasmic peptidoglycan precursors of glycopeptide-resistant enterococci", Mol. Microbiol. 1996; 21(1):33-44;
Chan, W.C. et al., " Structure-activity relationships in the peptide antibiotic nisin: antibacterial activity of fragments of nisin", FEBS Lett. 1996; 390(2):129-32;
Cooper, R.D. et al., "Reductive alkylation of glycopeptide antibiotics: synthesis and antibacterial activity", J. Antibiot. (Tokyo) 1996; 49(6):575-81;
Evers, S. et al., "Regulation of VanB-type vancomycin resistance gene expression by the VanS(B)-VanR (B) two-component regulatory system in Enterococcus faecalis V583", J. Bacteriol. 1996; 178(5):1302-9;
Rodriguez, M.J. et al., "Snyder NJ, Zweifel MJ, et al. Novel glycopeptide antibiotics: N-alkylated derivatives active against vancomycin-resistant enterococci", J. Antibiot. (Tokyo) 1998; 51(6):560-9;
van Kraaij, C. et al., "Pore formation by nisin involves translocation of its C-terminal part across the membrane", Biochemistry 1998; 37(46):16033-40;
Linton, S.M. et al., "Therapeutic efficacy of a novel membrane-targeted complement regulator in antigen-induced arthritis in the rat", Arthritis Rheum. 2000; 43(11):2590-7;
Smith, R.A., "Targeting anticomplement agents" Biochem. Soc. Trans. 2002; 30(Pt 6):1037-41;
Bonev, B.B. et al., "Targeting extracellular pyrophosphates underpins the high selectivity of nisin", FASEB. J. 2004; 18(15):1862-9.

### SUMMARY OF THE INVENTION

This invention is directed to antibiotic compositions comprising an antibiotic moiety covalently linked to a targeting moiety. These antibiotic compositions use a distinct mode of interaction with bacterial membranes and have antibacterial activities unexpectedly greater than those displayed by each component alone.

Thus, in one aspect, this invention is directed to antibiotic compositions as set out in claim 1. An antibiotic composition may be comprised of the following structure:

**T-L-P**

wherein:
T is a targeting moiety comprising a nisin[1-12] polypeptide fragment having a lanthionine ring structure and a β-methyllanthionine ring structure:
L is a linker moiety selected from the group consisting of (i) a direct covalent bond linkage between T and P and (ii) a linker molecule covalently bonded to T and P; and
P is an antibiotic moiety that is capable of altering at least one activity of a bacterial membrane or a bacterial cell wall.

An antibiotic composition may be comprised of the following structure:
wherein:
T is a targeting moiety comprising a polypeptide homologue of a nisin[1-12] polypeptide fragment, said polypeptide homologue having a lanthionine ring structure and a β-methyllanthionine ring structure and being selected from a subtilin[1-12] polypeptide fragment, an epidermin polypeptide fragment, a (I1V 16L) epidermin polypeptide fragment, a mutacin B-Ny266 polypeptide fragment and a mutacin 1140 polypeptide fragment;
L Is a linker moiety selected from the group consisting of (i) a direct covalent bond linkage between T and P and (ii) a linker molecule covalently bonded to T and P; and
P is an antibiotic moiety that is capable of altering at least one activity of a bacterial membrane or a bacterial cell wall;
or pharmaceutically acceptable salts thereof.

An antibiotic composition may be comprised of the following structure:

**T-L-P**

wherein:
(a) T is a targeting moiety comprising an N-terminal peptide fragment of a lantibiotic where the peptide fragment comprises the following formula:
   X₁X₂A₁X₃X₄X₅A₂A₃PGA₄X₆(SEQ ID Nos. 1-4)
   wherein
   X₁ is selected from F, I, V and W,
   X₂ is selected from A, K, α-aminobutyric acid and dehydrobutyrine,
   A₁, A₂ and A₄ are alanine and A₃ is α-aminobutyric acid,
   X₃ is selected from E, I, K and W,
   X₄ is selected from F, alanine and dehydroalanine,
   X₅ is selected from I, F and L,
   X₆ is selected from A, K, V, AX₇, KX₇ and VX₇, wherein X₇ is selected from A, G, I, L, M, P and V;
   and wherein A₁ and A₂ together form a lanthionine linkage and A₃ and A₄ together form a β-methyllanthionine linkage;
(b) L is a linker moiety selected from the group consisting of (i) a direct covalent bond linkage between T and P and (ii) a linker molecule covalently bonded to T and P; and
(c) P is an antibiotic moiety that is capable of altering at least one activity of a bacterial membrane or a bacterial cell wall;
or pharmaceutically acceptable salts thereof.

An antibiotic composition may be comprised of the following structure:

**T-L-P**

wherein:
(a) T is a targeting moiety comprising a N-terminal peptide fragment of a lantibiotic where the peptide fragment comprises the following formula:
   X₁X₂A₁X₃X₄X₅A₂A₃PGA₄X₆ (SEQ ID NO:5)
   wherein
   X₁ is selected from I, L, F, V, A and G,
   X₂ is selected from G, A, V, L, I, F, α-aminobutyric acid and dehydrobutyrine,
   A₁, A₂ and A₄ are alanine and A₃ is α-aminobutyric acid,
   X₃ is selected from I, L, F, V, A and G,
   X₄ is selected from A, G, V, L, I, F and dehydroalanine,
   X₅ is selected from L, I, F, V, A, G,
   X₆ is selected from K, G, A, V, N, Q, R, H;
   and wherein A₁ and A₂ together form a lanthionine linkage and A₃ and A₄ together form a β-methyllanthionine linkage;
(b) L is a linker moiety selected from the group consisting of (i) a direct covalent bond linkage between T and P and (ii) a linker molecule covalently bonded to T and P; and
(c) P is an antibiotic moiety that is capable of altering at least one activity of a bacterial membrane or a bacterial cell wall;
or a pharmaceutically acceptable salt thereof.

An antibiotic composition may be comprised of the following structure:

**T-L-P**

wherein:
(a) T is a targeting moiety comprising an N-terminal peptide fragment of a lantibiotic where the peptide fragment comprises the following formula:
   X₁X₂A₁X₃X₄X₅A₂A₃PGA₄X₆ (SEQ ID NO:6)
   wherein
   A₁, A₂ and A₄ are alanine and A₃ is α-aminobutyric acid, and wherein A₁ and A₂ together form a lanthionine linkage and A₃ and A₄ together form a β-methyllanthionine linkage;
   X₁, X₂, X₃, X₄, X₅ and X₆ are each independently selected from any natural or non-natural amino acid,
   and wherein T is capable of binding interactions with a pyrophosphate of bacterial cell wall precursor Lipid II wherein said Lipid II comprises undecaprenyl-pyrophsophoryl-MurNAc-(pentapeptide)-GlcNAc;
   L is a linker moiety selected from the group consisting of (i) a direct covalent bond linkage
(b) between T and P and (ii) a linker molecule covalently bonded to T and P; and
(c) P is an antibiotic moiety that is capable of altering at least one activity of a bacterial membrane or a bacterial cell wall;
or pharmaceutically acceptable salts thereof.

An antibiotic composition may be comprised of the following structure:

**T-L-P**

wherein:
(a) T is a targeting moiety comprising an N-terminal peptide fragment of a lantibiotic where the peptide fragment comprises the following formula:
   X₁X₂A₁X₃X₄X₅A₂A₃PGA₄X₆ (SEQ ID NO:7)
   wherein
   X₁ is I,
   X₂ is selected from A, K, α-aminobutyric acid and dehydrobutyrine,
   A₁, A₂ and A₄ are alanine and A₃ is α-aminobutyric acid,
   X₃ is I,
   X₄ is selected from F, alanine and dehydroalanine,
   X₅ is L,
   X₆ is K;
   and wherein A₁ and A₂ together form a lanthionine linkage and A₃ and A₄ together form a β-methyllanthionine linkage;
   L is a linker moiety selected from the group consisting of (i) a direct covalent bond linkage
(b) between T and P and (ii) a linker molecule covalently bonded to T and P; and
(c) P is an antibiotic moiety that is capable of altering at least one activity of a bacterial membrane or a bacterial cell wall;
or pharmaceutically acceptable salts thereof.

An antibiotic composition may be comprising a compound of formula:

**T-L-P**

wherein:
T is a targeting moiety comprising a nisin[1-12] polypeptide fragment having lanthionine ring structure and a β-methyllanthionine ring structure;
L is a linker moiety comprising a linker molecule, wherein the linker molecule forms a first amide linkage to the C-terminus of T and a second amide linkage to a C-terminus of P; and
P comprises vancomycin;
or pharmaceutically acceptable salts thereof.

In another embodiment, the invention is directed to pharmaceutical compositions comprising an antibiotic composition as defined in the claims, and a pharmaceutically acceptable excipient.

T is capable of binding interactions with a pyrophosphate of bacterial cell wall precursor Lipid II wherein said Lipid II comprises undecaprenyl-pyrophsophoryl-MurNAc-(pentapeptide)-GlcNAc. In certain related further embodiments L comprises a direct covalent bond linkage between the targeting moiety and the antibiotic moiety. In certain other related further embodiments L comprises a direct covalent bond linkage selected from an amide, ester, ether, thioether, phosphorodiester, thiophosphorodiester, carbonate, carbamate, hydrazone, oxime and amino linkage. In certain other related further embodiments L comprises a linker molecule covalently bonded to T and P. In certain other related further embodiments P comprises vancomycin or a vancomycin derivative.

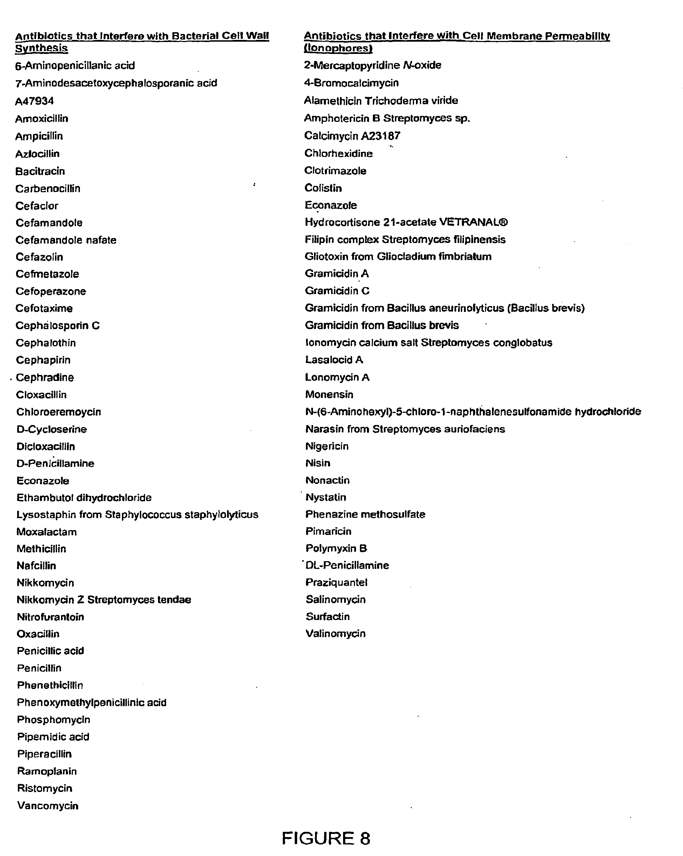

P comprises an antibiotic moiety that is capable of altering at least one activity of a bacterial membrane or a bacterial cell wall. In certain other related embodiments, the antibiotic entity capable of altering at least one activity of a bacterial membrane or a bacterial cell wall is selected from beta-lactam antibiotics such as penicillins, cephalosporins, carbapenems and monobactams; glycopeptide, lipoglycopeptide and lioglycodepsipetide antibiotics such as vancomycin, vancomycin derivatives, teicoplanin, ramoplanin, oritavancin and dalbavancin; polypeptides such as polymyxin and bacitracin; and the antibiotics listed in FIGURE 8. In certain other related embodiments, the antibiotic entity capable of altering at least one activity of a bacterial membrane or a bacterial cell wall is selected from vancomycin, a vancomycin derivative, teicoplanin, ramoplanin; oritavancin, dalbavancin, polymyxin, bacitracin, and an antibiotic moiety of FIGURE 8.

These and other aspects of the present invention will become apparent upon reference to the following detailed description and attached drawings.

### BRIEF DESCRIPTION OF FIGURES

FIGURE 1 illustrates a method of preparing Segment I (SEQ ID Nos. 12-15) of nisin[1-12] polypeptide fragment.
FIGURE 2 illustrates a method of preparing the A-ring of Segment I (SEQ ID Nos. 12, 16 and 17) of nisin[1-12] polypeptide fragment.
FIGURE 2A illustrates the preparation of certain amino acids used in the preparation of the A-ring of Segment I.
FIGURE 3 illustrates a method of preparing Segment II (SEQ ID Nos. 18-20) of nisin[1-12] polypeptide fragment.
FIGURE 3A illustrates a method of preparing an amino acid used in the preparation of Segment I.
FIGURE 4 illustrates a method of coupling Segment I (SEQ ID NO:15) and Segment II (SEQ ID NO:20) of nisin[1-12] polypeptide fragment to produce nisin[1-12] polypeptide fragment (SEQ ID NO:10).
FIGURE 5 illustrates a method of preparing Segment I' (SEQ ID Nos. 21 and 22) of saturated nisin[1-12] polypeptide fragment.
FIGURE 6 illustrates a method of preparing the A'-ring of Segment I' (SEQ ID Nos. 21, 23 and 24) of saturated nisin[1-12] polypeptide fragment.
FIGURE 7 illustrates a method of coupling Segment I' (SEQ ID NO:22) of saturated nisin[1-12] polypeptide fragment and Segment II (SEQ ID NO:20) of nisin[1-12] polypeptide fragment to produce saturated nisin[1-12] polypeptide fragment (SEQ ID NO:11).
FIGURE 8 shows examples of antibiotic moieties that are capable of altering at least one activity of a bacterial membrane or a bacterial cell wall.

### DETAILED DESCRIPTION OF THE INVENTION

Certain invention embodiments described herein derive from the surprising discovery that a lantibiotic N-terminal polypeptide fragment, such as a nisin[1-12] polypeptide fragment, may be usefully employed in the construction of the presently disclosed antibiotic compositions as a targeting moiety that is capable of binding interactions with a pyrophosphate of the bacterial cell wall precursor known as Lipid II (undecaprenyl-pyrophsophoryl-MurNAc-(pentapeptide)-GlcNAc, wherein the pentapeptide typically has the sequence L-Ala-D-γ-Glu-L-Lys-D-Ala-D-Ala (SEQ ID NO:8)). Unexpectedly, and as described in greater detail below, a lantibiotic N-terminal polypeptide fragment, such as the nisin[1-12] fragment derived from a full-length nisin polypeptide, retains biological activity as a targeting moiety for delivery of the antibiotic moiety. Thus, and according to non-limiting theory, the herein disclosed T-L-P conjugates provide surprisingly advantageous antibiotic biological activity in the form of targeted delivery of a distinct functional antibiotic moiety to a bacterial membrane or cell wall, via a lantibiotic polypeptide fragment targeting moiety that recognizes a molecular target which is not easily mutated in antibiotic-resistant bacterial strains. Accordingly and as described herein, there are provided antibiotic compositions having antibacterial activity that is greater than the antibacterial activity of either the targeting moiety or the antibiotic moiety alone.

The present disclosure, according to certain embodiments and as described herein, thus relates to antibiotic compositions having the generalized structure:

T-L-P

wherein T is a targeting moiety, L is a linker moiety, and P is an antibiotic moiety that is capable of altering at least one activity of a bacterial membrane or a bacterial cell wall, to methods of making such antibiotic compositions and to methods of using these compositions. The invention is defined as set forth in the claims.

In certain related embodiments the antibiotic composition may comprise combinations of two or more such T-L-P conjugates that may differ from one another by having distinct moieties for one or more of T, L and P as provided herein, for example, T₁-L₁-P₁, T₂-L₂-P₂, T₃-L₃-P₃, etc., or as another example, T₁-L₁-P₁, T₁-L₂-P₂, T₁-L₂-P₃, etc. In a certain such preferred embodiment, a combination of T-L-P conjugates may comprise common T and L moieties but may differ from one another by having two or more distinct antibiotic (P) moieties, for example, T-L-P₁, T-L-P₂, T-L-P₃, etc., wherein P₁, P₂, P₃, etc. represent distinct antibiotic moieties as provided herein.

Hence, according to certain embodiments there is provided an antibiotic composition comprising a plurality of non-identical compositions having the structure T-L-P, wherein T comprises at least one targeting moiety that is the same or different in said non-identical compositions and that is selected from a targeting moiety as described herein, L comprises at least one linker moiety as described herein and that is the same or different in said non-identical compositions, and P comprises at least one antibiotic moiety that is different in said non-identical compositions, each of said antibiotic moieties comprising an antibiotic moiety that is capable of altering at least one activity of a bacterial membrane or a bacterial cell wall as described herein; or a pharmaceutically acceptable salt thereof. In related preferred embodiments the antibiotic moiety is selected from vancomycin, a vancomycin derivative, teicoplanin, ramoplanin, oritavancin, dalbavancin, polymyxin, bacitracin, and an antibiotic moiety of FIGURE 8.

Briefly and by way of background, the glycopeptide antibiotic vancomycin and the lantibiotic nisin, a 34-amino acid polypeptide which is posttranslationally modified to contain five lanthionine rings and three dehydrated amino acids, have been long believed to recognize the same target molecule on bacterial cell surfaces, namely undecaprenyl-pyrophsophoryl-MurNAc-( L-Ala-D-γ-Glu-L-Lys-D-Ala-D-Ala)-GlcNAc (SEQ ID NO:8), also known as Lipid II, a bacterial cell wall precursor (*e.g*., Breukink et al., 1999 Science 286:2361). Vancomycin and lantibiotics such as nisin are further believed to exert their antibiotic effects by interacting with such lipid intermediates to inhibit peptidoglycan synthesis, and by binding to and/or inserting into bacterial membranes, thereby promoting pore formation in bacterial cytoplasmic membranes (*e.g*., Sahl, 1991 in Nisin and Novel Lantibiotics (Jung, G., and Sahl, H.G., Eds.), pp. 347-58, Escom, Leiden; Brotz et al., 2000 J. Antimicrob. Chemotherap. 46:1; Breukink et al., 1999 Science 286:2361; Brumfit et al., 2002 J. Antimicrobiol. Chemotherap. 50:731).

Recently, fine structural analysis of antibiotic interactions with Lipid II has permitted discernment of the vancomycin and nisin binding mechanisms, with vancomycin recognizing D-Ala-D-Ala in the Lipid II pentapeptide moiety. By contrast, the full-length nisin polypeptide, via its N-terminal region, recognizes the Lipid II pyrophosphate group (*e.g*., Bonev et al., 2004 FASEB J. 18:1862; Hsu et al., 2004 Nature Struct. Molec. Biol. 11:963). Consistent, however, with earlier characterization of the isolated nisin N-terminal dodecapeptide (nisin[1-12]) as being biologically inactive, thereby functionally distinguishing nisin[1-12] from the intact 34-amino acid nisin polypeptide (*e.g*., Chan et al., 1996 FEBS Lett. 390:129), Hsu et al. (2004) similarly concluded that nisin's lantibiotic biological activity relies upon its participation in bacterial membrane pore formation via membrane insertion of the nisin C-terminal region, and that nisin's lantibiotic biological activity also depends upon a conformational change mediated by the nisin hinge region (amino acid residues 20-22). Hence, prior to the present application the art has failed in any way to suggest that a nisin N-terminal fragment such as nisin[1-12], as distinct from the full-length (34-amino acid) nisin lantibiotic, is capable of any useful biological activity, much less as a targeting moiety for the delivery of a distinct antibiotic moiety in a manner that permits membrane insertion of the antibiotic moiety (*e.g*., vancomycin). The present disclosure thus advantageously provides compositions and methods to overcome antibiotic resistance in bacteria, by the selection of a targeting moiety that is insensitive to common bacterial mechanisms of acquiring resistance through mutation of drug target structures, wherein further such a targeting moiety may mediate delivery of a discrete antibiotic moiety that is capable of altering at least one activity of a bacterial membrane or a bacterial cell wall.

### Definitions

As used in the specification and appended claims, unless specified to the contrary, the following terms have the meaning indicated below.

Certain chemical groups named herein are preceded by a shorthand notation indicating the total number of carbon atoms that are to be found in the indicated chemical group. For example; C₇-C₁₂alkyl describes an alkyl group, as defined below, having a total of 7 to 12 carbon atoms, and C₄-C₁₂cycloalkylalkyl describes a cycloalkylalkyl group, as defined below, having a total of 4 to 12 carbon atoms. The total number of carbons in the shorthand notation does not include carbons that may exist in substituents of the group described.

"Alkyl" refers to a straight or branched hydrocarbon chain radical consisting solely of carbon and hydrogen atoms, containing no unsaturation, having from one to twelve carbon atoms, preferably one to eight carbon atoms or one to six carbon atoms, and which is attached to the rest of the molecule by a single bond, for example, methyl, ethyl, *n*-propyl, 1-methylethyl (*iso*-propyl), *n*-butyl, *n*-pentyl, 1,1-dimethylethyl (*t*-butyl), 3-methylhexyl, 2-methylhexyl, and the like. Unless stated otherwise specifically in the specification, an alkyl group may be optionally substituted by one or more of the following substituents: halo, cyano, nitro, oxo, trimethylsilanyl, -OR¹⁵, -OC(O)-R¹⁵, -N(R¹⁵)₂, -C(O)R¹⁵, -C(O)OR¹⁵, -C(O)N(R¹⁵)₂, -N(R¹⁵)C(O)OR¹⁵, -N(R¹⁵)C(O)R¹⁵, -N(R¹⁵)S(O)ₜR¹⁵ (where t is 1 or 2), -S(O)ₜOR¹⁵ (where t is 1 or 2), -S(O)ₚR¹⁵ (where p is 0, 1 or 2), and -S(O)ₜN(R¹⁵)₂ (where t is 1 or 2) where each R¹⁵ is independently hydrogen, alkyl, haloalkyl, cycloalkyl, cycloalkylalkyl, aryl (optionally substituted with one or more halo or alkyl groups), aralkyl, heterocyclyl, heterocylylalkyl, heteroaryl or heteroarylalkyl, and where each of the above substituents is unsubstituted unless otherwise indicated unless specifically defined otherwise.

"Alkenyl" refers to a straight or branched hydrocarbon chain radical group consisting solely of carbon and hydrogen atoms, containing at least one double bond, having from two to twelve carbon atoms, preferably two to eight carbon atoms and which is attached to the rest of the molecule by a single bond, for example, ethenyl, prop-1-enyl, but-1-enyl, pent-1-enyl, penta-1,4-dienyl, and the like. Unless stated otherwise specifically in the specification, an alkenyl group may be optionally substituted by one or more of the following substituents: cyano, nitro, oxo, trimethylsilanyl, -OR¹⁵, -OC(O)-R¹⁵, -N(R¹⁵)₂, -C(O)R¹⁵, -C(O)OR¹⁵, -C(O)N(R¹⁵)₂, -N(R¹⁵)C(O)OR¹⁵, -N(R¹⁵)C(O)R¹⁵, -N(R¹⁵)S(O)ₜR¹⁵ (where t is 1 or 2), -S(O)ₜOR¹⁵ (where t is 1 or 2), -S(O)ₚR¹⁵ (where p is 0, 1 or 2), and -S(O)ₜN(R¹⁵)₂ (where t is 1 or 2) were each R¹⁵ is independently hydrogen, alkyl, haloalkyl, cycloalkyl, cycloalkylalkyl, aryl (optionally substituted with one or more halo groups), aralkyl, heterocyclyl, heterocylylalkyl, heteroaryl or heteroarylalkyl, , and where each of the above substituents is unsubstituted unless otherwise indicated unless specifically defined otherwise.

"Alkynyl" refers to a straight or branched hydrocarbon chain radical group consisting solely of carbon and hydrogen atoms, containing at least one triple bond, optionally containing at least one double bond, having from two to twelve carbon atoms, preferably two to eight carbon atoms and which is attached to the rest of the molecule by a single bond, for example, ethynyl, propynyl, butynyl, pentynyl, hexynyl, and the like. Unless stated otherwise specifically in the specification, an alkynyl group may be optionally substituted by one or more of the following substituents: cyano, nitro, oxo, trimethylsilanyl, -OR¹⁵, -OC(O)-R¹⁵, -N(R¹⁵)₂, -C(O)R¹⁵, -C(O)OR¹⁵, -C(O)N(R¹⁵)₂, -N(R¹⁵)C(O)OR¹⁵, -N(R¹⁵)C(O)R¹⁵, -N(R¹⁵)S(O)ₜR¹⁵ (where t is 1 or 2), -S(O)ₜOR¹⁵ (where t is 1 or 2), -S(O)ₚR¹⁵ (where p is 0, 1 or 2), and -S(O)ₜN(R¹⁵)₂ (where t is 1 or 2) where each R¹⁵ is independently hydrogen, alkyl, haloalkyl, cycloalkyl, cycloalkylalkyl, aryl (optionally substituted with one or more halo groups), aralkyl, heterocyclyl, heterocylylalkyl, heteroaryl or heteroarylalkyl, and where each of the above substituents is unsubstituted unless specifically defined otherwise.

"Alkylene" or "alkylene chain" refers to a straight or branched divalent hydrocarbon chain linking the rest of the molecule to a radical group, consisting solely of carbon and hydrogen, containing no unsaturation and having from one to twelve carbon atoms, for example, methylene, ethylene, propylene, *n*-butylene, and the like. The alkylene chain is attached to the rest of the molecule through two single bonds. The points of attachment of the alkylene chain to the rest of the molecule can be through any two carbons within the chain. Unless stated otherwise specifically in the specification, an alkylene chain may be optionally substituted by one or more of the following substituents: halo, cyano, nitro, aryl, cycloalkyl, heterocyclyl, heteroaryl, oxo, trimethylsilanyl, -OR¹⁵, -OC(O)-R¹⁵, -N(R¹⁵)₂, -C(O)R¹⁵, -C(O)OR¹⁵, -C(O)N(R¹⁵)₂, -N(R¹⁵)C(O)OR¹⁵, -N(R¹⁵)C(O)R¹⁵, -N(R¹⁵)S(O)ₜR¹⁵ (where t is 1 or 2), -S(O)ₜOR¹⁵ (where t is 1 or 2), -S(O)ₚR¹⁵ (where p is 0, 1 or 2), and -S(O)ₜN(R¹⁵)₂ (where t is 1 or 2) where each R¹⁵ is independently hydrogen, alkyl, haloalkyl, cycloalkyl, cycloalkylalkyl, aryl (optionally substituted with one or more halo groups), aralkyl, heterocyclyl, heterocylylalkyl, heteroaryl or heteroarylalkyl, and where each of the above substituents is unsubstituted unless otherwise indicated.

"Alkenylene" or "alkenylene chain" refers to a straight or branched divalent hydrocarbon chain linking the rest of the molecule to a radical group, consisting solely of carbon and hydrogen, containing at least one double bond and having from two to twelve carbon atoms, for example, ethenylene, propenylene, *n*-butenylene, and the like. The alkenylene chain is attached to the rest of the molecule through two double bonds, through two single bonds or through one single bond and one double bond. The points of attachment of the alkenylene chain to the rest of the molecule can be through any two carbons within the chain. Unless stated otherwise specifically in the specification, an alkenylene chain may be optionally substituted by one or more of the following substituents: halo, cyano, nitro, aryl, cycloalkyl, heterocyclyl, heteroaryl, oxo, trimethylsilanyl, -OR¹⁵, -OC(O)-R¹⁵, -N(R¹⁵)₂, -C(O)R¹⁵, -C(O)OR¹⁵, -C(O)N(R¹⁵)₂, -N(R¹⁵)C(O)OR¹⁵, -N(R¹⁵)C(O)R¹⁵, -N(R¹⁵)S(O)ₜR¹⁵ (where t is 1 or 2), -S(O)ₜOR¹⁵ (where t is 1 or 2), -S(O)ₚR¹⁵ (where p is 0, 1 or 2), and -S(O)ₜN(R¹⁵)₂ (where t is 1 or 2) where each R¹⁵ is independently hydrogen, alkyl, haloalkyl, cycloalkyl, cycloalkylalkyl, aryl (optionally substituted with one or more halo groups), aralkyl, heterocyclyl, heterocylylalkyl, heteroaryl or heteroarylalkyl, and where each of the above substituents is unsubstituted unless otherwise indicated.

"Alkynylene" or "alkynylene chain" refers to a straight or branched divalent hydrocarbon chain linking the rest of the molecule to a radical group, consisting solely of carbon and hydrogen, containing at least one triple bond and having from two to twelve carbon atoms, for example, propynylene, *n*-butynylene, and the like. The alkynylene chain is attached to the rest of the molecule through two double bonds, through two single bonds or through one single bond and one double bond. The points of attachment of the alkynylene chain to the rest of the molecule can be through any two carbons within the chain. Unless stated otherwise specifically in the specification, an alkynylene chain may be optionally substituted by one or more of the following substituents: alkyl, alkenyl, halo, haloalkenyl, cyano, nitro, aryl, cycloalkyl, heterocyclyl, heteroaryl, oxo, trimethylsilanyl, -OR¹⁵, -OC(O)-R¹⁵, -N(R¹⁵)₂, -C(O)R¹⁵, -C(O)OR¹⁵, -C(O)N(R¹⁵)₂, -N(R¹⁵)C(O)OR¹⁵, -N(R¹⁵)C(O)R¹⁵, -N(R¹⁵)S(O)ₜR¹⁵ (where t is 1 or 2), -S(O)ₜOR¹⁵ (where t is 1 or 2), -S(O)ₚR¹⁵ (where p is 0, 1 or 2), and -S(O)ₜN(R¹⁵)₂ (where t is 1 or 2) where each R¹⁵ is independently hydrogen, alkyl, haloalkyl, cycloalkyl, cycloalkylalkyl, aryl (optionally substituted with one or more halo groups), aralkyl, heterocyclyl, heterocylylalkyl, heteroaryl or heteroarylalkyl, and where each of the above substituents is unsubstituted unless otherwise indicated.

"Aryl" refers to aromatic monocyclic or multicyclic hydrocarbon ring system consisting only of hydrogen and carbon and containing from 6 to 19 carbon atoms, where the ring system may be partially or fully saturated. Aryl groups include, but are not limited to, groups such as fluorenyl, phenyl and naphthyl. The aryl group is attached through the rest of the molecule through one single bond or through one double bond. Unless stated otherwise specifically in the specification, the term "aryl" or the prefix "ar-" (such as in "aralkyl") is meant to include aryl radicals optionally substituted by one or more substituents independently selected from the group consisting of alkyl, akenyl, alkynyl, halo, haloalkyl, haloalkenyl, cyano, nitro, aryl, aralkyl, cycloalkyl, cycloalkylalkyl, heterocyclyl, heterocyclylalkyl, heteroaryl, heteroarylalkyl, -R¹⁶-OR¹⁵, -R¹⁶-OC(O)-R¹⁵, -R¹⁶-N(R¹⁵)₂, -R¹⁶-C(O)R¹⁵, -R¹⁶-C(O)OR¹⁵, -R¹⁶-C(O)N(R¹⁵)₂, -R¹⁶-N(R¹⁵)C(O)OR¹⁵, -R¹⁶-N(R¹⁵)C(O)R¹⁵, -R¹⁶-N(R¹⁵)C(O)N(R¹⁵)₂, -R¹⁶-N(R¹⁵)S(O)ₜR¹⁵ (where t is 1 or 2), -R¹⁶-S(O)ₜOR¹⁵ (where t is 1 or 2), -R¹⁶-S(O)ₚR¹⁵ (where p is 0, 1 or 2), and -R¹⁶-S(O)ₜN(R¹⁵)₂ (where t is 1 or 2), where each R¹⁵ is independently hydrogen, alkyl, haloalkyl, cycloalkyl, cycloalkylalkyl, aryl, aralkyl, heterocyclyl, heterocyclylalkyl, heteroaryl or heteroarylalkyl, and each R¹⁶ is independently a direct bond or a straight or branched alkylene or alkenylene chain.

"Arylene" refers to a divalent aryl group, as defined above, which is attached to the rest of the molecule through two different carbons in the aryl group. An example of an arylene group is phenylene, as shown below:

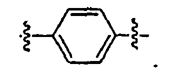

The arylene group may be optionally substituted as defined above for an aryl group. "Aralkyl" refers to a radical of the formula -RₐR_{b} where Rₐ is an alkylene radical as defined above and R_{b} is one or more aryl radicals as defined above, for example, benzyl, diphenylmethyl and the like. The aryl radical(s) may be optionally substituted as described above. The alkylene part of the aralkyl radical may be optionally substituted as defined above for an alkylene group.

"Aralkenyl" refers to a radical of the formula -R_{c}R_{b} where R_{c} is an alkenylene radical as defined above and R_{b} is one or more aryl radicals as defined above. The aryl part of the aralkenyl radical may be optionally substituted as described above for an aryl group. The alkenylene part of the aralkenyl radical may be optionally substituted as defined above for an alkenylene group.

"Aralkynyl" refers to a radical of the formula -R_{d}R_{b} where R_{d} is an alkynylene radical as defined above and R_{b} is one or more aryl radicals as defined above. The aryl part of the aralkynyl radical may be optionally substituted as described above for an aryl group. The alkynylene part of the aralkynyl radical may be optionally substituted as defined above for an alkynyl group.

"Aryloxy" refers to a radical of the formula -OR_{b} where R_{b} is an aryl group as defined above. The aryl part of the aryloxy radical may be optionally substituted as defined above.

"Aralkyloxy" refers to a radical of the formula -OR_{b} where R_{b} is an aralkyl group as defined above. The aralkyl part of the aralkyloxy radical may be optionally substituted as defined above.

"Cycloalkyl" refers to a non-aromatic stable non-aromatic monocyclic or polycyclic hydrocarbon radical consisting solely of carbon and hydrogen atoms, which may include fused or bridged ring systems, having from three to fifteen carbon atoms, preferably having from three to ten carbon atoms, and which is saturated or unsaturated and attached to the rest of the molecule by a single bond. Monocyclic radicals include, for example, cyclopropyl, cyclobutyl, cyclopentyl, cyclohexyl, cycloheptly, and cyclooctyl. Polycyclic radicals include, for example, adamantine, norbornane, decalinyl, 7,7-dimethyl-bicyclo[2.2.1]heptanyl, and the like. Unless otherwise stated specifically in the specification, the term "cycloalkyl" is meant to include cycloalkyl radicals which are optionally substituted by one or more substituents independently selected from the group consisting of alkyl, alkenyl, halo, haloalkyl, haloalkenyl, cyano, nitro, oxo, aryl, aralkyl, cycloalkyl, cycloalkylalkyl, heterocyclyl, heterocyclylalkyl, heteroaryl, heteroarylalkyl, -R¹⁶-OR¹⁵, -R¹⁶-OC(O)-R¹⁵, -R¹⁶-N(R¹⁵)₂, -R¹⁶-C(O)R¹⁵, -R¹⁶-C(O)OR¹⁵, -R¹⁶-C(O)N(R¹⁵)₂, -R¹⁶-N(R¹⁵)C(O)OR¹⁵, -R¹⁶-N(R¹⁵)C(O)R¹⁵, -R¹⁶-N(R¹⁵)C(O)N(R¹⁵)₂, -R¹⁶-N(R¹⁵)S(O)ₜR¹⁵ (where t is 1 or 2), -R¹⁶-S(O)ₜOR¹⁵ (where t is 1 or 2), -R¹⁶-S(O)ₚR¹⁵ (where p is 0, 1 or 2), and -R¹⁶-S(O)ₜN(R¹⁵)₂ (where t is 1 or 2), where each R¹⁵ is independently hydrogen, alkyl, haloalkyl, cycloalkyl, cycloalkylalkyl, aryl, aralkyl, heterocyclyl, heterocyclylalkyl, heteroaryl or heteroarylalkyl, and each R¹⁶ is independently a direct bond or a straight or branched alkylene or alkenylene chain.

"Cycloalkylene" refers to a divalent cycloalkyl group, as defined above, which is attached to the rest of the molecule through two different carbons in the cycloalkylene group. An example of an cycloalkylene group is shown below: 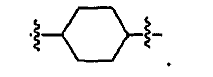 The cycloalkylene group may be optionally substituted as defined above for an cycloalkyl group.

"Cycloalkylalkyl" refers to a radical of the formula -RₐRₑ where Rₐ is an alkylene group as defined above and Rₑ is a cycloalkyl group as defined above. The alkylene group and the cycloalkyl group may be optionally substituted as defined above.

"Cycloalkylalkenyl" refers to a radical of the formula -R_{c}Rₑ where R_{c} is an alkenylene group as defined above and Rₑ is a cycloalkyl group as defined above. The alkenylene group and the cycloalkyl group may be optionally substituted as defined above.

"Halo" refers to bromo, chloro, fluoro or iodo.

"Haloalkyl" refers to an alkyl radical, as defined above, that is substituted by one or more halo radicals, as defined above, for example, trifluoromethyl, difluoromethyl, trichloromethyl, 2,2,2-trifluoroethyl, 1-fluoromethyl-2-fluoroethyl, 3-bromo-2-fluoropropyl, 1-bromomethyl-2-bromoethyl, and the like. The alkyl part of the haloalkyl radical may be optionally substituted as defined above for an alkyl group.

"Haloalkenyl" refers to an alkenyl radical, as defined above, that is substituted by one or more halo radicals, as defined above. The alkenyl part of the haloalkyl radical may be optionally substituted as defined above for an alkenyl group.

"Haloalkynyl" refers to an alkynyl radical, as defined above, that is substituted by one or more halo radicals, as defined above. The alkynyl part of the haloalkyl radical may be optionally substituted as defined above for an alkynyl group.

"Heterocyclyl" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include, but are not limited to, azepinyl, 2,5-diazabicyclo[2.2.1]heptan-2-yl, hexahydro-1*H*-1,4-diazepinyl, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxiranyl, oxazolidinyl, piperidinyl, piperazinyl, 4-piperidonyl, pyrrolidinyl, pyrazolidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, the term "heterocyclyl" is meant to include heterocyclyl radicals as defined above which are optionally substituted by one or more substituents selected from the group consisting of alkyl, alkenyl, halo, haloalkyl, haloalkenyl, cyano, oxo, thioxo, nitro, aryl, aralkyl, cycloalkyl, cycloalkylalkyl, heterocyclyl, heterocyclylalkyl, heteroaryl, heteroarylalkyl, -R¹⁶-OR¹⁵, -R¹⁶-OC(O)-R¹⁵, -R¹⁶-N(R¹⁵)₂, -R¹⁶-C(O)R¹⁵, -R¹⁶-C(O)OR¹⁵, -R¹⁶-C(O)N(R¹⁵)₂, -R¹⁶-N(R¹⁵)C(O)OR¹⁵, -R¹⁶-N(R¹⁵)C(O)R¹⁵, -R¹⁶-N(R¹⁵)C(O)N(R¹⁵)₂, -R¹⁶-N(R¹⁵)S(O)ₜR¹⁵ (where t is 1 or 2), -R¹⁶-S(O)ₜOR¹⁵ (where t is 1 or 2), -R¹⁶-S(O)ₚR¹⁵ (where p is 0, 1 or 2), and -R¹⁶-S(O)ₜN(R¹⁵)₂ (where t is 1 or 2), where each R¹⁵ is independently hydrogen, alkyl, haloalkyl, cycloalkyl, cycloalkylalkyl, aryl, aralkyl, heterocyclyl, heterocyclylalkyl, heteroaryl or heteroarylalkyl, and each R¹⁶ is independently a direct bond or a straight or branched alkylene or alkenylene chain.

"Heterocyclylalkyl" refers to a radical of the formula -RₐR_{f} where Rₐ is an alkylene group as defined above and R_{f} is a heterocyclyl group as defined above, and if the heterocyclyl is a nitrogen-containing heterocyclyl, the heterocyclyl may be attached to the alkyl radical at the nitrogen atom. The alkylene part of the heterocyclylalkyl radical may be optionally substituted as defined above for an alkylene group. The heterocyclyl part of the heterocyclylalkyl radical may be optionally substituted as defined above for a heterocyclyl group.

"Heteroaryl" refers to a 3- to 18-membered fully or partially aromatic ring radical which consists of three to thirteen carbon atoms and from one to six heteroatoms selected from the group consisting of nitrogen, oxygen and sulfur. For purposes of this invention, the heteroaryl radical may be a monocyclic, bicyclic, tricyclic or tetracyclic ring system, which may include fused or bridged ring systems; and the nitrogen, carbon or sulfur atoms in the heteroaryl radical may be optionally oxidized; the nitrogen atom may be optionally quaternized. Examples include, but are not limited to, acridinyl, benzimidazolyl, benzindolyl, benzodioxolyl, benzofuranyl, benzooxazolyl, benzothiazolyl, benzothiadiazolyl, benzo[*b*][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, indolizinyl, isoxazolyl, naphthyridinyl, oxadiazolyl, 2-oxoazepinyl, oxazolyl, phenazinyl, phenothiazinyl, phenoxazinyl, phthalazinyl, pteridinyl, purinyl, pyrazolyl, pyridinyl, pyrazinyl, pyrimidinyl, pyridazinyl, pyrrolyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e. thienyl). Unless stated otherwise specifically in the specification, the term "heteroaryl" is meant to include heteroaryl radicals as defined above which are optionally substituted by one or more substituents selected from the group consisting of alkyl, alkenyl, alkoxy, halo, haloalkyl, haloalkenyl, cyano, oxo, thioxo, nitro, oxo, aryl, aralkyl, cycloalkyl, cycloalkylalkyl, heterocyclyl, heterocyclylalkyl, heteroaryl, heteroarylalkyl, -R¹⁶-OR¹⁵, -R¹⁶-OC(O)-R¹⁵, -R¹⁶-N(R¹⁵)₂, -R¹⁶-C(O)R¹⁵, -R¹⁶-C(O)OR¹⁵, -R¹⁶-C(O)N(R¹⁵)₂, -R¹⁶-N(R¹⁵)C(O)OR¹⁵, -R¹⁶-N(R¹⁵)C(O)R¹⁵, -R¹⁶-N(R¹⁵)C(O)N(R¹⁵)₂, -R¹⁶-N(R¹⁵)S(O)ₜR¹⁵ (where t is 1 or 2), -R¹⁶-S(O)ₜOR¹⁵ (where t is 1 or 2), -R¹⁶-S(O)ₚR¹⁵ (where p is 0, 1 or 2), and -R¹⁶-S(O)ₜN(R¹⁵)₂ (where t is 1 or 2), where each R¹⁵ is independently hydrogen, alkyl, haloalkyl, cycloalkyl, cycloalkylalkyl, aryl, aralkyl, heterocyclyl, heterocyclylalkyl, heteroaryl or heteroarylalkyl, and each R¹⁶ is independently a direct bond or a straight or branched alkylene or alkenylene chain.

"Heteroarylalkyl" refers to a radical of the formula -RₐR_{g} where Rₐ is an alkylene group as defined above and R_{g} is a heteroaryl group as defined above: The heteroaryl part of the heteroarylalkyl radical may be optionally substituted as defined above for a heteroaryl group. The alkylene part of the heteroarylalkyl radical may be optionally substituted as defined above for an alkyl group.

"Stable compound" and "stable structure" are meant to indicate a compound that is sufficiently robust to survive isolation to a useful degree of purity from a reaction mixture, and formulation into an efficacious therapeutic agent.

"Mammal" includes humans and domestic animals, such as cats, dogs, swine, cattle, sheep, goats, horses; rabbits, and the like. Preferably, for purposes of this invention, the mammal is a human.

"Natural or non-natural amino acid" includes any of the common naturally occurring amino acids which serve as building blocks for the biosynthesis of peptides, polypeptides and proteins (*e.g*., alanine (Ala, A), cysteine (Cys, C), aspartic acid (Asp, D), glutamic acid (Glu, E), phenylalanine (Phe, F), glycine (Gly, G), histidine (His, H), isoleucine (Ile, I), lysine (Lys, K), leucine (Leu, L), methionine (Met, M), asparagine (Asn, N), proline (Pro, P), glutamine (Gln, Q), arginine (Arg, R), serine (Ser, S), threonine (Thr, T), valine (Val, V), tryptophan (Trp, W), tyrosine (Tyr, Y)) and also includes modified, derivatized, enantiomeric, rare and/or unusual amino acids, whether naturally occurring or synthetic, for instance, hydroxyproline, hydroxylysine, desmosine, isodesmosine, ε-N-methyllysine, ε-N-trimethyllysine, methylhistidine, dehydrobutyrine, dehydroalanine, α-aminobutyric acid, β-alanine, γ-aminobutyric acid, homocysteine, homoserine, citrulline, ornithine and other amino acids that may be isolated from a natural source and/or that may be chemically synthesized, for instance, as may be found in Proteins, Peptides and Amino Acids Sourcebook (White, J.S. and White, D.C., 2002 Humana Press, Totowa, NJ) or in Amino Acid and Peptide Synthesis (Jones, J., 2002 Oxford Univ. Press USA, New York) or in Unnatural Amino Acids, ChemFiles Vol. 1, No. 5 (2001 Fluka Chemie GmbH; Sigma-Aldrich, St. Louis, MO) or in Unnatural Amino Acids II, ChemFiles Vol. 2, No. 4 (2002 Fluka Chemie GmbH; Sigma-Aldrich, St. Louis, MO). Additional descriptions of natural and/or non-natural amino acids may be found, for example, in Kotha, 2003 Acc. Chem. Res. 36:342; Maruoka et al., 2004 Proc. Nat. Acad. Sci. USA 101:5824; Lundquist et al., 2001 Org. Lett. 3:781; Tang et al., 2002 J. Org. Chem. 67:7819; Rothman et al., 2003 J. Org. Chem. 68:6795; Krebs et al., 2004 Chemistry 10:544; Goodman et al., 2001 Biopolymers 60:229; Sabat et al., 2000 Org. Lett. 2:1089; Fu et al., 2001 J. Org. Chem. 66:7118; and Hruby et al., 1994 Meths. Mol. Biol. 35:249. A protein engineering approach to modify posttranslationally dehydrated amino acid residues in the *Lactococcus lactis* lantibiotic nisin is described by Kuipers et al. (1992 J. Biol. Chem. 267:24340).

The standard three-letter abbreviations and 1-letter symbols are used herein to designate natural and non-natural amino acids. In addition, "DAIa" is used herein to designate D-alanine; "Dhb" is used herein to designate dehydrobutyrine; "Dha" is used herein to designate dehydroalanine; "Abu" is used herein to designate α-aminobutyric acid; and "DAbu" is used herein to designate D-α-aminobutyric acid.

"Optional" or "optionally" means that the subsequently described event of circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution.

"Pharmaceutically acceptable excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" includes both acid and base addition salts.

"Pharmaceutically acceptable acid addition salt" refers to those salts which retain the biological effectiveness and properties of the free bases, which are not biologically or otherwise undesirable, and which are formed with inorganic acids such as, but not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxaiic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like. Particularly preferred organic bases are isopropylamine, diethylamine, ethanolamine, trimethylamine, dicyclohexylamine, choline and caffeine.

A "pharmaceutical composition" refers to a formulation of a compound of the invention and a medium generally accepted in the art for the delivery of the biologically active compound to mammals, for example, humans. Such a medium includes all pharmaceutically acceptable carriers, diluents or excipients therefor.

"Therapeutically effective amount" refers to that amount of an antibiotic composition of the invention which, when administered to a mammal, preferably a human, is sufficient to effect treatment, as defined below, of a disease or condition of interest in the mammal, preferably a human. The amount of an antibiotic composition of the invention which constitutes a "therapeutically effective amount" will vary depending on the antibiotic composition, the disease or condition and its severity, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, preferably a human, having the disease or condition of interest, and includes:
(i) preventing the disease or condition from occurring in a mammal, in particular, when such mammal is predisposed to the condition but has not yet been diagnosed as having it;
(ii) inhibiting the disease or condition, *i*.*e*., arresting its development;
(iii) relieving the disease or condition, *i*.*e*., causing regression of the disease or condition; or
(iv) stabilizing the disease or condition.

As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

As used herein and in the appended claims, the singular forms "a," "and," and "the" include plural referents unless the context clearly dictates otherwise. Thus, for example, reference to "an agent" or to "a composition" includes a plurality of such agents or compositions, and reference to "the cell" includes reference to one or more cells and equivalents thereof known to those skilled in the art, and so forth. The term "comprising" (and related terms such as "comprise" or "comprises" or "having" or "including") is not intended to exclude embodiments wherein, for example, any composition of matter, composition, method, or process, or the like, described herein may "consist of" or "consist essentially of" the described features.

### Antibiotic Compositions of the Invention

As set forth above in the Summary of the Invention, the antibiotic compositions of the invention comprise a targeting moiety covalently linked to an antibiotic moiety through a linker moiety as defined in the claims. The targeting moiety is a fragment of the lantibiotic nisin, which uses a distinct mode of interaction with bacterial membranes. The antibiotic compositions of the invention unexpectedly provide antibacterial activity greater than the individual antibacterial activity of the targeting moiety or the antibiotic moiety.

### A. Targeting Moieties of the Antibiotic Compositions of the Invention

Targeting moieties of the antibiotic compositions of the invention comprise a polypeptide fragment capable of binding to the pyrophosphate region of bacterial membrane lipids (particularly Lipid II). The polypeptide fragment of the targeting moiety is derived from nisin, and, in certain embodiments, contemplates a targeting moiety that may be a synthetic product and/or a targeting moiety that may be a bioengineered product, for instance, a product of presently available recombinant molecular biology methodologies, such as those described by Rink et al. (2005 Biochem. 44(24):8873-82), Kluskens et al., (2005 Biochem. 44(38):12827-34), Wirawan et al., (2006 Appl. Env. Microbiol. 72(2):1148-56), Aso et al., (2004 J. Biosci. Bioeng. 98(6):429-36), Patton et al., (2005 Curr. Opin. Microbiol. 8(5):543-51), and Cotter et al., (2005 Proc. Nat. Acad. Sci. USA 102(51):18584), or processes practiced by Novacta Biosystems Ltd. (Hertfordshire, UK) for the recombinant production of lantibiotics.

Nisin is a prominent member of the group of lanthionine and/or β-methyllanthionine containing peptides, called lantibiotics. Other members of this group include, but are not limited to, subtilin, epidermin, pep5, lacticin 481, ancovenin and duramycin. Lantibiotics are small sized (< 4 kDa) antimicrobial peptides which are active against a broad range of Gram-positive bacteria.

Nisin has the following amino acid sequence and structure (SEQ ID NO:9): 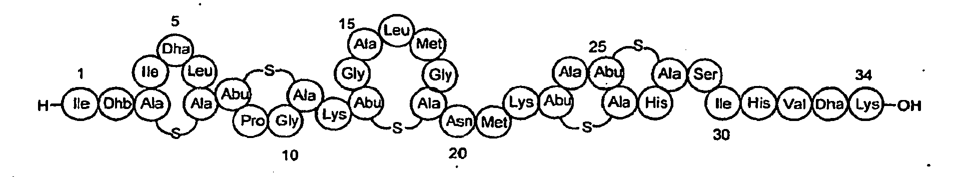 As set forth above, nisin consists of a modified polypeptide chain in which amino acids have been cyclized to give four ring structures (one lanthionine ring structure and three β-methyllanthionine ring structures) and a correspondingly rigid structure further defined by the presence of a dehydroalanine residue. The ring structure formed by amino acids 3-7 is designated herein as the A ring, the ring structure formed by amino acids 8-11 is designated herein as the B ring, the ring structure formed by amino acids 13-19 is designated herein as the C ring, the cumulated ring structure formed by amino acids 23-28 is designated herein as the D-E cumulated ring structure.

Nisin is a naturally occurring product of *lactococcus* fermentation and is to be found in milk and milk products. It is generally regarded as safe, being used as a food additivie, but prior to the present disclosure nisin has not been widely used in a pure form as a clinical antibacterial or as a component of an antibiotic composition. Nisin is believed to act in a two-step process, the first step being the recognition of the pyrophosphate of bacterial cell wall precursor Lipid II [undecaprenyl-pyrophsophoryl-MurNAc-(pentapeptide)-GlcNAc, wherein the pentapeptide may typically comprise L-Ala-D-γ-Glu-L-Lys-D-Ala-D-Ala (SEQ ID NO:8)], which is mediated by the N-terminal region of the modified polypeptide, and the second the creation of a pore in the pyrophosphate of bacterial cell wall precursor Lipid II [undecaprenyl-pyrophsophoryl-MurNAc-(pentapeptide)-GlcNAc, wherein the pentapeptide may typically comprise L-Ala-D-γ-Glu-L-Lys-D-Ala-D-Ala (SEQ ID NO:8)], which is mediated by the N-terminal region of the modified polypeptide, and the second the creation of a pore in the membrane by conformational change and self associated mediation by the C-terminal region.

An antibiotic composition comprises in pertinent part a targeting moiety that is capable of binding interactions with a pyrophosphate of the bacterial cell wall precursor Lipid II. Binding of a targeting moiety as disclosed herein to Lipid II may be determined according to any of a variety of art-accepted methodologies for detecting such binding, which is typically a result of one or more well known non-covalent intermolecular interactions such as electrostatic interactions, hydrogen bond formation, steric interactions, van der Waals forces, hydrophobic interactions and the like.

Non-limiting examples of assays that may be employed for determination of whether a molecule of interest, such as a herein described targeting moiety or antibiotic composition, is capable of binding interactions with Lipid II are described, for instance, by Brotz et al. (1998 Antimicrob. Agent. Chemother. 42:154) and by Wiedemann et al., (2001 J. Biol. Chem. 276:1772). Brotz et al. describe using a bacterial cell wall membrane preparation or a protoplast membrane fraction containing Lipid II as a binding substrate for a ligand of interest (which ligand, in the context of the present application, may be a targeting moiety, an antibiotic moiety or an antibiotic composition). These authors also describe a polyacrylamide gel electrophoretic assay for determination of a Lipid II binding ligand, in which a detectably labelled Lipid II ligand (*e.g*., an agent that is capable of binding to Lipid II) is prevented from migrating into an electrophoresis gel when Lipid II-containing micelles are available for binding interactions prior to electrophoresis. Quantification of such binding interactions may be achieved, for example, by radiometry of a detectably labelled Lipid II ligand (*e.g*., via radiometry of a radioisotope-labelled targeting moiety) or of biosynthetically radiolabeled Lipid II itself. Weidemann et al. (2001 J. Biol. Chem. 276: 1772) describe assays of radiolabeled nisin binding to Lipid II-containing artificial liposomes.

Modifications to, and variations upon, assays such as these may be employed by those familiar with the art based on the disclosure provided herein, for purposes of determining binding interactions of a candidate targeting moiety with Lipid II. Such modifications may further include the preparation for use in these assays of Lipid II

A targeting moiety as provided herein, or an antibiotic composition as provided herein, is capable of binding interactions with its target (*e.g*., Lipid II or, preferably, pyrophosphate of Lipid II) if it reacts at a detectable level with the target, preferably with an affinity constant, Kₐ, of greater than or equal to about 10⁴ M⁻¹, or greater than or equal to about 10⁵ M⁻¹, greater than or equal to about 10⁶ M⁻¹, greater than or equal to about 10⁷ M⁻¹, or greater than or equal to 10⁸ M⁻¹, and does not non-specifically bind to other structurally distinct molecules. Affinities of binding partners can be readily determined using conventional techniques, such as those described herein or, for example, those described by Scatchard et al. (Ann. N. Y. Acad. Sci. USA 51:660 (1949)), or by surface plasmon resonance (SPR; BlAcore™, Biosensor, Piscataway, NJ) or other methods routinely practiced in the art.

Of particular interest to the invention are targeting moieties comprised of polypeptide fragments of nisin, especially the polypeptide fragment comprising the first twelve N-terminal amino acids of nisin, as shown below (SEQ ID NO:10): 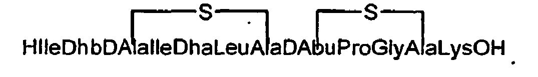 This fragment, which is defined herein as the nisin[1-12] polypeptide fragment, contains a lanthionine linkage between amino acid 3 and amino acid 7 (forming a lanthionine ring structure) and a β-methyllanthionine linkage between amino acid 13 and amino acid 19 (forming a β-methyllanthionine ring structure)

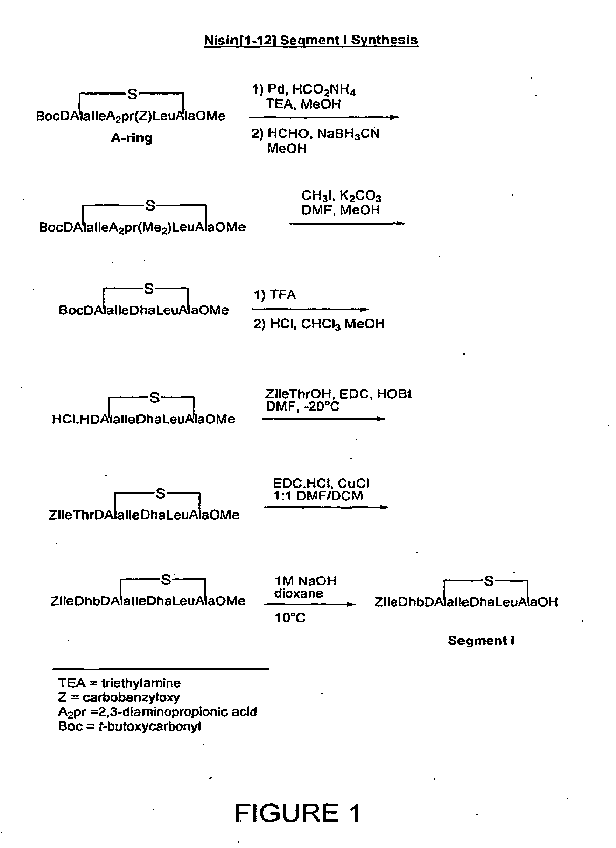

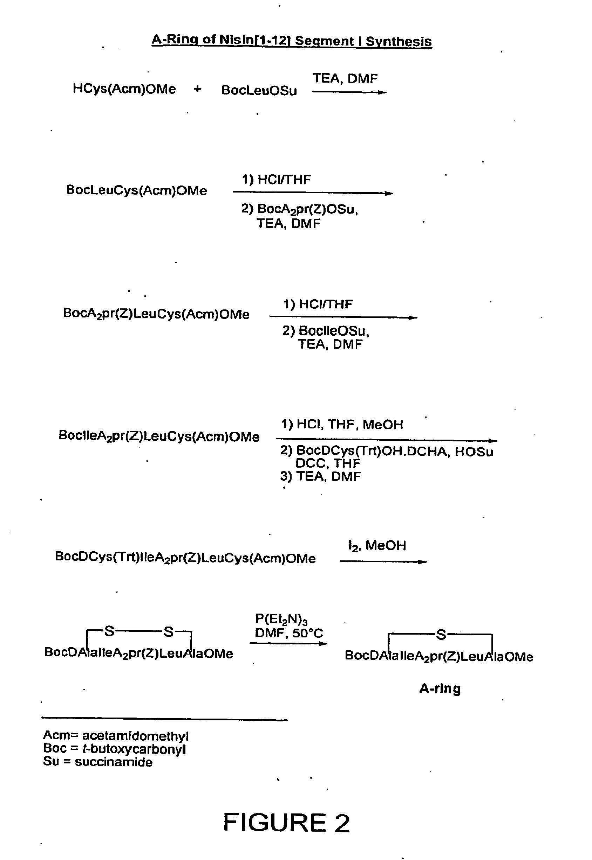

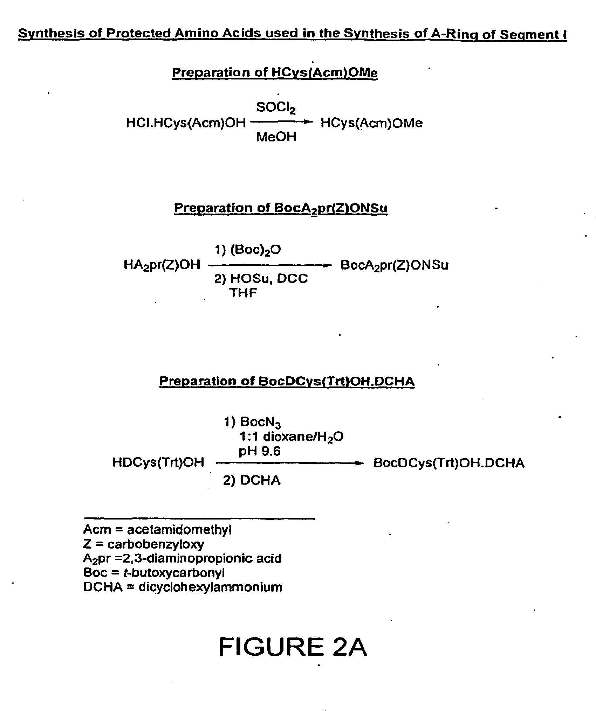

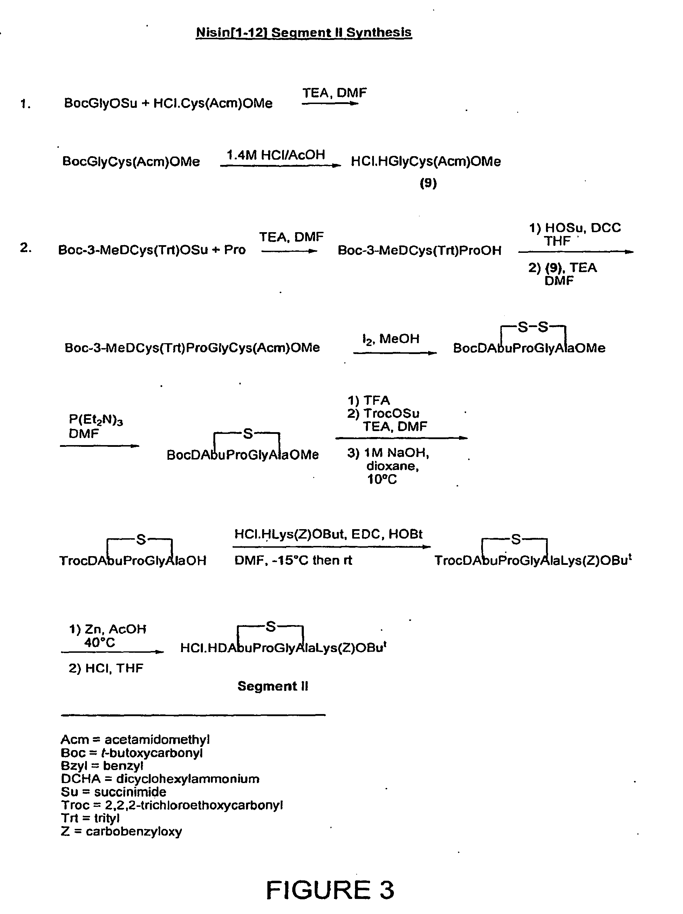

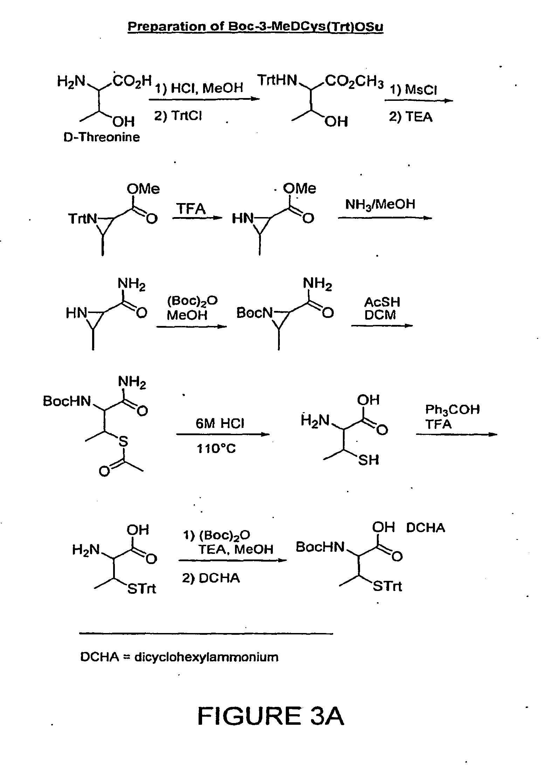

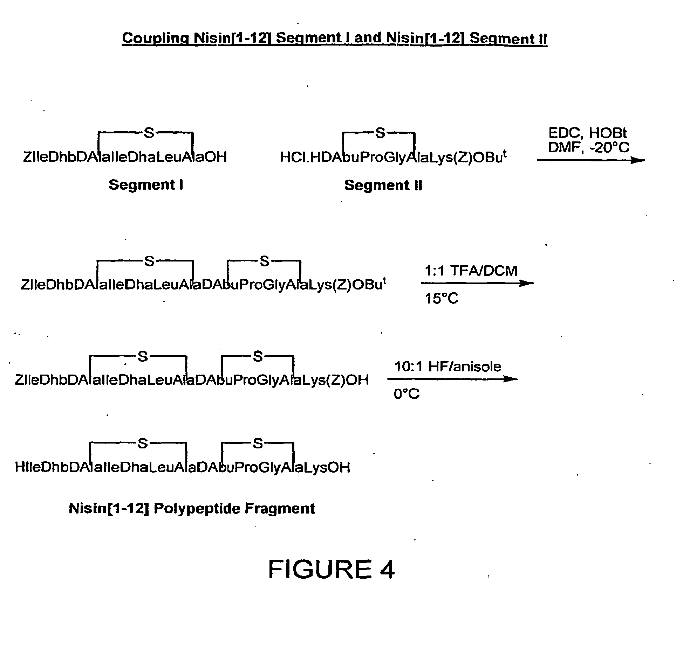

Nisin[1-12] polypeptide fragment can be synthesized from a polypeptide containing the lanthionine ring structure, defined herein as Segment I of nisin[1-12] polypeptide, and a polypeptide containing the β-methyllanthionine ring structure, defined herein as Segment II of nisin [1-12] polypeptide fragment, as illustrated in FIGURES 1-4. This synthesis is disclosed in more detail in Shiba, T. et al., "Chemistry of Lanthionine Peptides," Biopolymers (1986), Vol. 25, pp. S11-S19.

Another targeting moiety of interest to the invention is saturated nisin[1-12] polypeptide fragment. Saturated nisin[1-12] polypeptide fragment is nisin[1-12] polypeptide fragments, as defined above, wherein the second amino acid from the N-terminal, *i*.*e*., dehydrobutyrine, of the nisin[1-12] polypeptide fragment is replaced with α-aminobutyric acid and the fourth amino acid from the N-terminal, *i*.*e*., dehydroalanine, is replaced with alanine, as shown below (SEQ ID NO:11):

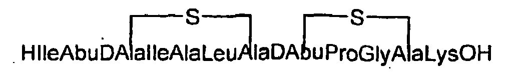

This saturated nisin[1-12] polypeptide fragment can be synthesized as dehydroalanine, is replaced with alanine, as shown below (SEQ ID NO; 11):

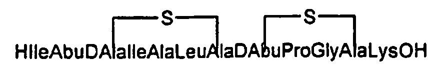

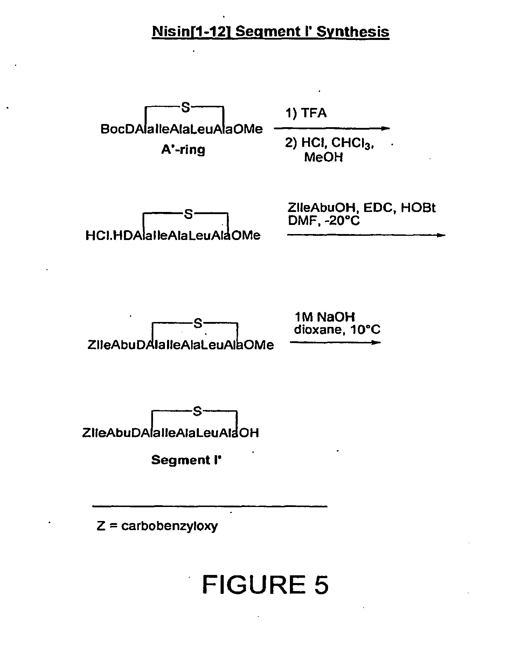

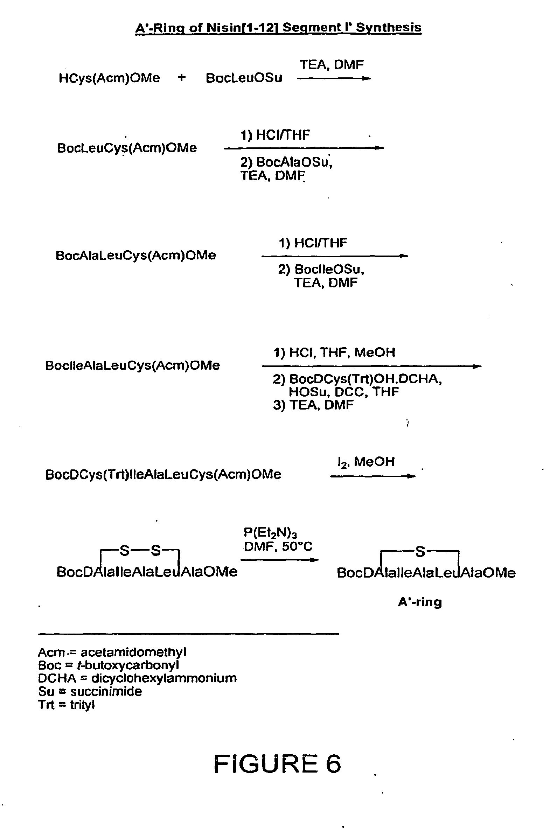

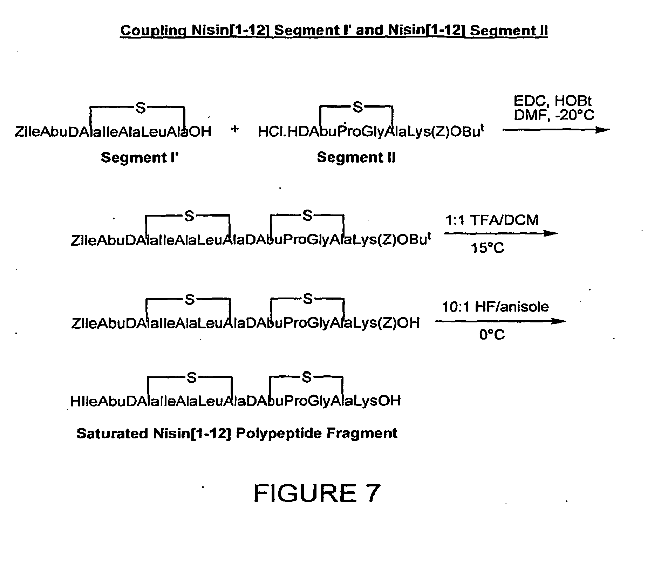

This saturated nisin[1-12] polypeptide fragment can be synthesized as illustrated in FIGURES 5-7.

In addition, nisin can be digested by trypsin to provide the nisin[1-12] polypeptide fragment, as disclosed in more detail below in the Examples (*e.g.,* Example 4).

Peptide homologues of the nisin[1-12] polypeptide fragment or the saturated nisin[1-12] polypeptide fragment are also targeting moieties. These homologues, as described in Hsu et al., 2004 Nature Struct. Molec. Biol. 11:963, have a lanthionine ring structure and a β-methyllanthionine ring structure and are selected from the group consisting of a subtilin[1-12] polypeptide fragment, an epidermin polypeptide fragment, a (I1V 16L) epidermin polypeptide fragment, a mutacin B-Ny266 polypeptide fragment and a mutacin 1140 polypeptide fragment.

Other targeting moieties include those targeting moieties comprising a N-terminal peptide fragment of a lantibiotic where the peptide fragment comprises the following formula:
X₁X₂A₁X₃X₄X₅A₂A₃PGA₄X₆ (SEQ ID NO:1)
wherein
X₁ is selected from F, I, V and W,
X₂ is selected from A, K, α-aminobutyric acid and dehydrobutyrine,
A₁, A₂ and A₄ are alanine and A₃ is α-aminobutyric acid,
X₃ is selected from E, I, K and W,
X₄ is selected from F, alanine and dehydroalanine,
X₅ is selected from I, F and L,
X₆ is selected from A, K and V;
and wherein A₁ and A₂ together form a lanthionine linkage and A₃ and A₄ together form a β-methyllanthionine linkage;

Other target moieties include those targeting moieties comprising a N-terminal peptide fragment of a lantibiotic where the peptide fragment comprises the following formula:
X₁X₂A₁X₃X₄X₅A₂A₃PGA₄X₆ (SEQ ID NO:6)
wherein
A₁, A₂ and A₄ are alanine and A₃ is α-aminobutyric acid, and wherein A₁ and A₂ together form a lanthionine linkage and A₃ and A₄ together form a β-methyllanthionine linkage;
X₁, X₂, X₃, X₄, X₅ and X₆ are each independently selected from any natural or non-natural amino acid,
and wherein T is capable of binding interactions with a pyrophosphate of bacterial cell wall precursor Lipid II [undecaprenyl-pyrophsophoryl-MurNAc-(pentapeptide)-GlcNAc].

Other target moieties include those targeting moieties comprising a N-terminal peptide fragment of a lantibiotic where the peptide fragment comprises the following formula:
X₁X₂A₁X₃X₄XA₂A₃PGA₄X₆ (SEQ ID NO:7)
wherein
X₁ is I,
X₂ is selected from A, K, α-aminobutyric acid and dehydrobutyrine,
A₁, A₂ and A₄ are alanine and A₃ is α-aminobutyric acid,
X₃ is I,
X₄ is selected from F, alanine and dehydroalanine,
X₅ is L,
X₆ is K;
and wherein A₁ and A₂ together form a lanthionine linkage and A₃ and A₄ together form a β-methyllanthionine linkage;

Targeting moieties are capable of binding interactions with a pyrophosphate of bacterial cell wall precursor Lipid II [undecaprenyl-pyrophsophoryl-MurNAc-(pentapeptide)-GlcNAc].

As noted above, established recombinant methodologies for design and synthesis of bioengineered peptides having desired sequences, such as a nisin[1-12] polypeptide fragment or a variant thereof, such as a polypeptide homologue of a nisin[1-12] polypeptide fragment, may also be employed and are well known to those having familiarity with the art. Recombinant molecular biology techniques may thus be used, including technologies that permit expression of bioengineered peptides that may be posttranslationally modified to have lanthionine linkages (*e*.*g*., Rink et al. 2005 Biochem. 44(24):8873-82, Kluskens et al., 2005 Biochem. 44(38):12827-34, Wirawan et al., 2006 Appl. Env. Microbiol. 72(2):1148-56, Aso et al., 2004 J. Biosci. Bioeng. recombinantly produced nisin[1-12] polypeptide fragments and polypeptide homologues of nisin[1-12] polypeptide fragment having lanthionine and β-methyllanthionine ring structures and having amino acid sequences as disclosed herein, and certain other disclosure embodiments contemplate variants of such peptides that have a similar amino acid sequence to the nisin[1-12] polypeptide fragment and nisin[1-12] polypeptide fragment homologue amino acid sequences disclosed herein. Such polypeptide variants may contain one or more substitutions, deletions, additions and/or insertions. Variants include, for example, naturally occurring polymorphisms (*e.g*., allelic variants) or recombinantly manipulated or engineered variants. The amino acid sequence of such a variant is at least about 80%, 85%, 90%, 95%, or 98% identical or similar to the native (*e.g*., naturally occurring) sequence. For example, a variant may have an amino acid addition, deletion or substitution at amino acid position number twelve of the nisin[1-12] polypeptide fragment sequence, which is the carboxy-terminus of the fragment, wherein by way of further non-limiting example such an addition, deletion or substitution may desirably introduce a chemical functionality in the amino acid side chain having useful properties for attachment to the linker moiety or to the antibiotic moiety. Thus, for instance, in certain embodiments the C-terminus of the targeting moiety may be selected from A, K, V, AX, KX and VX, wherein X is selected from A, G, I, L, M, P and V.

A variety of criteria known to persons skilled in the art indicate whether amino acids at a particular position in a peptide or polypeptide are conservative or similar. For example, a similar amino acid or a conservative amino acid substitution is one in which an amino acid residue is replaced with an amino acid residue having a similar side chain, such as amino acids with basic side chains (*e.g*., lysine, arginine, histidine); acidic side chains (*e.g*., aspartic acid, glutamic acid); uncharged polar side chains (*e.g*., glycine, asparagine, glutamine, serine, threonine, tyrosine, cysteine, histidine); nonpolar side chains (*e.g*., alanine, valine, leucine, isoleucine, proline, phenylalanine, methionine, tryptophan); beta-branched side chains (*e.g*., threonine, valine, isoleucine), and aromatic side chains (*e.g*., tyrosine, phenylalanine, tryptophan). Proline, which is considered more difficult to classify, shares properties with amino acids that have aliphatic side chains (*e.g*., leucine, valine, isoleucine, and alanine). In certain circumstances, substitution of glutamine for glutamic acid or asparagine for aspartic acid may be considered a similar substitution in that glutamine and asparagine are amide derivatives of glutamic acid and aspartic acid, respectively. The percent identity or similarity between amino acid sequences of two peptides, such as between a nisin[1-12] polypeptide fragment and a homologue or variant thereof, can be readily determined by visual inspection or by alignment methods (*e.g*., using GENEWORKS, Align or the BLAST algorithm), which are familiar to a person having ordinary skill in the art, and selection of sequence motifs that are amenable to posttranslational modifications such as those involved in lanthionine ring formation is within the state of the art and can be readily accomplished without undue experimentation (*e.g*., Rink et al. 2005 Biochem. 44(24):8873-82, Kluskens et al., 2005 Biochem. 44(38):12827-34).

A nisin[1-12] polypeptide fragment having a lanthionine ring structure and a β-methyllanthionine ring structure, or a homologue thereof or a variant thereof, may therefore be readily prepared by genetic engineering and recombinant molecular biology methods and techniques. Briefly, analysis of the primary and secondary amino acid sequences of such peptides and analysis by computer modeling to analyze the tertiary structure using the amino acid sequence and canonical structures and motifs of the polypeptide may aid in identifying specific amino acid residues that can be substituted, including computer-assisted prediction of sequence variants' structures (Bradley et al., Science 309:1868 (2005); Schueler-Furman et al., Science 310:638 (2005)). In addition, evolutionary conservation of or tolerance for amino acid variability in related polypeptides may provide insight into amino acid residues that may be altered to reduce, maintain, or enhance activity.

Modification of DNA encoding a nisin[1-12] polypeptide fragment or a homologue or variant thereof may be performed by a variety of methods, including site-specific or site-directed mutagenesis of the DNA, which methods include DNA amplification using primers to introduce and amplify alterations in the DNA template, such as PCR splicing by overlap extension (SOE). Mutations may be introduced at a particular location by synthesizing oligonucleotides containing a mutant sequence, flanked by restriction sites enabling ligation to fragments of the native sequence. Following ligation, the resulting reconstructed sequence encodes a variant (or derivative) having the desired amino acid insertion, substitution, or deletion.

Site-directed mutagenesis is typically effected using a phage vector that has single- and double-stranded forms, such as an M13 phage vector, which is well known and commercially available (*see, e.g*., Veira et al., Meth. Enzymol. 15:3 (1987); Kunkel et al., Meth. Enzymol. 154:367 (1987)) and in U.S. Patent Nos. 4,518,584 and 4,737,462). Oligonucleotide-directed site-specific (or segment specific) mutagenesis procedures may be employed to provide an altered polynucleotide that has particular codons altered according to the substitution, deletion, or insertion desired. Deletion or truncation derivatives of proteins may also be constructed by using convenient restriction endonuclease sites adjacent to the desired deletion. Exemplary methods of making the alterations set forth above are disclosed by Sambrook et al. (Molecular Cloning: A Laboratory Manual, 3d ed., Cold Spring Harbor Laboratory Press, NY 2001). Alternatively, random mutagenesis techniques, such as alanine scanning mutagenesis, error prone polymerase chain reaction mutagenesis, and oligonucleotide-directed mutagenesis may be used to prepare variants (*see, e.g.,* Sambrook et al., *supra*).

In addition to above-described computational prediction of polypeptide variant three-dimensional structure (Bradley et al., *supra*; Schueler-Furman et al., *supra*), confirmatory assays for assessing whether the variant folds into a conformation comparable to the non-variant polypeptide or fragment may include, for example, functional testing using the Lipid II binding assay described above.

Mutations that are made in the nucleic acid molecules encoding a nisin[1-12] polypeptide fragment or a homologue or variant thereof, for purposes of recombinant expression of the peptide fragment, preferably preserve the reading frame of the coding sequences. Furthermore, the mutations will preferably not create complementary regions that when transcribed could hybridize to produce secondary mRNA structures, such as loops or hairpins, that would adversely affect translation of the mRNA. Although a mutation site may be predetermined, the nature of the mutation *per se* need not be predetermined. For example, to select for optimum characteristics of a mutation at a given site, random mutagenesis may be conducted at the target codon and the expressed mutants screened for gain or loss or retention of biological activity (*e.g*., Lipid II binding).

Polynucleotide variants, which may be degenerate variants or which may include a polynucleotide variant that encodes a nisin[1-12] polypeptide fragment or homologue or a variant thereof, may also be identified by hybridization methods. Suitable moderately stringent conditions include, for example, pre-washing in a solution of 5X SSC, 0.5% SDS, 1.0 mM EDTA (pH 8.0); hybridizing at 50 °C-70°C, 5X SSC for 1-16 hours; followed by washing once or twice at 22-65 °C for 20-40 minutes with one or more each of 2X, 0.5X, and 0.2X SSC containing 0.05-0.1% SDS. For additional stringency, conditions may include a wash in 0.1X SSC and 0.1% SDS at 50-60 °C for 15 minutes. As understood by persons having ordinary skill in the art, variations in stringency of hybridization conditions may be achieved by altering the time, temperature, and/or concentration of the solutions used for pre-hybridization, additional stringency, conditions may include a wash in 0.1X SSC and 0.1% SDS at 50-60 °C for 15 minutes. As understood by persons having ordinary skill in the art, variations in stringency of hybridization conditions may be achieved by altering the time, temperature, and/or concentration of the solutions used for pre-hybridization, hybridization, and wash steps. Suitable conditions may also depend in part on the particular nucleotide sequences of the probe used (*i*.*e*., for example, the guanine plus cytosine (G/C) versus adenine plus thymidine (A/T) content). Accordingly, a person skilled in the art will appreciate that suitably stringent conditions can be readily selected without undue experimentation when a desired selectivity of the probe is identified.

### B. Antibiotic Moieties of the Antibiotic Compositions of the Invention

Antibiotic moieties are antibiotics which are capable of altering at least one activity of a bacterial membrane or a bacterial cell wall. "Altering" is defined herein as increasing or decreasing such an activity in a statistically significant manner, *i*.*e*., relative to an appropriate control, as may be selected by those skilled in the art. "Activity of a bacterial membrane or bacterial cell wall" is defined herein as including, but not limited to, any detectable parameter that directly relates to a condition, process, pathway, dynamic structure, state or other activity involving a bacterial membrane and/or a bacterial cell wall and for which altered (*e.g*., increased or decreased with statistical significance) function can be detected. The compositions and methods of certain embodiments of the present invention thus pertain in part to such correlation where the altered activity of a bacterial membrane or a bacterial cell wall may be, for example, an alteration in bacterial membrane permeability, including selective permeability such as altered passive or active transport of metabolites, catabolites, precursors, cofactors, mediators and the like, and/or altered biosynthetic activity for one or more bacterial membrane and/or bacterial cell wall components, or other criteria as provided herein.

"Altered activity of a bacterial membrane or a bacterial cell wall" may thus refer to any condition or state where any bacterial membrane or cell wall structure or activity that is directly or indirectly related to a bacterial function has been changed in a statistically significant manner relative to a control or standard. Altered bacterial membrane or bacterial cell wall function may have its origin in extracellular structures or events as well as in intramembranous or intracellular structures or events, in direct interactions between bacterial and host genes and/or their gene products, or in include altered respiratory, metabolic or other biochemical or biophysical activity in some or all cells. As non-limiting examples, markedly impaired maintenance of bacterial membrane potential may be related to altered bacterial membrane or cell wall function, as may be impaired bacterial replication competence or defective respiration. As further examples, altered bacterial membrane and/or bacterial cell wall biosynthesis, induction of non-selective bacterial membrane pores leading to loss of bacterial cell integrity, and formation of atypical chemical and/or biochemical crosslinked species within a bacterial cell, whether by enzymatic or non-enzymatic mechanisms, may all be regarded as indicative of altered bacterial membrane or bacterial cell wall function.

Bacterial cells in which at least one activity of a bacterial membrane or a bacterial cell wall is altered may also exhibit altered cell membrane permeability properties as may be readily detected through the use of vital dyes (*e.g*., trypan blue, fluorescent nucleic acid indicator dyes such as propidium iodide or SYTO®9, provided in, *e.g*., the LIVE/DEAD® BacLight™ Bacterial Viability Kit available from Molecular Probes, Medford, OR, or luminescent detection of cellular ATP using luciferase, such as the BacTiter-Glo™ Microbial Cell Viability assay kit available from ProMega, Madison, WI) or by the detection of a cytosolic marker (*e.g*., a readily detectable enzyme or metabolite) leakage into the extracellular milieu. These and other means for detecting cells having altered bacterial membrane or cell wall activity, by morphologic criteria, by biochemical criteria, by altered plasma membrane permeability and/or by related changes, will be apparent to those familiar with the art.

Antibiotic moieties for use according to the herein disclosed invention thus include, but are not limited to, beta-lactam antibiotics such as penicillins, cephalosporins, carbapenems and monobactams; glycopeptide, lipoglycopeptide and lioglycodepsipetide antibiotics such as vancomycin, vancomycin derivatives, teicoplanin, ramoplanin, oritavancin and dalbavancin; polypeptides such as polymyxin and bacitracin; and the antibiotics listed in FIGURE 8. Of particular interest are those antiobiotic moieties selected from the group consisting of vancomycin, a vancomycin derivative, teicoplanin, ramoplanin, oritavancin, dalbavancin, polymyxin, bacitracin, and an antibiotic moiety of FIGURE 8.

Of particular interest to the invention is the glycopeptide antibiotic known as vancomycin. Vancomycin is produced by *Streptococcus orientalis,* an actinomycete isolated from soil samples in Indonesia and India. Vancomycin is a complex tricyclic glycopeptide with a molecular mass of approximately 1500Da.

Vancomycin is active primarily against Gram-positive bacteria. Strains of bacteria are considered susceptible at a minimum inhibitory concentration of less than or equal to 4 µg/mL. *Strep. Pyogenes, Strep. Pneumoniae, Corynebacteraium* spp. are highly susceptible, as are most strains of *Enterococcus* spp. Most species of *Actinomyces* and *Clostridium* spp. are also sensitive to vancomycin, but at higher concentrations of antibiotic. Vancomycin is employed only to treat serious infections and is particularly useful in the management of infections due to methicillin-resistant staphylococci, including pneumonia, empyema, endocarditis, osteomyelitis, and soft-tissue abscesses. The agent is also extremely useful in the treatment of staphylococcal infections in patients who are allergic to penicillins and cephalosporins.

Vancomycin inhibits the synthesis of the cell wall in sensitive bacteria by blocking the cross-linking of adjacent peptidoglycan strands during the synthesis of the bacterial cell wall. Without sufficient cross-linking, the cell wall becomes mechanically fragile and the bacteria lyse when subjected to changes in osmotic pressure. Vancomycin binds with high affinity to the D-alanyl-D-alanine (D-Ala-D-Ala) terminus of the pentapeptide portion of the peptidoglycan precursor before cross-linking. The D-Ala-D-Ala dipeptide forms complementary hydrogen bonds with the peptide backbone of vancomycin. It is thought that the vancomycin-peptidoglycan complex physically blocks the action of the transpeptidase enzyme and thereby inhibits the formation of the peptide cross-bridges that strengthens the peptidoglycan: This activity also leads to the accumulation of peptidoglycan precursors in the bacterial cytoplasm.

As noted above in the Background of the Invention, resistance to antibiotics is well-known. Three types of resistance have been described for vancomycin. The VanA phenotype is inducible by vancomycin and confers resistance to both teicoplanin and vancomycin. The VanA phenotype is mediated by the transposable element Tn1546 or closely related elements. The transposon encodes a dehydrogenase (VanH) that reduces pyruvate to D-lactate (D-lac), and a ligase of broad substrate specificity (VanA) that catalyses the formation of an ester bond between D-Ala and D-Lac. The resulting D-Ala-D-Lac depsipeptide replaces the dipeptide D-Ala-D-Ala in the pathway of peptidoglycan synthesis. The substitution eliminates a hydrogen bond that is critical for antibiotic binding. The VanB phenotype is also induced upon exposure to vancomycin; however, in contrast to the VanA phenotype, these microorganisms are not resistant to teicoplanin because teicoplanin does not induce the expression of the genes required for resistance in VanB bacteria. Resistance to vancomycin by bacteria of the VanB phenotype occurs through a similar mechanism to VanA resistance, namely the substitution of the terminal D-Ala-A-Ala peptidoglycan precursor on the immature peptidoglycan by the D-Ala-D-Lac depsipeptide. One further vancomycin-resistant phenotype has been described (VanC) in *enterococci* belonging to the species *E. gallinarum, E. casseliflavus* and E. *flavescens.* These bacteria are intrinsically resistant to low levels of vancomycin and are susceptible to teicoplanin. Resistance results from the production of peptidoglycan precursors ending in D-Serine. Substitution of D-Ala by D-Ser at the carboxy-terminus of the peptidoglycan precursor analogues lowers the affinity for the precursors for vancomycin with a relatively small change in the affinity for teicoplanin.

Other antibiotic moieties that may be used according to certain embodiments of the herein disclosed invention, which antibiotic moieties are capable of altering at least one activity of a bacterial membrane or a bacterial cell wall include, but are not limited to, teicoplanin, ramoplanin, oritavancin, dalbavancin, polymyxin, bacitracin, 6-aminopenicillanic acid, 7-aminodesacetoxycephalosporanic acid, amoxicillin, ampicillin, azlocillin, bacitracin, carbenocillin, cefaclor, cefamandole, cefazolin, cefmetazole, cefoperazone, cefotaxime, cefsulodin, ceftriaxone, cephalexin, cephalosporin C, cephalothin, cephapirin, cephradine, cloxacillin, d-(-)-penicillamine, d-cycloserine, dicloxacillin, econazole, ethambutol, lysostaphin (from *staphylococcus staphylolyticus*), moxalactam, nafcillin, nikkomycin, nikkomycin Z (from *streptomyces tendae*), 2-mercaptopyridine *N*-oxide, 4-bromocalcimycin, alamethicin, amphotericin B, calcimycin (A23187), chlorhexidine diacetate, clotrimazole, colistin, econazole, hydrocortisone 21-acetate (VETRANAL®), filipin,gliotoxin (from *gliocladium fimbriatum*), gramicidins A, B, C and D, and paracelsin (from *trichoderma reesei*)*.* Suitable antibiotic moieties are also described, for example, by Fisher et al. (2005 Chem. Rev. 105:395-424), Kahne et al. (2005 Chem. Rev. 105:425-448), Walker et al. (2005 Chem. Rev. 105:449-476), Chatterjee et al. (2005 Chem. Rev. 105:633-684), and Bagley et al. (2005 Chem. Rev. 105:685-714). Examples of antibiotic moieties that may be used according to certain embodiments of the herein disclosed invention, which antibiotic moieties are capable of altering at least one activity of a bacterial membrane or a bacterial cell wall, include, but are not limited to, those shown in FIGURE 8.

Accordingly, and without wishing to be bound by theory, the preferred antibiotic moiety (P) in the herein disclosed antibiotic compositions having the general structure T-L-P is an antibiotic moiety that is capable of altering at least one activity of a bacterial membrane or a bacterial cell wall as described herein. Certain T-L-P antibiotic composition invention embodiments, however, may not be so limited and thus may include T-L-P conjugates which exert their effects intracellularly in a bacterial cell, even where the antibiotic moiety (P) is known to be capable of altering an activity of a bacterial membrane or bacterial cell wall.

### C. Linker Moieties of the Antibiotic Compositions of the Invention

The linker moieties of the antibiotic compositions of the invention link the antibiotic moieties to the targeting moieties. The linker moieties can be a direct covalent bond linkage between a functional group on the antibiotic moiety and a functional group on the targeting moiety (such as an amide covalent bond between a carboxy group on the antibiotic moiety and an amino group on the targeting moiety). Examples of such direct covalent bond linkages include amide, ester, ether, thioether, phosphorodiester, thiophosphorodiester, carbonate, carbamate, hydrazone, oxime or amino linkages. It is understood that that protecting groups may have to be used on functional groups on either the targeting moiety or the antibiotic moiety in order to prepare the desired direct linkage between the two moieties. The use of protecting groups is described in detail in Green, T.W. and P.G.M. Wutz, Protective Groups in Organic Synthesis (1999), 3rd Ed., Wiley. Alternatively, judicious control of reaction pH may provide selectivity for the desired direct linkage.

The antibiotic moiety and the targeting moiety may also be covalently bonded to each other through a linker molecule to form the antibiotic compositions of the invention. The linker molecule is derived from a corresponding parent molecule preferably containing at least two functional groups, such as a carboxyl, hydroxyl, aldehyde or amino group, each of which can be used to form a covalent bond with the appropriate functional group on the antibiotic moiety and with the appropriate functional group on the targeting moiety to form the linker molecule. The functional groups on the parent molecule can be the same or different. It is understood that protecting groups may have to be used on the functional groups on the parent molecule, the antibiotic moiety and the targeting moiety in order to prepare the desired linkage of the antibiotic moiety to the targeting molecule through a linker molecule

Preferably, the linker molecule is covalently bonded to T and P and comprises a) a straight or branched C₂-C₁₀alkylene chain, straight or branched C₂-C₁₀alkenylene chain, cycloalkylene or arylene; and b) two or more functional groups selected from the group consisting of -O-, -S-, -C(O)-, -N(R⁴)-, -C(O)N(R⁴)-, -C(O)O-, -C(O)S- and -OC(O)N(R⁴)-, where each R⁴ is independently hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl.

More preferably, the linker molecule is selected from the group consisting of -N(R⁴)-R²-N(R⁴)-, -C(O)N(R⁴)-R²-C(O)N(R⁴), -C(O)O-R²-C(O)N(R⁴), -C(O)S-R²-C(O)N(R⁴), -C(O)N(R⁴)-R²-N(R⁴), -C(O)O-R²-N(R⁴), -C(O)S-R²-N(R⁴), -N(R⁴)C(O)-R²-C(O)N(R⁴)-, and -N(R⁴)-R²-C(O)N(R⁴)-; wherein each R² is independently a straight or branched C₂-C₁₀alkylene chain, straight or branched C₂-C₁₀alkenylene chain, cycloalkylene or arylene; and wherein each R⁴ is independently hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl.

Even more preferably, the linker molecule is -N(R⁴)-R²-N(R⁴)- where each R⁴ is hydrogen and R² is hexylene.

The following diagrams, which are not limiting to the scope of the invention, illustrate various approaches one of ordinary skill in the art can pursue in preparing the antibiotic compositions of the invention wherein the antibiotic moiety is covalently linked to the targeting molecule through either a direct bond or through a linker molecule. In the following diagrams, the targeting moiety is shown as a schematic of nisin[1-12] polypeptide fragment of the invention: 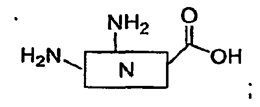 where one of the amino (-NH₂) groups represents the N-terminus of the fragment and the other amino group represents the free amino on the lysine amino acid in the fragment and the carboxy (-C(O)OH) group represents the C-terminus of the fragment. The antibiotic moiety is shown in the following diagrams as a schematic of vancomycin: 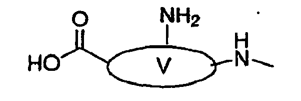 where the carboxy group represents the C-terminus of vancomycin, the amino group represents the vancosamine sugar amine and the methylamino (-N(CH₃)-) group represents the N-terminus of vancomycin. It is understood that other targeting moieties, as set forth herein, and other antibiotic moieties, as set forth herein, may be used in a similar manner as shown below to prepare the antibiotic compositions of the invention. It is also understand that the preparation of antibiotic compositions of the invention wherein a targeting moiety is directly linked to an antibiotic entity through a covalent bond will depend upon the type of the functional group present on the targeting moiety and on the antibiotic moiety, the judicious use of protecting groups on the functional groups and the coupling conditions and reagents required to perfect the linkage, which can easily be ascertained by one skilled in the art by reference to known chemical treatises (see, *e.g.,* March, J., Advanced Organic Chemistry, Reactions, Mechanisms, and Structure (1992), 4th Ed., Wiley, and John Wiley & Sons (1992), and Green, T.W. and P.G.M. Wutz, Protective Groups in Organic Synthesis (1999), 3rd Ed., Wiley.).

In one approach, the C terminus of the targeting moiety may be linked to the C terminus of the antibiotic moiety through a linker molecule of the formula:

H-R¹-R²-R³-H

where R¹ and R³ are each independently -N(R⁴)-, -O- or -S-; each R⁴, if present, is hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl; and R² is straight or branched C₂-C₁₀alkylene chain, straight or branched C₂-C₁₀alkenylene chain, cycloalkylene, or arylene to form the following antibiotic composition of the invention where R¹, R² and R³ are as defined above: 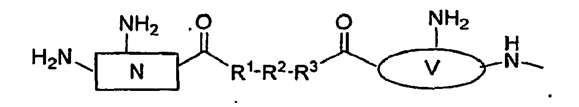 Preferably, the linker molecule is a diaminoalkane of 2 to 10 carbons in length, such as 1,6-diaminohexane. This approach is further exemplified in the Examples below.

In another approach, the C terminus of the targeting moiety may be directly linked to the N-terminus of the antibiotic moiety through an amide bond to prepare the antibiotic compositions of the invention as shown in the diagram below where R is hydrogen or alkyl: 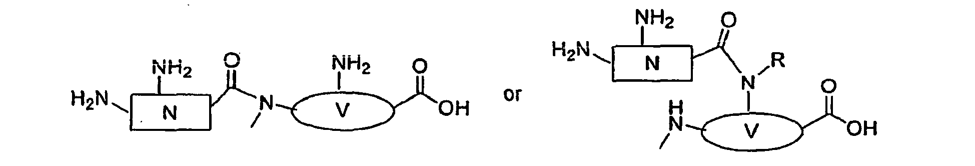 Selectivity of the formation of the amide linkage may be achieved through adjusting reaction pH and judicious use of protecting groups on the functional groups.

The formation of this direct amide linkage is exemplified in the following diagram where Fmoc is 9-fluorenylmethoxycarbonyl:

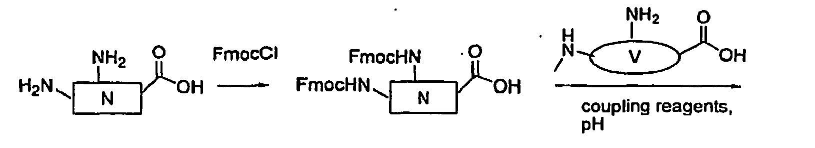

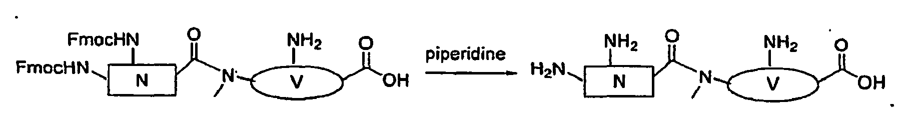

In another approach, the C terminus of the targeting moiety may be directly linked to the N-terminus of the antibiotic moiety through a linker molecule of the formula: 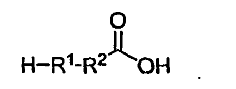 where R¹ is -N(R⁴)-, -O- or -S-; R⁴, if present, is hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl; and R² is straight or branched C₂-C₁₀alkylene chain, straight or branched C₂-C₁₀alkenylene chain, cycloalkylene, or arylene to form the following antibiotic compositions of the invention where each R¹ and R² are as defined above:

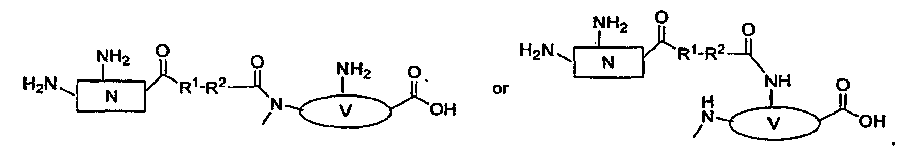

The above antibiotic compositions where R¹ is -N(R⁴)-can be prepared through direct amidation as exemplified in the following diagram where R¹ and R⁴ are as defined above, TFA is trifluoroacetic acid and Fmoc is 9-fluorenylmethoxycarbonyl: 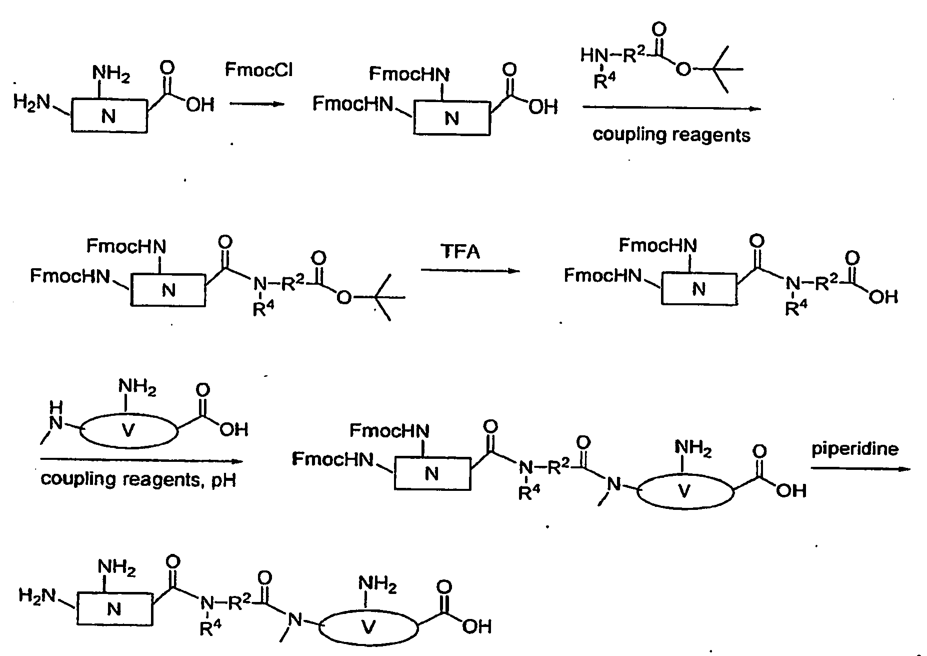 It is understood that corresponding antibiotic compositions where -N(R⁴)- is replaced with -O- or -S- can be prepared in a similar manner by methods known to one skilled in the art.

In another approach, the C terminus of the targeting moiety may be directly linked to the N-terminus of the antibiotic moiety through a linker molecule of the formula: 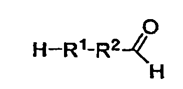 where R¹ is -N(R⁴)-, -O- or -S-; R⁴, if present, is hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl; and R² is straight or branched C₂-C₁₀alkylene chain, straight or branched C₂-C₁₀alkenylene chain, cycloalkylene, or arylene to form the following antibiotic compositions of the invention where each R¹ and R² are as defined above: 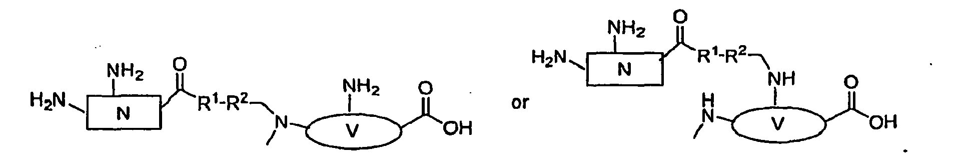 The above antibiotic compositions of the invention where R¹ is -N(R⁴)- can be prepared through reductive amination as exemplified in the following diagram where R¹ and R⁴ are as defined above, TFA is trifluoroacetic acid and Fmoc is 9-fluorenylmethoxycarbonyl (for purposes of convenience only the preparation of only one of the above antibiotic compositions is shown in the following diagram; it is understood that the other antibiotic composition can be similarly prepared using methods known to one skilled in the art):

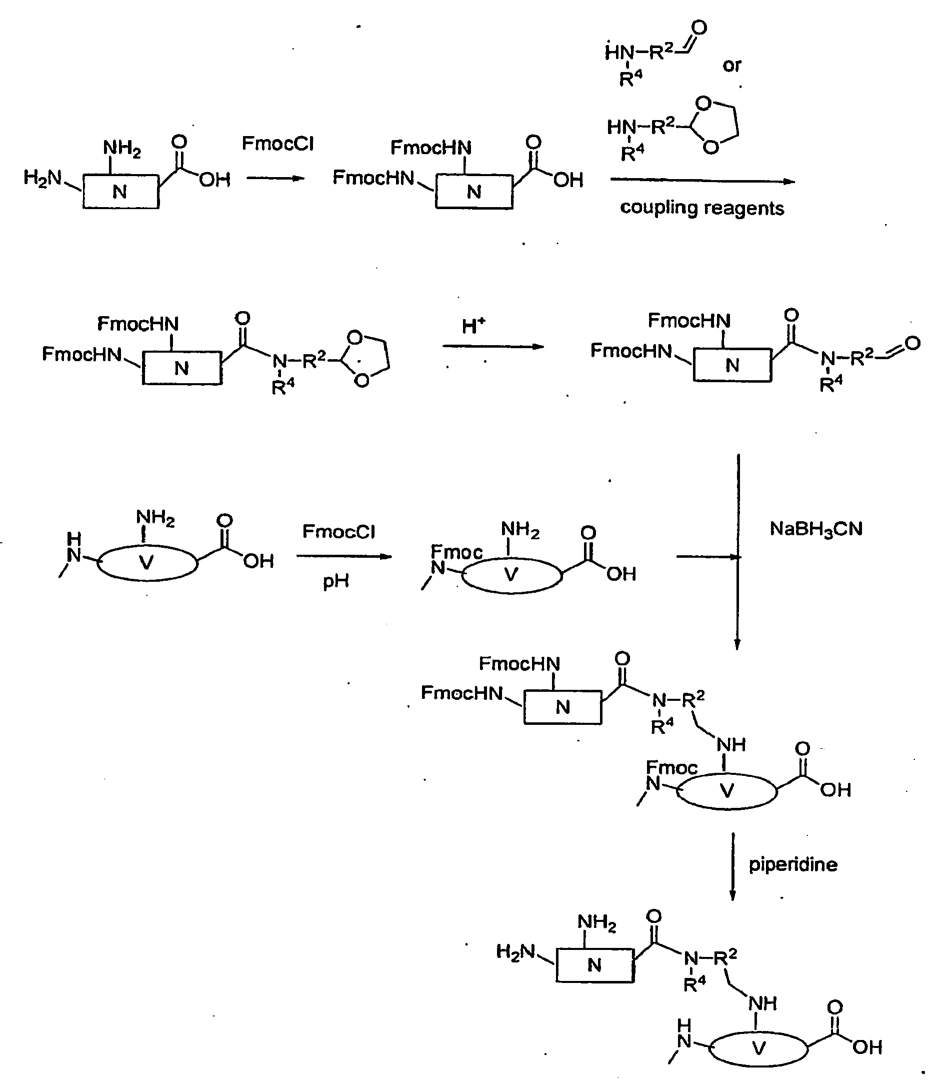

In another approach, the N terminus of the targeting moiety may be directly linked to the C-terminus of the antibiotic moiety through an amide bond producing a mixture of antibiotic compositions of the invention as shown below in the following diagram: 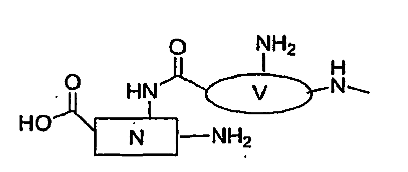 or

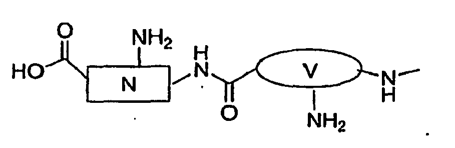

This approach is further exemplified in the Examples below.

In another approach, the N terminus of the targeting moiety may be linked to the C-terminus of the antibiotic moiety through a linker molecule of the formula: 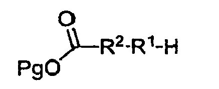 where Pg is an hydroxy protecting group, R¹ is -N(R⁴)-, -O- or -S-; R⁴, if present, is hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl; and R² is straight or branched C₂-C₁₀alkylene chain, straight or branched C₂-C₁₀alkenylene chain, cycloalkylene, or arylene, to form the following antibiotic compositions of the invention where each R¹, R² and R⁴ are as defined above: 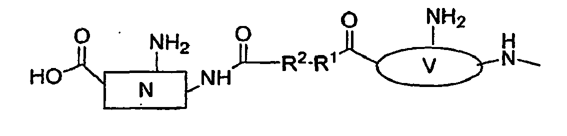 or

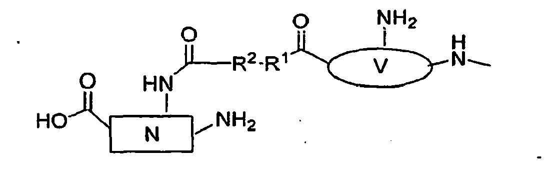

The above antibiotic compositions can be prepared through amidation (or esterification, depending on what R¹ is) as shown below in the following diagram where R¹ and R² are as defined above and Pg is an oxygen-protecting group, such as benzyl:

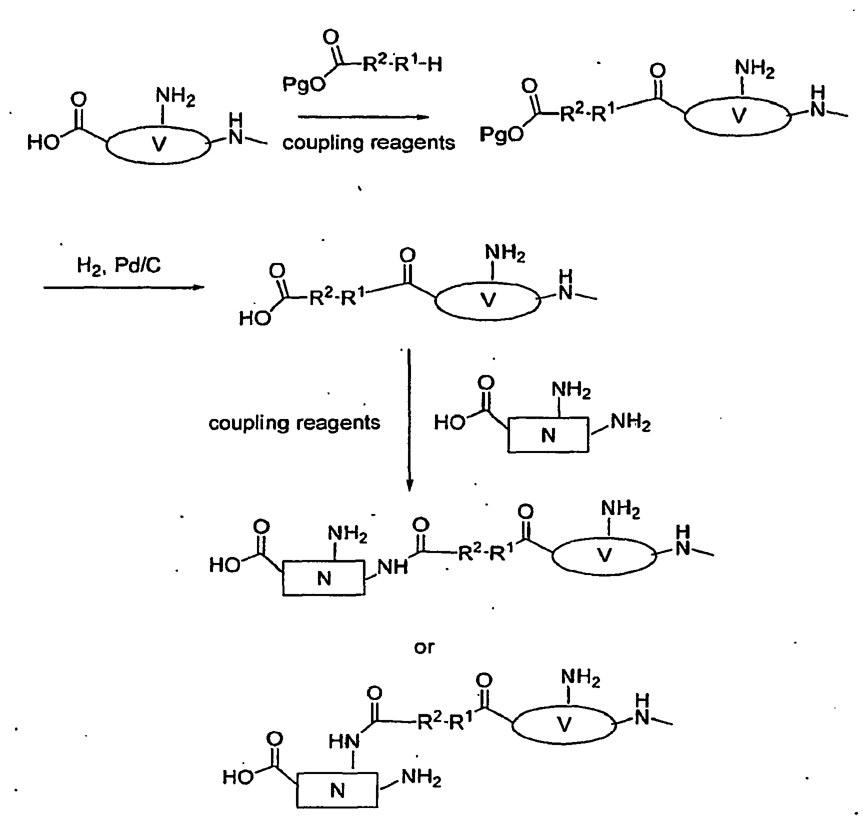

In another approach, the N terminus of the targeting moiety may be linked to the C-terminus of the antibiotic moiety through a linker molecule of the formula: 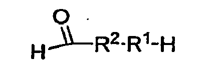 where R¹ is -N(R⁴)-, -O- or -S-; R⁴, if present, is hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl; and R² is straight or branched C₂-C₁₀alkylene chain, straight or branched C₂-C₁₀alkenylene chain, cycloalkylene, or arylene, to form the following antibiotic compositions of the invention where each R¹ and R² are as defined above:

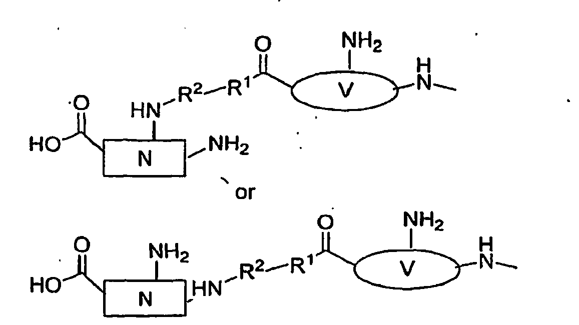

The above antibiotic compositions can be prepared through amidation (or esterification depending on what R¹ is) followed by reductive amination as exemplified in the following diagram where R¹ and R² are as defined above and R' is hydrogen or a nitrogen protecting group, such as Fmoc:

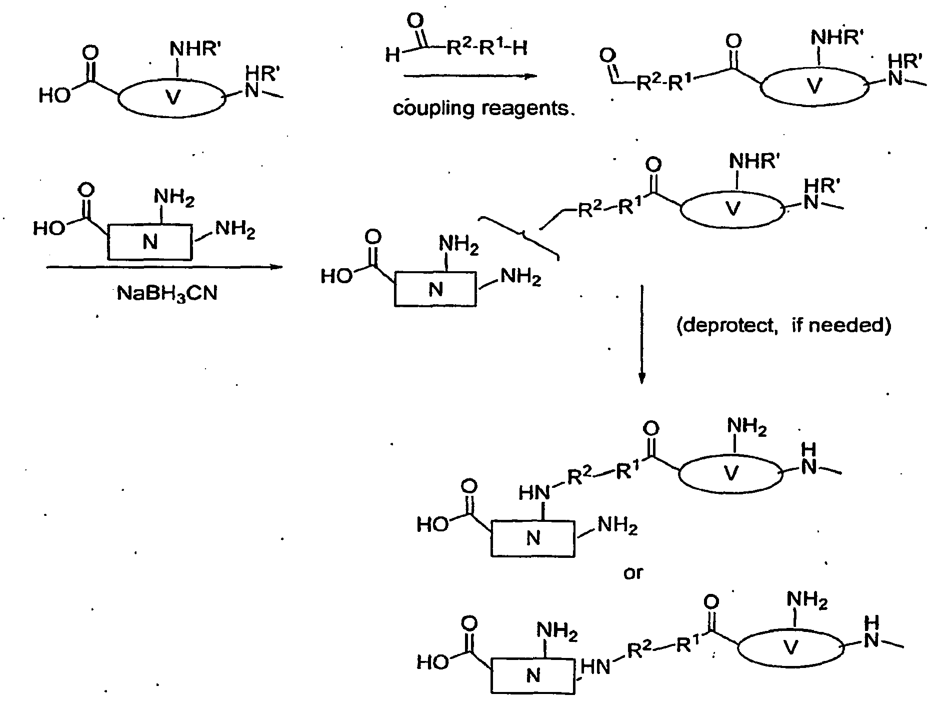

In another approach, the N terminus of the targeting moiety may be linked to the N-terminus of the antibiotic moiety through a linker molecule as exemplified below in the following antibiotic compositions of the invention where each R² is independently a straight or branched C₂-C₁₀alkylene chain, straight or branched C₂-C₁₀alkenylene chain, cycloalkylene, or arylene:

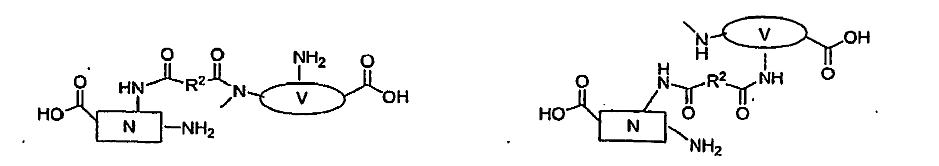

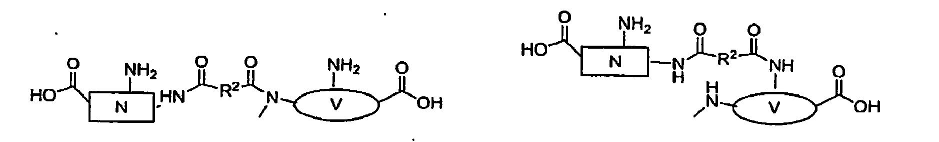

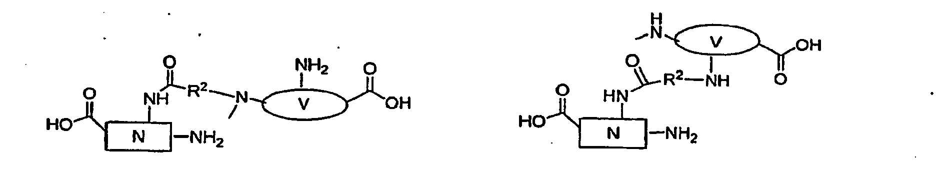

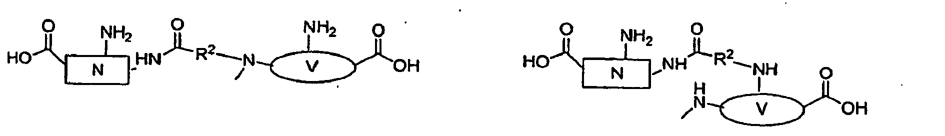

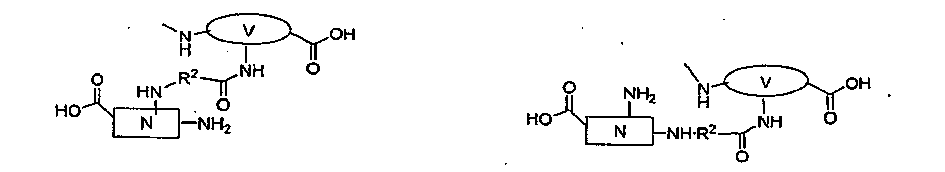

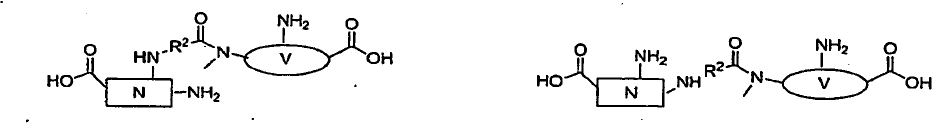

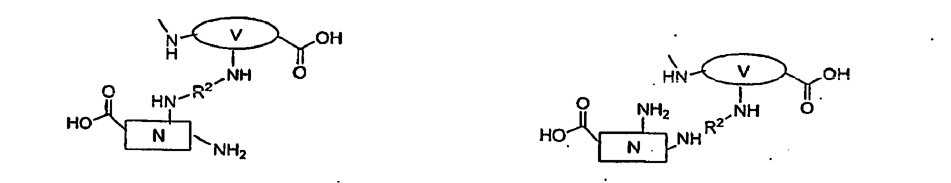

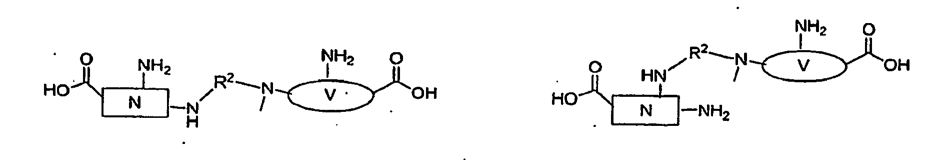

It is understood that other antibiotic compositions of the invention not exemplified above may be prepared by similar methods known to one skilled in the art.

In a preferred embodiment of the invention, the targeting moiety is nisin[1-12] polypetide, which almost completely lacks antibacterial activity, and the antibiotic moiety is vancomycin. Linkage of nisin[1-12] polypeptide fragment to vancomycin may be accomplished through three points of derivatization on each component (at the N-terminus (2), C-terminus (1) and vancosamine sugar amine (3) on vancomyin [see Figure A below] and at the N-terminus, C-terminus and N-epsilon amine of lysine in nisin). In a more preferred embodiment of the invention, this linkage is accomplished through the C-termini of both components and the linker moiety is a symmetrical diamine alkyl group.

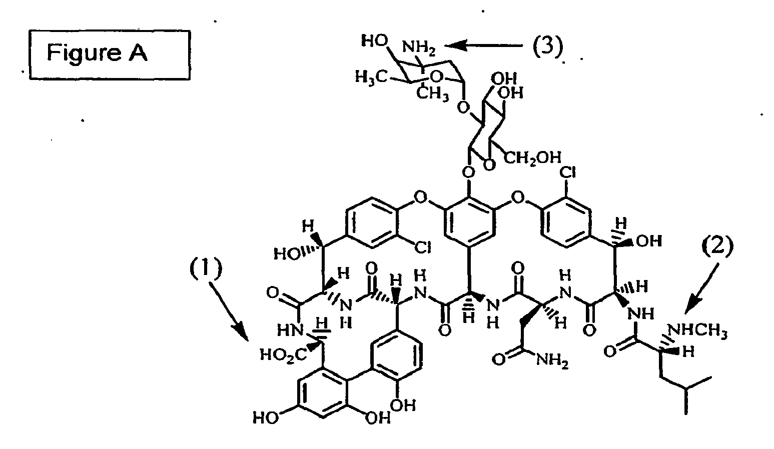

Thus, in one preferred embodiment of the invention, an antibiotic composition of the invention comprises the following structure:

T-L-P,

wherein:
T is a targeting moiety selected from one of the following nisin[1-12] polypeptide fragments: 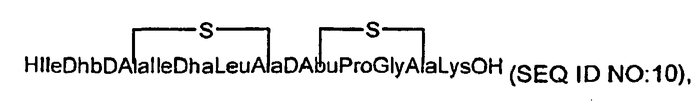 or 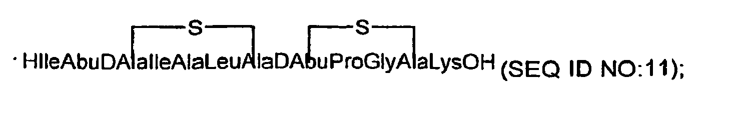
L is -N(R⁴)-R²-N(R⁴)- where each R⁴ is hydrogen and R² is hexylene;
and P is vancomycin or a vancomycin derivative as described above;
or a pharmaceutically acceptable salt thereof.

### Pharmaceutical Compositions of the Invention and Administration

Administration of the antibiotic compositions of the invention, or their pharmaceutically acceptable salts, in pure form or in an appropriate pharmaceutical composition, can be carried out via any of the accepted modes of administration of agents for serving similar utilities. The pharmaceutical compositions of the invention can be prepared by combining an antibiotic composition of the invention with an appropriate pharmaceutically acceptable carrier, diluent or excipient, and may be formulated into preparations in solid, semi-solid, liquid or gaseous forms, such as tablets, capsules, powders, granules, ointments, solutions, suppositories, injections, inhalants, gels, microspheres, and aerosols. Typical routes of administering such pharmaceutical compositions include, without limitation, oral, topical, transdermal, inhalation, parenteral, sublingual, rectal, vaginal, and intranasal. The term parenteral as used herein includes subcutaneous injections, intravenous, intramuscular, intrasternal injection or infusion techniques. Pharmaceutical compositions of the invention are formulated so as to allow the active ingredients contained therein to be bioavailable upon administration of the composition to a patient. Compositions that will be administered to a subject or patient take the form of one or more dosage units, where for example, a tablet may be a single dosage unit, and a container of a compound of the invention in aerosol form may hold a plurality of dosage units. Actual methods of preparing such dosage forms are known, or will be apparent, to those skilled in this art; for example, see *The* Science and Practice of Pharmacy, 20th Edition (Philadelphia College of Pharmacy and Science, 2000). The composition to be administered will, in any event, contain a therapeutically effective amount of a compound of the invention, or a pharmaceutically acceptable salt thereof, for treatment of a disease or condition of interest in accordance with the teachings of this invention.

A pharmaceutical composition of the invention may be in the form of a solid or liquid. In one aspect, the carrier(s) are particulate, so that the compositions are, for example, in tablet or powder form. The carrier(s) may be liquid, with the compositions being, for example, an oral syrup, injectable liquid or an aerosol, which is useful in, for example, inhalatory administration.

When intended for oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavoring agent such as peppermint, methyl salicylate or orange flavoring; and a coloring agent.

When the pharmaceutical composition is in the form of a capsule, for example, a gelatin capsule, it may contain, in addition to materials of the above type, a liquid carrier such as polyethylene glycol or oil.

The pharmaceutical composition may be in the form of a liquid, for example, an elixir, syrup, solution, emulsion or suspension. The liquid may be for oral administration or for delivery by injection, as two examples. When intended for oral administration, preferred compositions contain, in addition to the present compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions of the invention, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

A liquid pharmaceutical composition of the invention intended for either parenteral or oral administration should contain an amount of a compound of the invention such that a suitable dosage will be obtained. Typically, this amount is at least 0.01% of an antibiotic composition of the invention in the composition. When intended for oral administration, this amount may be varied to be between 0.1 and about 70% of the weight of the composition. Preferred oral pharmaceutical compositions contain between about 4% and about 50% of an antibiotic composition of the invention. Preferred pharmaceutical compositions and preparations according to the present invention are prepared so that a parenteral dosage unit contains between 0.01 to 10% by weight of the antibiotic composition prior to dilution of the invention.

The pharmaceutical composition of the invention may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device. Topical formulations may contain a concentration of the antibiotic composition of the invention from about 0.1 to about 10% w/v (weight per unit volume).

The pharmaceutical composition of the invention may be intended for rectal administration, in the form, for example, of a suppository, which will melt in the rectum and release the antibiotic composition. The composition for rectal administration may contain an oleaginous base as a suitable nonirritating excipient. Such bases include, without limitation, lanolin, cocoa butter and polyethylene glycol.

The pharmaceutical composition of the invention may include various materials, which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule.

The pharmaceutical composition of the invention in solid or liquid form may include an agent that binds to the compound of the invention and thereby assists in the delivery of the compound. Suitable agents that may act in this capacity include a monoclonal or polyclonal antibody, a protein or a liposome.

The pharmaceutical composition of the invention may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of antibiotic compositions of the invention may be delivered in single phase, bi-phasic, or tri-phasic systems in order to deliver the active ingredient(s). Delivery of the aerosol includes the necessary container, activators, valves, subcontainers, and the like, which together may form a kit. One skilled in the art, without undue experimentation may determine preferred aerosols.

The pharmaceutical compositions of the invention may be prepared by methodology well known in the pharmaceutical art. For example, a pharmaceutical composition intended to be administered by injection can be prepared by combining an antibiotic composition of the invention with sterile, distilled water so as to form a solution. A surfactant may be added to facilitate the formation of a homogeneous solution or suspension. Surfactants are compounds that non-covalently interact with the antibiotic composition of the invention so as to facilitate dissolution or homogeneous suspension of the compound in the aqueous delivery system.

The antibiotic compositions of the invention, or their pharmaceutically acceptable salts, are administered in a therapeutically effective amount, which will vary depending upon a variety of factors including the activity of the specific antibiotic composition employed; the metabolic stability and length of action of the antibiotic composition; the age, body weight, general health, sex, and diet of the patient; the mode and time of administration; the rate of excretion; the drug combination; the severity of the particular disorder or condition; and the subject undergoing therapy. Generally, a therapeutically effective daily dose is (for a 70 kg mammal) from about 0.001 mg/kg (*i*.*e*., 0.07 mg) to about 100 mg/kg (*i*.*e*., 7.0 g); preferaby a therapeutically effective dose is (for a 70 kg mammal) from about 0.01 mg/kg (*i*.*e*., 7 mg) to about 50 mg/kg (*i*.*e*., 3.5 g); more preferably a therapeutically effective dose is (for a 70 kg mammal) from about 1 mg/kg (*i*.*e*., 70 mg) to about 25 mg/kg (*i*.*e*., 1.75 g).

Antibiotic compositions of the invention, or pharmaceutically acceptable salts thereof, may also be administered simultaneously with, prior to, or after administration of one or more other therapeutic agents. Such combination therapy includes administration of a single pharmaceutical dosage formulation which contains an antibiotic composition of the invention and one or more additional active agents, as well as administration of the antibiotic composition of the invention and each active agent in its own separate pharmaceutical dosage formulation. For example, an antibiotic composition of the invention and the other active agent can be administered to the patient together in a single oral dosage composition such as a tablet or capsule, or each agent administered in separate oral dosage formulations. Where separate dosage formulations are used, the antibiotic compositions of the invention and one or more additional active agents can be administered at essentially the same time, *i*.*e*., concurrently, or at separately staggered times, *i*.*e*., sequentially; combination therapy is understood to include all these regimens.

### Preparation of the Antibiotic Compositions of the Invention

The following specific Examples are provided as a guide to assist in the practice of the invention, and are not intended as a limitation on the scope of the invention.

In general, the antibiotic compositions of the invention can be prepared by methods employing steps known to those skilled in the art or analogous to those steps. General synthetic methods can be found in "Comprehensive Organic Transformations", R.C. Larock, VCH Publishers, New York, N.Y., 1989 and references cited therein.

### EXAMPLE 1

### Synthesis of nisin[1-12]-vancomycin antibiotic composition through a direct bond

Trypsin digests nisin to allow isolation of the fragment of nisin consisting of the first 12 amino acids (nisin[1-12] polypeptide fragment). This nisin[1-12] polypeptide fragment contains lysine at the C-terminus. Nisin[1-12] polypeptide fragment contains two amino groups that are available to react with vancomycin using amide formation conditions to give up to two different antibiotic compositions of the invention. Judicious choice of nitrogen-protecting groups will allow for specific preparation of each of the antibiotic compositions.

In particular, nisin[1-12] polypeptide fragment (8.1 mg, 0.007 mmol.) and vancomycin hydrochloride (10.4 mg, 0.007 mmol) were dissolved in a mixture of DMF/DMSO (1 mL/0.5 mL) at room temperature. HOBt (∼1 mg) and EDC (6.7 mg, 0.035 mmol) were added, followed by DIEA (6.1 µL, 0.035 mmol). The reaction was stirred at room temperature for 24 hours and then treated with small amounts of water. The crude mixture was separated by preparative HPLC (Method A), and the products were pooled and lyophilized. Yield: 1.0 mg (6%, white foam); MS: 2564.3 [M-16+H]⁺, 1282.7 [M-16+2H]²⁺, 855.8 [M-16+3H]³⁺.

### EXAMPLE 2

### Synthesis of nisin[1-12]-vancomycin antibiotic composition through a linker molecule.

The targeting moiety, nisin[1-12] polypeptide fragment can be conjugated to an antibiotic moiety, such as vancomycin, through a linker molecule. One method uses a diamino compound such as 1,6-diaminohexane to which are attached both nisi[1-12] polypeptide fragment and vancomycin via amide couplings. Synthesis of nisin[1-12)]-1,6-diaminohexane-vancomycin is a representative example of this approach.

In general, the two amino groups of nisin[1-12] polypeptide fragment are first protected with Fmoc groups. The protected nisin[1-12] polypeptide fragment is then treated with the linker molecule, mono-trityl-1-6-diaminohexane. In so doing, the C-terminus lysine of the nisin[1-12] polypeptide fragment undergoes an amide formation by reaction of its carboxyl group with mono-trityl-1,6-diaminohexane. The trityl group is then removed from the linker molecule and its free amine is then coupled with the carboxyl group of vancomycin. Removal of the Fmoc groups on the resulting conjugate affords a nisin[1-12]-vancomycin conjugate,an antibiotic composition of the invention.

### A. Synthesis of Fmoc-protected nisin[1-12] polypeptide fragment

To a stirred solution of nisin[1-12] polypeptide fragment (34.5 mg, 0.03 mmol) in DMF (2 mL) at room temperature was added FmocCl (21.7 mg, 0.084 mmol) followed by DIEA (26 µL, 0.15 mmol). The mixture was stirred at room temperature for 4 hours and then the crude product was purified by preparative HPLC (Method B). Yield: 10.4 mg (22%, yellowish glass). 1H NMR (300 MHz, CD₃OD) δ: Fmoc signals: 7.8 (4H, d), 7.7 (4H, m), 7.3 (8H, m).

### B. Coupling of Fmoc-protected nisin[1-12] polypeptide fragment with mono-trityl 1,6-diaminohexane (C₆ linker molecule)

To a stirred solution of Fmoc-protected nisin[1-12] polypeptide fragment (18.6 mg, 0.012 mmol) and mono-trityl 1,6-diaminohexane (10.5 mg, 0.029 mmol) in DMF (3 mL) at room temperature were added HOBt (4 mg, 0.029 mmol) and EDC (22.4 mg, 0.12 mmol). The mixture was stirred for 66 hours and then the crude product was purified by preparative HPLC (Method B). Yield: 10 mg (44%). 1H NMR showed both Fmoc and trityl signals between 7.8 and 7.2 ppm.

### C. Cleavage of trityl protecting group from the C₆ linker molecule

The product from Paragraph B above (10 mg + 3.4 mg from another batch) was treated with a mixture of TFA/water/triisopropylsilane (TIPS) (9:0.5:0.5, 2 mL) at room temperature for 1 hour. The solvents were removed by rotary evaporation and the residue was purified by preparative HPLC to provide Fmoc-protected nisin[1-12]-diaminohexane. Yield: 4.7 mg (40%). 1H NMR showed disappearance of trityl signals.

### D. Coupling of Fmoc-protected nisin[1-12]-diaminohexane with vancomycin

The product from Paragraph C above (4.7 mg, 0.028 mmol) was dissolved in DMF/DMSO (1 mL/0.5 mL) and to the resulting solution were added vancomycin hydrochloride (7.2 mg, 0.0048 mmol), HOBt (1 mg), EDC (3.1 mg, 0.016 mmol), and DIEA (1.1 µL, 0.0063 mmol). The mixture was stirred at room temperature for 1 day and additional vancomycin (3.6 mg, 0.0024 mmol), EDC (3.1 mg, 0.016 mmol) were added. The reaction was continued for another day and then treated with small amounts of water. The crude mixture was purified by preparative HPLC to provide Fmoc-protected nisin[1-12]-diaminohexane-vancomycin: Yield: 5.4 mg (62%). 1H NMR showed both Fmoc Nisin (1-12) and vancomycin signals.

### E. Cleavage of Fmoc protecting groups

The product from paragraph above (5.4 mg, 0.0017 mmol) was treated with 20% piperidine in DMF (1 mL) at room temperature for 20 minutes. HOAc (∼0.2 mL) and small amounts of water were added carefully while stirring. The crude mixture was purified by preparative HPLC (Method D). Yield: 1.1 mg (24%). 1H NMR showed both nisin (1-12) and vancomycin signals. MS: 1340.4 [M+2H]²⁺.

### EXAMPLE 3

### Purification of Nisin

Commercial nisin (Nutrition 21 Inc., NY, USA, Batch# 84FC) contained approximately 80% nisin by weight. Impurity salts were removed by preparative HPLC.

Commercial nisin (518 mg) was dissolved in 27% (v/v) ACN/water with 0.1% (v/v) TFA (10 mL) and purified by preparative HPLC (254 nm detection, SunFire Prep C18 OBD 5µm 30 x 100 mm), making five injections. Product was collected from 4.3 to 5.8 minutes, concentrated by rotary evaporation and lyophilized. Yield: 470 mg (fluffy white solid). The minimum inhibitory concentration of nisin was 0.3-0.6 µg/ml against MRSA and 5-10 µg/ml against VRE.

### HPLC Method

Solvent A = 0.1% (v/v) TFA in water
Solvent B = 0.1% (v/v) TFA in 9:1 ACN/water
Gradient changes are linear over the times indicated.
Flow rate: 40 mL/min.
Profile: 30%B for 3 min., 30 to 50%B over 1 min., 50%B for 3 min., 50 to 30%B over 1 min., 30%B for 4 min.

### EXAMPLE 4

### Preparation and Purification of Nisin[1-12] Polypeptide Fragment

### A. Preparation of Digestion Buffer

Sodium acetate (1.41 g), Trizma base (402 mg) and CaCl₂·2H₂O (407 mg) were dissolved in water (550 mL) and the solution was adjusted to pH 7.0 with aqueous HCl and aqueous NaOH to give digestion buffer (25 mM sodium acetate, 6 mM Tris acetate, 5 mM CaCl₂).

### B. Preparation of Trypsin Solution (LNB CH104-86)

Trypsin (125 mg) was dissolved in digestion buffer (25 mL) to give trypsin solution (5 mg/mL).

### C. Tryptic Digestion of Nisin to Nisin[1-12] polypeptide fragment

Two methods were employed for tryptic digestion of nisin.

### 1. Method A

Nisin (625 mg, ∼80 wt% nisin, ∼150 µmol, 1 eq.) was dissolved in digestion buffer (500 mL) then 0.5 M aqueous NaOH (1.19 mL, 596 µmol, 4 eq.) was added to give pH 7. Trypsin solution (10 mL, 50 mg, 10 wt%) was added and the mixture was incubated at 30°C for 6 days. Additional trypsin (5 mL each day, 25 mg, 5 wt%) was added at 1, 2 and 3 days.

The mixture was concentrated by rotovap to approximately 60 mL volume. Addition of trifluoroacetic acid (TFA) (1 mL) gave pH 4 and helped dissolve some precipitate. The product was purified by preparative HPLC (215 nm detection, Phenomenex Jupiter 10 µm C18 300Å, 250 x 21.20 mm). Reaction mixture (1.7 mL) was diluted to 3.0 mL with water, filtered (0.45 µm Nylon, 25 mm syringe filter) then injected. Product was collected from approximately 16.25 to 17.5 minutes, concentrated and lyophilized. Analytical HPLC (APT method 1, 210 nm detection, Waters XTerra RP18 3.5 µm 4.6 x 150 mm) indicated 98% purity. Yield: 158 mg (white solid). MS: 1150.8 [M+H]⁺, 575.9 [M+2H]²⁺.

### 2. Method B

Nisin (625 mg, ∼80 wt% nisin, ∼150 µmol, 1 eq.) was dissolved in digestion buffer that was not pH-adjusted (100 mL). Trypsin solution (10 mL, 50 mg, 10 wt%) was added and the mixture was adjusted to pH 7.0 with HCl_{(aq)} then incubated at 30°C for 7 days. Additional trypsin (5 mL each day, 25 mg, 5 wt%) was added at 1, 2 and 3 days.

Addition of TFA (1 mL) gave pH 1 and helped dissolve some precipitate. The product was purified by preparative HPLC (215 nm detection, Phenomenex Jupiter 10 µm C18 3001Å, 250 x 21.20 mm), loading 4 mL reaction mixture per injection. Nisin[1-12] polypeptide fragment was collected from approximately 16.25 to 17.25 minutes, concentrated and lyophilized. Analytical HPLC (215 nm detection, Waters XTerra MS C18 3.5 µm 4.6 x 150 mm) indicated 99% purity. Yield: 186 mg (white solid). MS: 1150.8 [M+H]⁺, 576.1 [M+2H]²⁺. Nisin[1-12] polypeptide fragment did not inhibit either MRSA nor VRE at as high as 10 or 40 µg/ml, respectively.

### EXAMPLE 5

### Antimicrobial Activity Assay

Antibiotic compositions of the invention were tested for antimicrobial activity against methicillin resistant *Staphylococcus aureus* (MRSA) ATCC#33591 and vancomycin resistant *Enterococcus faecalis* (VRE) ATCC#51575. Pathogen cultures were grown in tryptic soya broth (TSB) with 5 g/L NaCl (TSB+) overnight (∼18 hrs) at 37°C. The cultures were removed from the incubator and refrigerated at least 3 hours to stop further culture growth. Determined aliquot ratios of culture to broth to obtain approximately 2.5x10⁵ CFU/ml., for which accurate counts were measured by serial dilution onto Tryptic Soy Agar with 5 g/L NaCl (TSA+) plates. Two fold dilution series' of 0.005-10 and 0.005-40 µg/ml were prepared for MRSA and VRE, respectively. All components except thiazolyl blue tetrazolium bromide (MTT) were added to the wells prior to an initial overnight incubation at 37°C. MTT was added to each well, 29 µl or 37 µl for MRSA or VRE plates, respectively, followed by 1 hour incubation at 37°C. Vancomycin and/or Zyvoxam were included as reference compounds with vancomycin prepared in the same way as the test compounds. Zyvoxam was supplied as a 2 mg/ml solution. Minimum inhibitory concentration (MIC) endpoints were read as the lowest concentration showing no visible growth.

The minimum inhibitory concentration of zyvoxam, when tested in this assay, was 0.3 µg/mL against MRSA and 1.2 µg/mL against VRE. The minimum inhibitory concentration of vancomycin, when tested in this assay, was 1.2 µg/mL against MRSA and 500 µg/mL against VRE.

The minimum inhibitory concentration of the nisin[1-12]-vancomycin antibiotic composition prepared in Example 1, when tested in this assay, was 5.0 µg/mL against MRSA and 40 µg/mL against VRE.

The minimum inhibitory concentration of nisin(1-12)-1,6-diaminohexane-vancomycin conjugate prepared in Example 2, when tested in this assay, was 0.04 µg/mL against MRSA and 20 µg/mL against VRE.

Although the foregoing invention has been described in some detail to facilitate understanding, it will be apparent that certain changes and modifications may be practiced within the scope of the appended claims. Accordingly, the described embodiments are to be considered as illustrative and not restrictive, and the invention is not to be limited to the details given herein, but may be modified within the scope and equivalents of the appended claims.

## Claims

1. An antibiotic composition comprised of the following structure:
T-L-P
wherein:
(a) T is a targeting moiety comprising a N-terminal peptide fragment of a lantibiotic where the peptide fragment comprises the following formula:
X₁X₂A,X₃X₄X₅A₂A₃PGA₄X₆ (SEQ ID NO:6)
wherein
A₁, A₂ and A₄ are alanine and A₃ is α-aminobutyric acid, and wherein A₁ and A₂ together form a lanthionine linkage and A₃ and A₄ together form a β-methyllanthionine linkage;
X₁, X₂, X₃, X₄, X₅ and X₆ are each independently selected from any natural or non-natural amino acid,
and wherein T is capable of binding interactions with a pyrophosphate of bacterial cell wall precursor Lipid II wherein said Lipid II comprises undecaprenyl-pyrophsophoryl-MurNAc-(pentapeptide)-GlcNAc;
(b) L is a linker moiety selected from the group consisting of (i) a direct covalent bond linkage between T and P and (ii) a linker molecule covalently bonded to T and P; and
(c) P is an antibiotic moiety that is capable of altering at least one activity of a bacterial membrane or a bacterial cell wall;
or a pharmaceutically acceptable salt thereof.

2. An antibiotic composition according to claim 1 wherein T comprises a N-terminal peptide fragment of a lantibiotic where the peptide fragment comprises the following formula:
X₁X₂A₁X₃X₄X₅A₂A₃PGA₄X₆ (SEQ ID Nos. 1-4)
wherein
X₁ is selected from F, I, V and W,
X₂ is selected from A, K, α-aminobutyric acid and dehydrobutyrine,
A₁, A₂ and A₄ are alanine and A₃ is α-aminobutyric acid,
X₃ is selected from E, I, K and W,
X₄ is selected from F, alanine and dehydroalanine,
X₅ is selected from I, F and L,
X₆ is selected from A, K, V, AX₇, KX₇ and VX₇, wherein X₇ is selected from A, G, I, L, M, P and V; and,
wherein A₁ and A₂ together form a lanthionine linkage and A₃ and A₄ together form a β-methyllanthionine linkage.

3. An antibiotic composition according to claim 1 wherein T comprises a N-terminal peptide fragment of a lantibiotic where the peptide fragment comprises the following formula:
X₁X₂A₁X₃X₄X₅A₂A₃PGA₄X₆ (SEQ ID NO:5)
wherein
X₁ is selected from I, L, F, V, A and G,
X₂ is selected from G, A, V, L, I, F, α-aminobutyric acid and dehydrobutyrine,
A₁, A₂ and A₄ are alanine and A₃ is α-aminobutyric acid,
X₃ is selected from I, L, F, V, A and G,
X₄ is selected from A, G, V, L, I, F and dehydroalanine,
X₅ is selected from L, I, F, V, A, G,
X₆ is selected from K, G, A, V, N, Q, R, H; and,
wherein A₁ and A₂ together form a lanthionine linkage and A₃ and A₄ together form a β-methyllanthionine linkage.

4. An antibiotic composition according to claim 2 or claim 3 wherein T comprises a N-terminal peptide fragment of a lantibiotic where the peptide fragment comprises the following formula:
X₁X₂A₁X₃X₄X₅A₂A₃PGA₄X₆ (SEQ ID NO:7)
wherein
X₁ is I,
X₂ is selected from A, K, α-aminobutyric acid and dehydrobutyrine,
A₁, A₂ and A₄ are alanine and A₃ is α-aminobutyric acid,
X₃ is I,
X₄ is selected from F, alanine and dehydroalanine,
X₅ is L,
X₆ is K; and,
wherein A₁ and A₂ together form a lanthionine linkage and A₃ and A₄ together form a β-methyllanthionine linkage.

5. An antibiotic composition according to claim 4 wherein T comprises a nisin[1-12] polypeptide fragment having a lanthionine ring structure and a β-methyllanthionine ring structure.

6. An antibiotic composition according to claim 1 or claim 2 wherein T comprises a polypeptide homologue of nisin[1-12] polypeptide fragment, said polypeptide homologue having lanthionine ring structure and a β-methyllanthionine ring structure and being selected from the group consisting of a subtilin[1-12] polypeptide fragment, an epidermin polypeptide fragment, a (I1V 16L) epidermin polypeptide fragment, a mutacin B-Ny266 polypeptide fragment and a mutacin 1140 polypeptide fragment.

7. The antibiotic composition of any one of claims 1 to claim 6 wherein L comprises a direct covalent bond linkage selected from the group consisting of an amide, ester, ether, thioether, phosphorodiester, thiophosphorodiester, carbonate, carbamate, hydrazone, oxime and amino linkage.

8. The antibiotic composition of any one of claims 1 to claim 6 wherein the linker molecule comprises:
a) a straight or branched C₂-C₁₀alkylene chain, straight or branched C₂-C₁₀alkenylene chain, cycloalkylene or arylene; and
b) two or more functional groups selected from the group consisting of -O-, -S-, -C(O)-, -N(R⁴)-, -C(O)N(R⁴)-, -C(O)O-, -C(O)S- and -OC(O) N(R⁴)-
where each R⁴ is independently hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl.

9. The antibiotic composition of claim 8 wherein the linker molecule is selected from the group consisting
of -N(R⁴)-R²-N(R⁴), -C(O)N(R⁴)-R²-C(O)N(R⁴), -C(O)O-R²-C(O)N(R⁴), -C(O) S-R²-C(O)N(R⁴), -C(O)N(R⁴)-R²-N(R⁴), -C(O)O-R²-N(R⁴), -C(O)S-R²-N(R⁴), -N (R⁴)C(O)-R²-C(O)N(R⁴)-, and -N(R⁴)-R²-C(O)N(R⁴)-;
wherein each R² is independently a straight or branched C₂-C₁₀alkylene chain, straight or branched C₂-C₁₀alkenylene chain, cycloalkylene or arylene; and
wherein each R⁴ is independently hydrogen, alkyl, alkenyl, alkynyl, aryl, aralkyl aralkenyl, aralkynyl, cycloalkyl, cycloalkylalkyl, cycloalkylalkenyl, heterocyclyl, heterocyclylalkyl, heteroaryl, heteroarylalkyl.

10. The antibiotic composition according to claim 9 wherein the linker molecule is -N(R⁴)-R²-N(R⁴)- where each R⁴ is hydrogen and R² is hexylene.

11. The antibiotic composition of any one of claims 1 to 6 wherein P comprises vancomycin or a vancomycin derivative.

12. The antibiotic composition of any one of claims 1 to 5 wherein:
T is a targeting moiety comprising a nisin[1-12] polypeptide fragment having lanthionine ring structure and a β-methyllanthionine ring structure;
L is a linker moiety comprising a linker molecule, wherein the linker molecule forms a first amide linkage to the C-terminus of T and a second amide linkage to a C-terminus of P; and
P comprises vancomycin..

13. The antibiotic composition of any one of the preceding claims wherein P comprises an antibiotic moiety that is capable of altering at least one activity of a bacterial membrane or a bacterial cell wall.

14. The antibiotic composition of claim 13 wherein the antibiotic moiety is selected from the group consisting of vancomycin, a vancomycin derivative, teicoplanin, ramoplanin, oritavancin, dalbavancin, polymyxin, bacitracin, 6-Aminopenicillanic acid, 2-Mercaptopyridine N-oxide, 7-Aminodesacetoxycephalosporanic acid, 4-Bromocalcimycin, A47934, Alamethicin Trichoderma viride, Amoxicillin, Amphotericin B Streptomyces sp. Ampicillin, Calcimycin A23187, Azlocillin, Chlorhexidine, Bacitracin, clotrimazole, Carbenocillin, Colistin, Cefaclor, Econazole, Cefamandole, Hydrocortisone 21-acetate VETRANAL®, Cefamandole nafate, Filipin complex Streptomyces filipinensis, Cefazolin, Gliotoxin from Gliocladium fimbriatum, Cefmetazole, Gramicidin A, Cefoperazone, Gramicidin C, Cefotaxime, Gramicidin from Bacillus aneurinolyticus (Bacillus brevis), Cephalosporin C, Gramicidin from Bacillus brevis, Cephalothin, lonomycin calcium salt Streptomyces conglobatus, Cephapirin, Lasalocid A, Cephradine, Lonomycin A, Cloxacillin, Monensin, Chloroeremoycin, N-(6-Aminohexyl)-5-chloro-1-naphthalenesulfonamide hydrochloride, D-Cycloserine, Narasin from Streptomyces auriofaciens, Dicloxacillin, Nigericin, D-Penicillamine, Nisin, Econazole, Nonactin, Ethambutol dihydrochloride, Nystatin, Lysostaphin from Staphylococcus staphylolyticus, Phenazine methosulfate, Moxalactam, imaricin, Methicillin, Polymyxin B, Nafcillin, DL-Penicillamine, Nikkomycin, Praziquantel, Nikkomycin Z Streptomyces tendae, Salinomycin, Nitrofurantoin, Surfactin, Oxacillin, Valinomycin, Penicillic acid, Penicillin, Phenethicillin, Phenoxymethylpenicillinic acid, Phosphomycin, Pipemidic acid, Piperacillin, Ramoplanin and Ristomycin.

15. A pharmaceutical composition comprising an antibiotic composition according to any one of claims 1-14 and a pharmaceutically acceptable excipient.

16. An antibiotic composition according to any one of claims 1 to 14 for use in a method of treating a bacterial infection in a mammal.

17. An antibiotic composition comprising a plurality of non-identical antibiotic compositions according to any one of claims 1 to 14 wherein the antibiotic compositions have the structure:
T-L-P,
wherein:
T comprises at least one targeting moiety that is the same or different in said non-identical compositions and that is selected from the group consisting of a targeting moiety according to any one or more of claims to 1 to 6;
L comprises at least one linker moiety that is the same or different in said non-identical compositions and that is selected from the group consisting of (i) a direct covalent bond linkage between T and P and (ii) a linker molecule covalently bonded to T and P; and
P comprises at least one antibiotic moiety that is different in said non-identical compositions, each of said antibiotic moieties comprising an antibiotic moiety that is capable of altering at least one activity of a bacterial membrane or a bacterial cell wall;
or a pharmaceutically acceptable salt thereof.

18. The antibiotic composition of claim 17 wherein the antibiotic moiety is selected from the group consisting of vancomycin, a vancomycin derivative, teicoplanin, ramoplanin, oritavancin, dalbavancin, polymyxin, bacitracin, 6-Aminopenicillanic acid, 2-Mercaptopyridine N-oxide, 7-Aminodesacetoxycephalosporanic acid, 4-Bromocalcimycin, A47934, Alamethicin Trichoderma viride, Amoxicillin, Amphotericin B Streptomyces sp. Ampicillin, Calcimycin A23187, Azlocillin, Chlorhexidine, Bacitracin, clotrimazole, Carbenocillin, Colistin, Cefaclor, Econazole, Cefamandole, Hydrocortisone 21-acetate VETRANAL®, Cefamandole nafate, Filipin complex Streptomyces filipinensis, Cefazolin, Gliotoxin from Gliocladium fimbriatum, Cefmetazole, Gramicidin A, Cefoperazone, Gramicidin C, Cefotaxime, Gramicidin from Bacillus aneurinolyticus (Bacillus brevis), Cephalosporin C, Gramicidin from Bacillus brevis, Cephalothin, lonomycin calcium salt Streptomyces conglobatus, Cephapirin, Lasalocid A, Cephradine, Lonomycin A, Cloxacillin, Monensin, Chloroeremoycin, N-(6-Aminohexyl)-5-chloro-1-naphthalenesulfonamide hydrochloride, D-Cycloserine, Narasin from Streptomyces auriofaciens, Dicloxacillin, Nigericin, D-Penicillamine, Nisin, Econazole, Nonactin, Ethambutol dihydrochloride, Nystatin, Lysostaphin from Staphylococcus staphylolyticus, Phenazine methosulfate, Moxalactam, imaricin, Methicillin, Polymyxin B, Nafcillin, DL-Penicillamine, Nikkomycin, Praziquantel, Nikkomycin Z Streptomyces tendae, Salinomycin, Nitrofurantoin, Surfactin, Oxacillin, Valinomycin, Penicillic acid, Penicillin, Phenethicillin, Phenoxymethylpenicillinic acid, Phosphomycin, Pipemidic acid, Piperacillin, Ramoplanin and Ristomycin.

19. The antibiotic composition of claim 17 wherein L is a direct covalent bond linkage between T and P selected from the group consisting of an amide, ester, ether, thioether, phosphorodiester, thiophosphorodiester, carbonate, carbamate, hydrazone, oxime and amino linkage.

20. The antibiotic composition of claim 17 wherein L is a linker molecule covalently bonded to T and P and wherein the linker molecule comprises:
a) a straight or branched C₂-C₁₀alkylene chain, straight or branched C₂-C₁₀alkenylene chain, cycloalkylene or arylene; and
b) two or more functional groups selected from the group consisting of -O-, -S-, -C(O)-, -N(R⁴)-, -C(O)N(R⁴)-, -C(O)O-, -C(O)S- and -OC(O) N(R⁴)-
where each R4 is independently hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl.

21. The antibiotic compositon of claim 20 wherein the linker molecule is selected from the group consisting of -N(R⁴)-R²-N(R⁴)-
, -C(O)N(R⁴)-R²-C(O)N(R⁴), -C(O)O-R²-C(O)N(R⁴), -C(O)S-R²-C(O)N(R⁴), -C( O)N(R⁴)-R²-N(R⁴), -C(O)O-R²-N(R⁴), -C(O)S-R²-N(R⁴), -N(R⁴)C(O)-R²-(O)N( R⁴)-, and -N(R⁴)-R²-C(O)N(R⁴)-;
wherein each R² is independently a straight or branched C₂-C₁₀alkylene chain, straight or branched C₂-C₁₀alkenylene chain, cycloalkylene or arylene; and
wherein each R⁴ is independently hydrogen, alkyl, alkenyl, alkynyl, aryl, aralkyl, aralkenyl, aralkynyl, cycloalkyl, cycloalkylalkyl, cycloalkylalkenyl, heterocyclyl, heterocyclylalkyl, heteroaryl, heteroarylalkyl.

22. The antibiotic composition of claim 21 wherein the linker molecule is -N(R⁴)-R²-N(R⁴)- where each R⁴ is hydrogen and R² is hexylene.

23. An antibiotic composition according to any one of claims 1 to 5 wherein:
T is selected from one of the following nisin[1-12] polypeptide fragments: 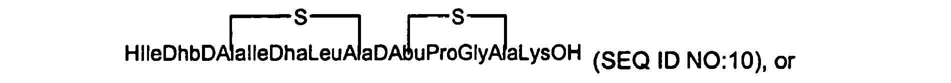 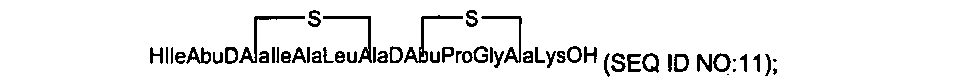
L is -N(R⁴)-R²-N(R⁴)- where each R⁴ is hydrogen and R² is hexylene; and P is vancomycin or a vancomycin derivative.

## Patentansprüche

1. Antibiotische Zusammensetzung, welche die folgende Struktur umfasst:
T-L-P
worin:
(a) T eine Zielgruppierung ist, die ein N-terminales Peptidfragment eines Lantibiotikums umfasst, worin das Peptidfragment die folgende Formel aufweist:
X₁X₂A₁X₃X₄X₅A₂A₃PGA₄X₆ (Seq.-ID Nr. 6)
worin
A₁, A₂ und A₄ Alanin sind und A₃ α-Aminobuttersäure ist und worin A₁ und A₂ zusammen eine Lanthioninbindung bilden und A₃ und A₄ zusammen eine β-Methyllanthioninbindung bilden;
X₁, X₂, X₃, X₄, X₅ und X₆ jeweils unabhängig voneinander aus beliebigen natürlichen oder nichtnatürlichen Aminosäuren ausgewählt sind
und worin T zu Bindungswechselwirkungen mit einem Pyrophosphat des Bakterienzellwandvorläufers Lipid II in der Lage ist, wobei das Lipid II Undecaprenylpyro-phosphoryl-MurNAc-(pentapeptid)-GlcNAc umfasst;
(b) L eine Linkergruppierung ist, die aus der aus (i) einer direkten kovalenten Bindung zwischen T und P und (ii) einem Linkermolekül, das kovalent an T und P gebunden ist, bestehenden Gruppe ausgewählt ist; und
(c) P eine antibiotische Gruppierung ist, die in der Lage ist, zumindest eine Aktivität einer Bakterienmembran oder einer Bakterienzellwand zu verändern;
oder ein pharmazeutisch annehmbares Salz davon.

2. Antibiotische Zusammensetzung nach Anspruch 1, worin T ein N-terminales Peptidfragment eines Lantibiotikums umfasst, worin das Peptidfragment die folgende Formel aufweist:
X₁X₂A₁X₃X₄X₅A₂A₃PGA₄X₆ (Seq.-ID Nr. 1-4)
worin
X₁ aus F, I, V und W ausgewählt ist,
X₂ aus A, K, α-Aminobuttersäure und Dehydrobutyrin ausgewählt ist,
A₁, A₂ und A₄ Alanin sind und A₃ α-Aminobuttersäure ist,
X₃ aus E, I, K und W ausgewählt ist,
X₄ aus F, Alanin und Dehydroalanin ausgewählt ist,
X₅ aus I, F und L ausgewählt ist,
X₆ aus A, K, V, AX₇, KX₇ und VX₇ ausgewählt ist, worin X₇ aus A, G, I, L, M, P und V ausgewählt ist; und
worin A₁ und A₂ zusammen eine Lanthioninbindung bilden und A₃ und A₄ zusammen eine β-Methyllanthioninbindung bilden.

3. Antibiotische Zusammensetzung nach Anspruch 1, worin T ein N-terminales Peptidfragment eines Lantibiotikums umfasst, worin das Peptidfragment die folgende Formel aufweist:
X₁X₂A₁X₃X₄XA₂A₃PGA₄X₆ (Seq.-ID Nr. 5)
worin
X₁ aus I, L, F, V, A und G ausgewählt ist,
X₂ aus G, A, V, L, I, F, α-Aminobuttersäure und Dehydrobutyrin ausgewählt ist,
A₁, A₂ und A₄ Alanin sind und A₃ α-Aminobuttersäure ist,
X₃ aus I, L, F, V, A und G ausgewählt ist,
X₄ aus A, G, V, L, I, F und Dehydroalanin ausgewählt ist,
X₅ aus L, I, F, V, A, G ausgewählt ist,
X₆ aus K, G, A, V, N, Q, R, H ausgewählt ist; und
worin A₁ und A₂ zusammen eine Lanthioninbindung bilden und A₃ und A₄ zusammen eine β-Methyllanthioninbindung bilden.

4. Antibiotische Zusammensetzung nach Anspruch 2 oder 3, worin T ein N-terminales Peptidfragment eines Lantibiotikums umfasst, worin das Peptidfragment die folgende Formel aufweist:
X₁X₂A₁X₃X₄X₅A₂A₃PGA₄X₆ (Seq.-ID Nr. 7)
worin
X₁ I ist,
X₂ aus A, K, α-Aminobuttersäure und Dehydrobutyrin ausgewählt ist,
A₁, A₂ und A₄ Alanin sind und A₃ α-Aminobuttersäure ist,
X₃ I ist,
X₄ aus F, Alanin und Dehydroalanin ausgewählt ist,
X₅ L ist,
X₆ K ist; und
worin A₁ und A₂ zusammen eine Lanthioninbindung bilden und A₃ und A₄ zusammen eine β-Methyllanthioninbindung bilden.

5. Antibiotische Zusammensetzung nach Anspruch 4, worin T ein Nisin[1-12]-Poly-peptidfragment umfasst, das eine Lanthioninringstruktur und eine β-Methyllanthioninringstruktur aufweist.

6. Antibiotische Zusammensetzung nach Anspruch 1 oder Anspruch 2, worin T ein Polypeptidhomolog eines Nisin[1-12]-Polypeptidfragments umfasst, wobei das Polypeptidhomolog eine Lanthioninringstruktur und eine β-Methyllanthioninringstruktur umfasst und aus der aus einem Subtilin[1-12]-Polypeptidfragment, einem Epiderminpolypeptidfragment, einem (I1V 16L)-Epiderminpolypeptidfragment, einem Mutacin-B-Ny266-Polypeptidfragment und einem Mutacin-1140-Polypeptidfragment bestehenden Gruppe ausgewählt ist.

7. Antibiotische Zusammensetzung nach einem der Ansprüche 1 bis 6, worin L eine direkte kovalente Bindung umfasst, die aus der aus einer Amid-, Ester-, Ether-, Thioether-, Phosphordiester-, Thiophosphordiester-, Carbonat-, Carbamat-, Hydrazon-, Oxim- und Aminobindung bestehenden Gruppe ausgewählt ist.

8. Antibiotische Zusammensetzung nach einem der Ansprüche 1 bis 6, worin das Linkermolekül Folgendes umfasst:
a) eine unverzweigte oder verzweigte C₂-C₁₀-Alkylenkette, eine unverzweigte oder verzweigte C₂-C₁₀-Alkenylenkette, Cycloalkylen oder Arylen; und
b) zwei oder mehr funktionelle Gruppen, die aus der aus -O-, -S-, -C(O)-, -N(R⁴)-, -C(O)N(R⁴)-, -C(O)O-, -C(O)S- und -OC(O)N(R⁴)- bestehenden Gruppe ausgewählt sind,
worin die R⁴ jeweils unabhängig voneinander Wasserstoff, Alkyl, Alkenyl, Alkinyl, Aryl, Aralkyl, Aralkenyl, Aralkinyl, Cycloalkyl, Cycloalkylalkyl, Cycloalkylalkenyl, Heterocyclyl, Heterocyclylalkyl, Heteroaryl, Heteroarylalkyl sind.

9. Antibiotische Zusammensetzung nach Anspruch 8, worin das Linkermolekül aus der aus Folgendem bestehenden Gruppe ausgewählt ist:
-N(R⁴)-R²-N(R⁴)-, -C(O)N(R⁴)-R²-C(O)N(R⁴), -C(O)O-R²-C(O)N(R⁴), -C(O)S-R²-C(O)N(R⁴), -C(O)N(R⁴)-R²-N(R⁴), -C(O)O-R²-N(R⁴), -C(O)S-R²-N(R⁴), -N(R⁴)C(O)-R²-C(O)N(R⁴)- und -N(R⁴)-R²-C(O)N(R⁴)-_{;}
worin die R² unabhängig voneinander eine unverzweigte oder verzweigte C₂-C₁₀-Alkylenkette, unverzweigte oder verzweigte C₂-C₁₀-Alkenylenkette, Cycloalkylen oder Arylen sind; und
worin die R⁴ unabhängig voneinander Wasserstoff, Alkyl, Alkenyl, Alkinyl, Aryl, Aralkyl, Aralkenyl, Aralkinyl, Cycloalkyl, Cycloalkylalkyl, Cycloalkylalkenyl, Heterocyclyl, Heterocyclylalkyl, Heteroaryl, Heteroarylalkyl sind.

10. Antibiotische Zusammensetzung nach Anspruch 9, worin das Linkermolekül -N(R⁴)-R²-N(R⁴)- ist, worin die R⁴ Wasserstoff sind und R² Hexylen ist.

11. Antibiotische Zusammensetzung nach einem der Ansprüche 1 bis 6, worin P Vancomycin oder ein Vancomycinderivat umfasst.

12. Antibiotische Zusammensetzung nach einem der Ansprüche 1 bis 5, worin:
T eine Zielgruppierung ist, die ein Nisin[1-12]-Polypeptidfragment umfasst, das eine Lanthioninringstruktur und eine β-Methyllanthioninringstruktur aufweist;
L eine Linkergruppierung ist, die ein Linkermolekül umfasst, worin das Linkermolekül eine erste Amidbindung an den C-Terminus von T und eine zweite Amidbindung an den C-Terminus von P bildet; und
P Vancomycin umfasst.

13. Antibiotische Zusammensetzung nach einem der vorangegangenen Ansprüche, worin P eine antibiotische Gruppierung umfasst, die in der Lage ist, zumindest eine Aktivität einer Bakterienmembran oder einer Bakterienzellwand zu verändern.

14. Antibiotische Zusammensetzung nach Anspruch 13, worin die antibiotische Gruppierung aus der aus Vancomycin, einem Vancomycinderivat, Teicoplanin, Ramoplanin, Oritavancin, Dalbavancin, Polymyxin, Bacitracin, 6-Aminopenicillansäure, 2-Mercaptopyridin-N-Oxid, 7-Aminodesacetoxycephalosporansäure, 4-Bromcalcimycin, A47934, Alamethicin Trichoderma viride, Amoxicillin, Amphotericin B Streptomyces sp., Ampicillin, Calcimycin A23187, Azlocillin, Chlorhexidin, Bacitracin, Clotrimazol, Carbenocillin, Colistin, Cefaclor, Econazol, Cefamandol, Hydrocortison-21-Acetat VETRANAL®, Cefamandol-Nafat, Filipin-Komplex Streptomyces filipinensis, Cefazolin, Gliotoxin von Gliocladium fimbriatum, Cefmetazol, Gramicidin A, Cefoperazon, Gramicidin C, Cefotaxim, Gramicidin von Bacillus aneurinolyticus (Bacillus brevis), Cephalosporin C, Gramicidin von Bacillus brevis, Cephalothin, lonomycin-Calciumsalz Streptomyces conglobatus, Cephapirin, Lasalocid A, Cephradin, Lonomycin A, Cloxacillin, Monensin, Chloreremoycin, N-(6-Aminohexyl)-5-chlor-1-naphthalinsulfonamidhydrochlorid, D-Cycloserin, Narasin von Streptomyces auriofaciens, Dicloxacillin, Nigericin, D-Penicillamin, Nisin, Econazol, Nonactin, Ethambutoldihydrochlorid, Nystatin, Lysostaphin von Staphylococcus staphylolyticus, Phenazinmethosulfat, Moxalactam, Imaricin, Methicillin, Polymyxin B, Nafcillin, DL-Penicillamin, Nikkomycin, Praziquantel, Nikkomycin Z Streptomyces tendae, Salinomycin, Nitrofurantoin, Surfactin, Oxacillin, Valinomycin, Penicillinsäure, Penicillin, Phenthicillin, Phenoxymethylpenicillinsäure, Phosphomycin, Pipemidsäure, Piperacillin, Ramoplanin und Ristomycin bestehenden Gruppe ausgewählt ist.

15. Pharmazeutische Zusammensetzung, die eine antibiotische Zusammensetzung nach einem der Ansprüche 1-14 und eine pharmazeutisch annehmbaren Exzipienten umfasst.

16. Antibiotische Zusammensetzung nach einem der Ansprüche 1 bis 14 zur Verwendung in einem Verfahren zur Behandlung einer Bakterieninfektion in einem Säugetier.

17. Antibiotische Zusammensetzung, die mehrere nichtidentische antibiotische Zusammensetzungen nach einem der Ansprüche 1 bis 14 umfasst, wobei die antibiotischen Zusammensetzungen folgende Struktur aufweisen:
T-L-P,
worin:
T zumindest eine Zielgruppierung umfasst, die in den nichtidentischen Zusammensetzungen gleich oder unterschiedlich ist und die aus der Gruppe ausgewählt ist, die aus Zielgruppierungen nach einem oder mehreren der Ansprüche 1 bis 5 besteht;
L zumindest eine Linkergruppierung umfasst, die in den nichtidentischen Zusammensetzungen gleich oder unterschiedlich ist und aus der aus (i) einer direkten kovalenten Bindung zwischen T und P und (ii) einem Linkermolekül, das kovalent an T und P gebunden ist, bestehenden Gruppe ausgewählt ist; und
P zumindest eine antibiotische Gruppierung umfasst, die in den nichtidentischen Zusammensetzungen unterschiedlich ist, wobei jede der antibiotischen Gruppierungen eine antibiotischen Gruppierung umfasst, die in der Lage ist, zumindest eine Aktivität einer Bakterienmembran oder Bakterienzellwand zu verändern;
oder ein pharmazeutisch annehmbares Salz davon.

18. Antibiotische Zusammensetzung nach Anspruch 17, wobei die antibiotische Gruppierung aus der aus Vancomycin, einem Vancomycinderivat, Teicoplanin, Ramoplanin, Oritavancin, Dalbavancin, Polymyxin, Bacitracin, 6-Aminopenicillansäure, 2-Mercaptopyridin-N-Oxid, 7-Aminodesacetoxycephalosporansäure, 4-Bromcalcimycin, A47934, Alamethicin Trichoderma viride, Amoxicillin, Amphotericin B Streptomyces sp., Ampicillin, Calcimycin A23187, Azlocillin, Chlorhexidin, Bacitracin, Clotrimazol, Carbenocillin, Colistin, Cefaclor, Econazol, Cefamandol, Hydrocortison-21-Acetat VETRANAL®, Cefamandol-Nafat, Filipin-Komplex Streptomyces filipinensis, Cefazolin, Gliotoxin von Gliocladium fimbriatum, Cefmetazol, Gramicidin A, Cefoperazon, Gramicidin C, Cefotaxim, Gramicidin von Bacillus aneurinolyticus (Bacillus brevis), Cephalosporin C, Gramicidin von Bacillus brevis, Cephalothin, lonomycin-Calciumsalz Streptomyces conglobatus, Cephapirin, Lasalocid A, Cephradin, Lonomycin A, Cloxacillin, Monensin, Chloreremoycin, N-(6-Aminohexyl)-5-chlor-1-naphthalinsulfonamidhydrochlorid, D-Cycloserin, Narasin von Streptomyces auriofaciens, Dicloxacillin, Nigericin, D-Penicillamin, Nisin, Econazol, Nonactin, Ethambutoldihydrochlorid, Nystatin, Lysostaphin von Staphylococcus staphylolyticus, Phenazinmethosulfat, Moxalactam, Imaricin, Methicillin, Polymyxin B, Nafcillin, DL-Penicillamin, Nikkomycin, Praziquantel, Nikkomycin Z Streptomyces tendae, Salinomycin, Nitrofurantoin, Surfactin, Oxacillin, Valinomycin, Penicillinsäure, Penicillin, Phenthicillin, Phenoxymethylpenicillinsäure, Phosphomycin, Pipemidsäure, Piperacillin, Ramoplanin und Ristomycin bestehenden Gruppe ausgewählt ist.

19. Antibiotische Zusammensetzung nach Anspruch 17, worin L eine direkte kovalente Bindung zwischen T und P ist, die aus der aus einer Amid-, Ester-, Ether-, Thioether-, Phosphordiester-, Thiophosphordiester-, Carbonat-, Carbamat-, Hydrazon-, Oxim- und Aminobindung bestehenden Gruppe ausgewählt ist.

20. Antibiotische Zusammensetzung nach Anspruch 17, worin L ein Linkermolekül ist, das kovalent an T und P gebunden ist und worin das Linkermolekül Folgendes umfasst:
a) eine unverzweigte oder verzweigte C₂-C₁₀-Alkylenkette, eine unverzweigte oder verzweigte C₂-C₁₀-Alkenylenkette, Cycloalkylen oder Arylen; und
b) zwei oder mehr funktionelle Gruppen, die aus der aus -O-, -S-, -C(O)-, -N(R⁴)-, -C(O)N(R⁴)-, -C(O)O-, -C(O)S- und -OC(O)N(R⁴)- bestehenden Gruppe ausgewählt sind,
worin die R⁴ jeweils unabhängig voneinander Wasserstoff, Alkyl, Alkenyl, Alkinyl, Aryl, Aralkyl, Aralkenyl, Aralkinyl, Cycloalkyl, Cycloalkylalkyl, Cycloalkylalkenyl, Heterocyclyl, Heterocyclylalkyl, Heteroaryl, Heteroarylalkyl sind.

21. Antibiotische Zusammensetzung nach Anspruch 20, worin das Linkermolekül aus der aus Folgendem bestehenden Gruppe ausgewählt ist: -N(R⁴)-R²-N(R⁴)-, -C(O)N(R⁴)-R²-C(O)N(R⁴), -C(O)O-R²-C(O)N(R⁴), -C(O)S-R²-C(O)N(R⁴), -C(O)N(R⁴)-R²-N(R⁴), -C(O)O-R²-N(R⁴), -C(O)S-R²-N(R⁴), -N(R⁴)C(O)-R²-C(O)N(R⁴)- und -N(R⁴)-R²-C(O)N(R⁴)-;
worin die R² unabhängig voneinander eine unverzweigte oder verzweigte C₂-C₁₀-Alkylenkette, unverzweigte oder verzweigte C₂-C₁₀-Alkenylenkette, Cycloalkylen oder Arylen sind; und
worin die R⁴ unabhängig voneinander Wasserstoff, Alkyl, Alkenyl, Alkinyl, Aryl, Aralkyl, Aralkenyl, Aralkinyl, Cycloalkyl, Cycloalkylalkyl, Cycloalkylalkenyl, Heterocyclyl, Heterocyclylalkyl, Heteroaryl, Heteroarylalkyl sind.

22. Antibiotische Zusammensetzung nach Anspruch 21, worin das Linkermolekül -N(R⁴)-R²-N(R⁴)- ist, worin die R⁴ Wasserstoff sind und R² Hexylen ist.

23. Antibiotische Zusammensetzung nach einem der Ansprüche 1 bis 5, worin:
T aus einem der folgenden Nisin[1-12]-Polypeptidfragmente ausgewählt ist: 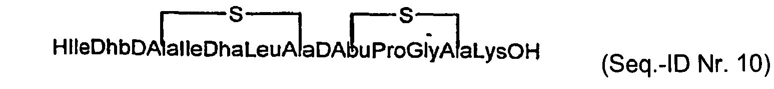 oder 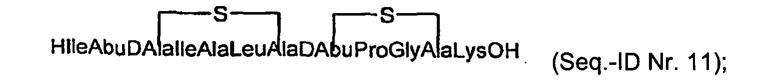
L -N(R⁴)-R²-N(R⁴)- ist, worin die R⁴ Wasserstoff sind und R² Hexylen ist;
und P Vancomycin oder ein Vancomycinderivat ist.

## Revendications

1. Composition antibiotique composée de la structure suivante:
T-L-P
dans laquelle:
(a) T est une fraction ciblante comprenant un fragment de peptide N-terminal d'un lantibiotique où le fragment de peptide comprend la formule suivante:
X₁X₂A₁X₃X₄X₅A₂A₃PGA₄X₆ (SEQ ID NO: 6)
où
A₁, A₂ et A₄ sont l'alanine et A₃ est l'acide α-aminobutyrique, et où A₁ et A₂ forment ensemble une liaison lanthionine et A₃ et A₄ forment ensemble une liaison β-méthyllanthionine;
X₁, X₂, X₃, X₄, X₅ et X₆ sont chacun indépendamment choisis parmi tout acide amino naturel ou non naturel,
et où T est capable d'interactions de liaison avec un pyrophosphate d'un lipide II de précurseur de paroi de cellule bactérienne où ledit lipide II comprend undécaprénylpyrophosphoryl-MurNAc-(pentapeptide)-GlcNAc;
(b) L est une fraction lieuse choisie dans le groupe consistant en (i) une liaison covalente directe entre T et P et (ii) une molécule lieuse liée de manière covalente à T et P; et
(c) P est une fraction antibiotique qui est capable de modifier au moins une activité d'une membrane bactérienne ou d'une paroi de cellule bactérienne;
ou sel pharmaceutiquement acceptable de celle-ci.

2. Composition antibiotique selon la revendication 1, dans laquelle T comprend un fragment de peptide N-terminal d'un lantibiotique où le fragment de peptide comprend la formule suivante:
X₁X₂A₁X₃X₄X₅A₂A₃PGA₄X₆ (SEQ ID Nos. 1 à 4)
dans laquelle
X₁ est choisi parmi F, I, V et W,
X₂ est choisi parmi A, K, l'acide α-aminobutyrique et la déshydrobutyrine,
A₁, A₂ et A₄ sont l'alanine et A₃ est l'acide α-aminobutyrique,
X₃ est choisi parmi E, I, K et W,
X₄ est choisi parmi F, l'alanine et la déshydroalanine,
X₅ est choisi parmi I, F et L,
X₆ est choisi parmi A, K, V, AX₇, KX₇ et VX₇, où X₇ est choisi parmi A, G, I, L, M, P et V; et,
où A₁ et A₂ forment ensemble une liaison lanthionine et A₃ et A₄ forment ensemble une liaison β-méthyllanthionine.

3. Composition antibiotique selon la revendication 1, dans laquelle T comprend un fragment de peptide N-terminal d'un lantibiotique où le fragment de peptide comprend la formule suivante:
X₁X₂A₁X₃X₄X₅A₂A₃PGA₄X₆ (SEQ ID NO: 5)
dans laquelle
X₁ est choisi parmi I, L, F, V, A et G,
X₂ est choisi parmi G, A, V, L, I, F, l'acide α-aminobutyrique et la déshydrobutyrine,
A₁, A₂ et A₄ sont l'alanine et A₃ est l'acide α-aminobutyrique,
X₃ est choisi parmi I, L, F, V, A et G,
X₄ est choisi parmi A, G, V, L, I, F et la déshydroalanine,
X₅ est choisi parmi L, I, F, V, A, G,
X₆ est choisi parmi K, G, A, V, N, Q, R, H; et,
où A₁ et A₂ forment ensemble une liaison lanthionine et A₃ et A₄ forment ensemble une liaison β-méthyllanthionine.

4. Composition antibiotique selon la revendication 2 ou la revendication 3, dans laquelle T comprend un fragment de peptide N-terminal d'un lantibiotique où le fragment de peptide comprend la formule suivante:
X₁X₂A_{I}X₃X₄X₅A₂A₃PGA₄X₆ (SEQ ID NO: 7) dans laquelle
X₁ est I,
X₂ est choisi parmi A, K, l'acide α-aminobutyrique et la déshydrobutyrine,
A₁, A₂ et A₄ sont l'alanine et A₃ l'acide α-aminobutyrique,
X₃ est I,
X₄ est choisi parmi F, l'alanine et la déshydroalanine,
X₅ est L,
X₆ est K; et,
où A₁ et A₂ forment ensemble une liaison lanthionine et A₃ et A₄ forment ensemble une liaison β-méthyllanthionine.

5. Composition antibiotique selon la revendication 4, dans laquelle T comprend un fragment de nisine[1-12] polypeptide ayant une structure de cycle lanthionine et une structure de cycle β-méthyllanthionine.

6. Composition antibiotique selon la revendication 1 ou la revendication 2, dans laquelle T comprend un homologue polypeptide de fragment de nisine[1-12] polypeptide, ledit homologue de polypeptide ayant une structure de cycle lanthionine et une structure de cycle β-méthyllanthionine, et étant choisi dans le groupe consistant en un fragment de subtiline[1-12] polypeptide, un fragment d'épidermine polypeptide, un fragment de (11V 16L) épidermine polypeptide, un fragment de mutacine B-Ny266 polypeptide et un fragment de mutacine 1140 polypeptide.

7. Composition antibiotique selon l'une quelconque des revendications 1 à 6, dans laquelle L comprend une liaison covalente directe choisie dans le groupe consistant en une liaison amide, ester, éther, thioéther, phosphorodiester, thiophosphorodiester, carbonate, carbamate, hydrazone, oxime et amino.

8. Composition antibiotique selon l'une quelconque des revendications 1 à 6, dans laquelle la molécule lieuse comprend:
a) une chaîne alkylène en C₂ à C₁₀ droite ou ramifiée, une chaîne alcénylène en C₂ à C₁₀ droite ou ramifiée, un cycloalkylène ou arylène; et
b) deux groupes fonctionnels ou plus choisis dans le groupe consistant en -O-, -S-, -C(O)-, -N(R⁴)-, -C(O)N(R⁴)-, -C(O)O-, -C(O)S- et -OC(O)N(R⁴)-
où chaque R⁴ est indépendamment un atome d'hydrogène, un groupe alkyle, alcényle, alcynyle, aryle, aralkyle, aralcényle, aralcynyle, cycloalkyle, cycloalkylalkyle, cycloalkylalcényle, hétérocyclyle, hétérocyclylalkyle, hétéroaryle, hétéroarylalkyle.

9. Composition antibiotique selon la revendication 8, dans laquelle la molécule lieuse est choisie dans le groupe consistant en
-N(R⁴)-R²-N(R⁴), -C(O)N(R⁴)-R²-C(O)N(R⁴), -C(O)O-R²-C(O)N(R⁴), -C(O)S-R²-C(O)N(R⁴), -C(O)N(R⁴)-R²-N(R⁴), -C(O)O-R²-N(R⁴), -C(O)S-R²-N(R⁴), -N(R⁴)C(O)-R²-C(O)N(R⁴)-, et -N(R⁴)-R²-C(O)N(R⁴)-;
où chaque R² est indépendamment une chaîne alkylène en C₂ à C₁₀ droite ou ramifiée, une chaîne alcénylène en C₂ à C₁₀ droite ou ramifiée, un cycloalkylène ou arylène; et
où chaque R⁴ est indépendamment un atome d'hydrogène,
un groupe alkyle, alcényle, alcynyle, aryle, aralkyle, aralcényle, aralcynyle, cycloalkyle, cycloalkylalkyle, cycloalkylalcényle, hétérocyclyle, hétérocyclylalkyle, hétéroaryle, hétéroarylalkyle.

10. Composition antibiotique selon la revendication 9, dans laquelle la molécule lieuse est -N(R⁴)-R²-N(R⁴)-où chaque R⁴ est un atome d'hydrogène et R² est un hexylène.

11. Composition antibiotique selon l'une quelconque des revendications 1 à 6, dans laquelle P comprend de la vancomycine ou un dérivé de vancomycine.

12. Composition antibiotique selon l'une quelconque des revendications 1 à 5, dans laquelle:
T est une fraction ciblante comprenant un fragment de nisine[1-12] polypeptide ayant une structure de cycle lanthionine et une structure de cycle β-méthyllanthionine;
L est une fraction lieuse comprenant une molécule lieuse, où la molécule lieuse comprend une première liaison amide à l'extrémité C-terminale de T et une seconde liaison amide à une extrémité C-terminale de P; et
P comprend de la vancomycine.

13. Composition antibiotique selon l'une quelconque des revendications précédentes, dans laquelle P comprend une fraction antibiotique qui est capable de modifier au moins une activité d'une membrane bactérienne ou d'une paroi de cellule bactérienne.

14. Composition antibiotique selon la revendication 13, dans laquelle la fraction antibiotique est choisie dans le groupe consistant en la vancomycine, un dérivé de vancomycine, la téicoplanine, la ramoplanine, l'oritavancine, la dalbavancine, la polymyxine, la bacitracine, l'acide 6-aminopénicillanique, le N-oxyde de 2-mercaptopyridine, l'acide 7-aminodésacétoxycéphalosporanique, la 4-bromocalcimycine, A47934, l'alaméthicine trichoderma viride, l'amoxicilline, l'amphotéricine B streptomyces sp. ampicilline, la calcimycine A23187, l'azlocilline, la chlorhexidine, la bacitracine, le clotrimazole, la carbénocilline, la colistine, le céfaclor, l'éconazole, le céfamandole, le 21-acétate d'hydrocortisone VETRANAL^{®}, le nafate de céfamandole, le complexe de filipine streptomyces filipinensis, la céfazoline, la gliotoxine issue de gliocladium fimbriatum, le Céfmétazole, la gramicidine A, le céfopérazone, la gramicidine C, le céfotaxime, la gramicidine issue de bacillus aneurinolyticus (bacillus brevis), la céphalosporine C, la Gramicidine issue de bacillus brevis, la Céphalothine, le sel de calcium d'ionomycine streptomyces conglobatus, la céphapirine, le lasalocide A, la céphradine, la lonomycine A, la cloxacilline, la monensine, la chloroérémoycine, le chlorhydrate de N-(6-aminohexyl)-5-chloro-1-naphtalènesulfonamide, la D-cyclosérine, la narasine issue de streptomyces auriofaciens, la dicloxacilline, la nigéricine, la D-pénicillamine, la nisine, l'éconazole, la nonactine, le dichlorhydrate d'éthambutol, la nystatine, la lysostaphine issue de staphylococcus staphylolyticus, le méthosulfate de phénazine, le moxalactame, l'imaricine, la méthicilline, la polymyxine B, la nafcilline, la DL-pénicillamine, la nikkomycine, le Praziquantel, la nikkomycine Z streptomyces tendae, la salinomycine, la nitrofurantoïne, la surfactine, l'oxacilline, la valinomycine, l'acide pénicillique, la pénicilline, la phénéthicilline, l'acide phénoxyméthylpénicillinique, la phosphomycine, l'acide pipémidique, la pipéracilline, la ramoplanine et la ristomycine.

15. Composition pharmaceutique comprenant une composition antibiotique selon l'une quelconque des revendications 1 à 14, et un excipient pharmaceutiquement acceptable.

16. Composition antibiotique selon l'une quelconque des revendications 1 à 14, à utiliser dans un procédé de traitement d'une infection bactérienne chez un mammifère.

17. Composition antibiotique comprenant une pluralité de compositions antibiotiques non identiques selon l'une quelconque des revendications 1 à 14, où les compositions antibiotiques présentent la structure:
T-L-P
dans laquelle:
T comprend au moins une fraction ciblante qui sont identiques ou différentes dans lesdites compositions non identiques et qui est choisie dans le groupe consistant une fraction ciblante selon l'une quelconque des revendications 1 à 6;
L comprend au moins une fraction lieuse qui sont identiques ou différentes dans lesdites compositions non identiques et qui est choisie dans le groupe consistant en (i) une liaison covalente directe entre T et P et (ii) une molécule lieuse liée de manière covalente à T et P; et
P comprend au moins une fraction antibiotique qui sont différentes dans lesdites compositions non identiques, chacune desdites fractions antibiotiques comprenant une fraction antibiotique qui est capable de modifier au moins une activité d'une membrane bactérienne ou d'une paroi de cellule bactérienne;
ou sel pharmaceutiquement acceptable de celle-ci.

18. Composition antibiotique selon la revendication 17, dans laquelle la fraction antibiotique est choisie dans le groupe consistant en la vancomycine, un dérivé de vancomycine, la téicoplanine, la ramoplanine, l'oritavancine, la dalbavancine, la polymyxine, la bacitracine, l'acide 6-aminopénicillanique, le N-oxyde de 2-mercaptopyridine, l'acide 7-aminodésacétoxycéphalosporanique, la 4-bromocalcimycine, A47934, l'alaméthicine trichoderma viride, l'amoxicilline, l'amphotéricine B streptomyces sp. ampicilline, la calcimycine A23187, l'azlocilline, la chlorhexidine, la bacitracine, le clotrimazole, la carbénocilline, la colistine, le céfaclor, l'éconazole, le céfamandole, le 21-acétate d'hydrocortisone VETRANAL^{®}, le nafate de céfamandole, le complexe de filipine streptomyces filipinensis, la céfazoline, la gliotoxine issue de gliocladium fimbriatum, le Céfmétazole, la gramicidine A, le céfopérazone, la gramicidine C, le céfotaxime, la gramicidine issue de bacillus aneurinolyticus (bacillus brevis), la céphalosporine C, la Gramicidine issue de bacillus brevis, la Céphalothine, le sel de calcium d'ionomycine streptomyces conglobatus, la céphapirine, le lasalocide A, la céphradine, la lonomycine A, la cloxacilline, la monensine, la chloroérémoycine, le chlorhydrate de N-(6-aminohexyl)-5-chloro-1-naphtalènesulfonamide, la D-cyclosérine, la narasine issue de streptomyces auriofaciens, la dicloxacilline, la nigéricine, la D-pénicillamine, la nisine, l'éconazole, la nonactine, le dichlorhydrate d'éthambutol, la nystatine, la lysostaphine issue de staphylococcus staphylolyticus, le méthosulfate de phénazine, le moxalactame, l'imaricine, la méthicilline, la polymyxine B, la nafcilline, la DL-pénicillamine, la nikkomycine, le Praziquantel, la nikkomycine Z streptomyces tendae, la salinomycine, la nitrofurantoïne, la surfactine, l'oxacilline, la valinomycine, l'acide pénicillique, la pénicilline, la phénéthicilline, l'acide phénoxyméthylpénicillinique, la phosphomycine, l'acide pipémidique, la pipéracilline, la ramoplanine et la ristomycine.

19. Composition antibiotique selon la revendication 17, dans laquelle L est une liaison covalente directe entre T et P choisie dans le groupe consistant en une liaison amide, ester, éther, thioéther, phosphorodiester, thiophosphorodiester, carbonate, carbamate, hydrazone, oxime et amino.

20. Composition antibiotique selon la revendication 17, dans laquelle L est une molécule lieuse liée de manière covalente à T et P et où la molécule lieuse comprend:
a) une chaîne alkylène en C₂ à C₁₀ droite ou ramifiée, une chaîne alcénylène en C₂ à C₁₀ droite ou ramifiée, un cycloalkylène ou arylène; et
b) deux groupes fonctionnels ou plus choisis dans le groupe consistant en -O-, -S-, -C(O)-, -N(R⁴)-, -C(O)N(R⁴)-, -C(O)O-, -C(O)S- et -OC(O)N(R⁴)-
où chaque R⁴ est indépendamment un atome d'hydrogène, un groupe alkyle, alcényle, alcynyle, aryle, aralkyle, aralcényle, aralcynyle, cycloalkyle, cycloalkylalkyle, cycloalkylalcényle, hétérocyclyle, hétérocyclylalkyle, hétéroaryle, hétéroarylalkyle.

21. Composition antibiotique selon la revendication 20, dans lequel la molécule lieuse est choisie dans le groupe consistant en
-N(R⁴)-R²-N(R⁴)-, -C(O)N(R⁴)-R²-C(O)N(R⁴), -C(O)O-R²-C(O)N(R⁴), -C(O)S-R²-C(O)N(R⁴), -C(O)N(R⁴)-R²-N(R⁴),-C(O)O-R²-N(R⁴), -C(O)S-R²-N(R⁴), -N(R⁴)C(O)-R²-C(O)N(R⁴)-, et -N(R⁴)-R²-C(O)N(R⁴)-;
où chaque R² est indépendamment une chaîne alkylène en C₂ à C₁₀ droite ou ramifiée, une chaîne alcénylène en C₂ à C₁₀ droite ou ramifiée, un cycloalkylène ou arylène; et
où chaque R⁴ est indépendamment un atome d'hydrogène, un groupe alkyle, alcényle, alcynyle, aryle, aralkyle, aralcényle, aralcynyle, cycloalkyle, cycloalkylalkyle, cycloalkylalcényle, hétérocyclyle, hétérocyclylalkyle, hétéroaryle, hétéroarylalkyle.

22. Composition antibiotique selon la revendication 21, dans laquelle la molécule lieuse est -N(R⁴)-R²-N(R⁴)-où chaque R⁴ est un atome d'hydrogène et R² est un hexylène.

23. Composition antibiotique selon l'une quelconque des revendications 1 à 5, dans laquelle:
T est choisi parmi l'un des fragments nisine[1-12] polypeptide suivants: 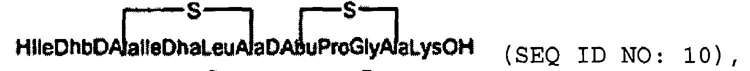 ou 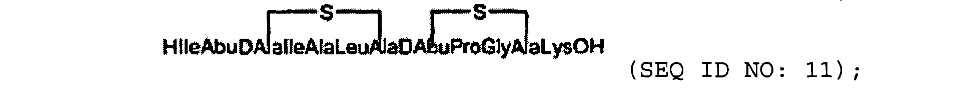
L est -N(R⁴)-R²-N(R⁴)- où chaque R⁴ est un atome d'hydrogène et R² est un hexylène;
et P est la vancomycine ou un dérivé de vancomycine.


### SEQUENCE LISTING

<110> Inflazyme Pharmaceuticals Ltd.
   Coleman, John
   Han, Kang
<120> NOVEL ANTIBIOTIC COMPOSITIONS
<130> 480117.419PC
<140> PCT
   <141> 2007-03-09
<160> 24
<170> FastSEQ for Windows Version 4.0
<210> 1
   <211> 12
   <212> PRT
   <213> Artificial Sequence
<220>
   <223> Synthetic N-terminal peptide fragment of a lantibiotic
<220>
   <221> MOD_RES
   <222> 1
   <223> Xaa = F, I, V or W
<220>
   <221> MOD_RES
   <222> 2
   <223> Xaa = A, K, alpha-aminobutyric acid or dehydrobutyrine
<220>
   <221> MOD_RES
   <222> 4
   <223> Xaa = E, I, K or W
<220>
   <221> MOD_RES
   <222> 5
   <223> Xaa = F, A or dehydroalanine
<220>
   <221> MOD_RES
   <222> 6
   <223> Xaa = I, F or L
<220>
   <221> MOD_RES
   <222> (8)...(8)
   <223> Xaa = alpha-aminobutyric acid
<220>
   <221> MOD_RES
   <222> (12)...(12)
   <223> Xaa = A, K or V
<400> 1
   Xaa Xaa Ala Xaa Xaa Xaa Ala Xaa Pro Gly Ala Xaa
    1               5                   10
<210> 2
   <211> 13
   <212> PRT
   <213> Artificial Sequence
<220>
   <223> Synthetic N-terminal peptide fragment of a lantibiotic
<220>
   <221> MOD_RES
   <222> 1
   <223> Xaa = F, I, V or W
<220>
   <221> MOD_RES
   <222> 2
   <223> Xaa = A, K, alpha-aminobutyric acid or dehydrobutyrine
<220>
   <221> MOD_RES
   <222> 4
   <223> Xaa = E, I, K or W
<220>
   <221> MOD_RES
   <222> 5
   <223> Xaa = F, A or dehydroalanine
<220>
   <221> MOD_RES
   <222> 6
   <223> Xaa = I, F or L
<220>
   <221> MOD_RES
   <222> (8)...(8)
   <223> Xaa = alpha-aminobutyric acid
<220>
   <221> MOD_RES
   <222> (13)...(13)
   <223> Xaa = A, G, I, L, M, P or V
<400> 2
   Xaa Xaa Ala Xaa Xaa Xaa Ala Xaa Pro Gly Ala Ala Xaa
   1 5 10
<210> 3
   <211> 13
   <212> PRT
   <213> Artificial Sequence
<220>
   <223> Synthetic N-terminal peptide fragment of a lantibiotic
<220>
   <221> MOD_RES
   <222> 1
   <223> Xaa = F, I, V or W
<220>
   <221> MOID_RES
   <222> 2
   <223> Xaa = A, K, alpha-aminobutyric acid or dehydrobutyrine
<220>
   <221> MOD_RES
   <222> 4
   <223> Xaa = E, I, K or W
<220>
   <221> MOD_RES
   <222> 5
   <223> Xaa = F, A or dehydroalanine
<220>
   <221> MOD_RES
   <222> 6
   <223> Xaa = I, F or L
<220>
   <221> MOD_RES
   <222> (8)...(8)
   <223> Xaa = alpha-aminobutyric acid
<220>
   <221> MOD_RES
   <222> (13)...(13)
   <223> Xaa = A, G, I, L, M, P or V
<400> 3
   Xaa Xaa Ala Xaa Xaa Xaa Ala Xaa Pro Gly Ala Lys Xaa
   1 5 10
<210> 4
   <211> 13
   <212> PRT
   <213> Artificial Sequence
<220>
   <223> Synthetic N-terminal peptide fragment of a lantibiotic
<220>
   <221> MOD_RES
   <222> 1
   <223> Xaa = F, I, V or W
<220>
   <221> MOD_RES
   <222> 2
   <223> Xaa = A, K, alpha-aminobutyric acid or dehydrobutyrine
<220>
   <221> MOD_RES
   <222> 4
   <223> Xaa = E, I, K or W
<220>
   <221> MOD_RES
   <222> 5
   <223> Xaa = F, A or dehydroalanine
<220>
   <221> MOD_RES
   <222> 6
   <223> Xaa = I, F or L
<220>
   <221> MOD_RES
   <222> (8)...(8)
   <223> Xaa = alpha-aminobutyric acid
<220>
   <221> MOD_RES
   <222> (13)...(13)
   <223> Xaa = A, G, I, L, M, P or V
<400> 4
   Xaa Xaa Ala Xaa Xaa Xaa Ala Xaa Pro Gly Ala Val Xaa
    1               5                   10
<210> 5
   <211> 12
   <212> PRT
   <213> Artificial Sequence
<220>
   <223> Synthetic N-terminal peptide fragment of a lantibiotic
<220>
   <221> MOD_RES
   <222> (1)...(1)
   <223> Xaa = I, L, F, V, A or G
<220>
   <221> MOD_RES
   <222> (2)...(2)
   <223> Xaa = G, A, V, L, I, F, alph-aminobutyric acid or dehydrobutyrine
<220>
   <221> MOD_RES
   <222> (4)...(4)
   <223> Xaa = I, L, F, V, A or G
<220>
   <221> MOD_RES
   <222> (5)...(5)
   <223> Xaa = A, G, V, L, I, F or dehydroalanine
<220>
   <221> MOD_RES
   <222> (6)...(6)
   <223> Xaa = L, I, F, V, A or G
<220>
   <221> MOD_RES
   <222> (8)...(8)
   <223> Xaa = Abu
<220>
   <221> MOD_RES
   <222> (12)...(12)
   <223> Xaa = K, G, A, V, N, Q, R, H
<400> 5
   Xaa Xaa Ala Xaa Xaa Xaa Ala Xaa Pro Gly Ala Xaa
    1               5                   10
<210> 6
   <211> 12
   <212> PRT
   <213> Artificial Sequence
<220>
   <223> Synthetic N-terminal peptide fragment of a lantibiotic
<220>
   <221> MOD_RES
   <222> (1)...(1)
   <223> Xaa = Any natural or non-natural amino acid
<220>
   <221> MOD_RES
   <222> (2)...(2)
   <223> Xaa = Any natural or non-natural amino acid
<220>
   <221> MOD_RES
   <222> (4)...(4)
   <223> Xaa = Any natural or non-natural amino acid
<220>
   <221> MOD_RES
   <222> (5)...(5)
   <223> Xaa = Any natural or non-natural amino acid
<220>
   <221> MOD_RES
   <222> (6)...(6)
   <223> Xaa = Any natural or non-natural amino acid
<220>
   <221> MOD_RES
   <222> (8)...(8)
   <223> Xaa = Abu
<220>
   <221> MOD_RES
   <222> (12)...(12)
   <223> Xaa = Any natural or non-natural amino acid
<400> 6
   Xaa Xaa Ala Xaa Xaa Xaa Ala Xaa Pro Gly Ala Xaa
    1               5                   10
<210> 7
   <211> 12
   <212> PRT
   <213> Artificial Sequence
<220>
   <223> Synthetic N-terminal peptide fragment of a lantibiotic
<220>
   <221> MOD_RES
   <222> (2)...(2)
   <223> Xaa = A, K, Abu or dehydrobutyrine
<220>
   <221> MOD_RES
   <222> (5)...(5)
   <223> Xaa = F, A or dehydroalanine
<220>
   <221> MOD_RES
   <222> (8)...(8)
   <223> Xaa = Abu
<400> 7
   Ile Xaa Ala Ile Xaa Leu Ala Xaa Pro Gly Ala Lys
    1               5                   10
<210> 8
   <211> 5
   <212> PRT
   <213> Artificial Sequence
<220>
   <223> Synthetic N-terminal peptide fragment of a lantibiotic
<220>
   <221> MOD_RES
   <222> (2)...(2)
   <223> D-gamma-glutamic acid
<220>
   <221> MOD_RES
   <222> (4)...(4)
   <223> D-Alanine
<220>
   <221> MOD_RES
   <222> (5)...(5)
   <223> D-Alanine
<400> 8
   Ala Glu Lys Ala Ala
    1               5
<210> 9
   <211> 34
   <212> PRT
   <213> Lactococcus lactis
<220>
   <221> MOD_RES
   <222> (2)...(2)
   <223> Xaa = dehydrobutyrine
<220>
   <221> MOD_RES
   <222> (5)...(5)
   <223> Xaa = dehydroalanine
<220>
   <221> MOD_RES
   <222> (8)...(8)
   <223> Xaa = Abu
<220>
   <221> MOD_RES
   <222> (13)...(13)
   <223> Xaa = Abu
<220>
   <221> MOD_RES
   <222> (23)...(23)
   <223> Xaa = Abu
<220>
   <221> MOD_RES
   <222> (25)...(25)
   <223> Xaa = Abu
<220>
   <221> MOD_RES
   <222> (33)...(33)
   <223> Xaa = dehydroalanine
<400> 9
   Ile Xaa Ala Ile Xaa Leu Ala Xaa Pro Gly Ala Lys Xaa Gly Ala Leu
    1               5                  10                  15
   Met Gly Ala Asn Met Lys Xaa Ala Xaa Ala His Ala Ser Ile His Val
               20                  25                  30
   Xaa Lys
<210> 10
   <211> 12
   <212> PRT
   <213> Lactococcus lactis
<220>
   <221> MOD_RES
   <222> (2)...(2)
   <223> Xaa = dehydrobutyrine
<220>
   <221> MOD_RES
   <222> (3)...(3)
   <223> D-Alanine
<220>
   <221> MOD_RES
   <222> (5)...(5)
   <223> Xaa = dehydroalanine
<220>
   <221> MOD_RES
   <222> (8)...(8)
   <223> Xaa = D-Abu
<400> 10
   Ile Xaa Ala Ile Xaa Leu Ala Xaa Pro Gly Ala Lys
    1               5                  10
<210> 11
   <211> 12
   <212> PRT
   <213> Artificial Sequence
<220>
   <223> Synthetic N-terminal peptide fragment of a lantibiotic
<220>
   <221> MOD_RES
   <222> (2)...(2)
   <223> Xaa = Abu
<220>
   <221> MOD_RES
   <222> (3)...(3)
   <223> D-Alanine
<220>
   <221> MOD_RES
   <222> (8)...(8)
   <223> Xaa = D-Abu
<400> 11
   Ile Xaa Ala Ile Ala Leu Ala Xaa Pro Gly Ala Lys
    1               5                   10
<210> 12
   <211> 5
   <212> PRT
   <213> Artificial Sequence
<220>
   <223> Synthetic N-terminal peptide fragment of a lantibiotic
<220>
   <221> MOD_RES
   <222> (1)...(1)
   <223> D-Alanine
<220>
   <221> MOD_RES
   <222> (3)...(3)
   <223> Xaa = Dpr
<400> 12
   Ala Ile Xaa Leu Ala
    1               5
<210> 13
   <211> 5
   <212> PRT
   <213> Artificial sequence
<220>
   <223> Synthetic N-terminal peptide fragment of a lantibiotic
<220>
   <221> MOD_RES
   <222> (1)...(1)
   <223> D-Alanine
<220>
   <221> MOD_RES
   <222> (3)...(3)
   <223> Xaa = dehydroalanine
<400> 13
   Ala Ile Xaa Leu Ala
   1 5
<210> 14
   <211> 7
   <212> PRT
   <213> Artificial Sequence
<220>
   <223> Synthetic N-terminal peptide fragment of a lantibiotic
<220>
   <221> MOD_RES
   <222> (3)...(3)
   <223> D-Alanine
<220>
   <221> MOD_RES
   <222> (5)...(5)
   <223> Xaa = dehydroalanine
<400> 14
   Ile Thr Ala Ile Xaa Leu Ala
   1 5
<210> 15
   <211> 7
   <212> PRT
   <213> Artificial Sequence
<220>
   <223> Synthetic N-terminal peptide fragment of a lantibiotic
<220>
   <221> MOD_RES
   <222> (2)...(2)
   <223> Xaa = dehydrobutyrine
<220>
   <221> MOD_RES
   <222> (3)...(3)
   <223> D-Alanine
<220>
   <221> MOD_RES
   <222> (5)...(5)
   <223> Xaa = dehydroalanine
<400> 15
   Ile Xaa Ala Ile Xaa Leu Ala
   1 5
<210> 16
   <211> 4
   <212> PRT
   <213> Artificial Sequence
<220>
   <223> Synthetic N-terminal peptide fragment of a lantibiotic
<220>
   <221> MOD_RES
   <222> (2)...(2)
   <223> Xaa = Dpr with carbobenzyloxy modification on side chain
<220>
   <221> MOD_RES
   <222> (4)...(4)
   <223> acetamidomethyl modificiation on side chain
<400> 16
   Ile Xaa Leu Cys
   1
<210> 17
   <211> 5
   <212> PRT
   <213> Artificial Sequence
<220>
   <223> Synthetic N-terminal peptide fragment of a lantibiotic
<220>
   <221> MOD_RES
   <222> (1)...(1)
   <223> D-Cysteine with trityl modification on side chain
<220>
   <221> MOD_RES
   <222> (3)...(3)
   <223> Xaa = Dpr with carbobenzyloxy modification on side chain
<220>
   <221> MOD_RES
   <222> (5)...(5)
   <223> Xaa = acetamidomethyl modification on side chain
<400> 17
   Cys Ile Xaa Leu Cys
   1 5
<210> 18
   <211> 4
   <212> PRT
   <213> Artificial Sequence
<220>
   <223> Synthetic N-terntinal peptide fragment of a lantibiotic
<220>
   <221> MOD_RES
   <222> (1)...(1)
   <223> D-Cysteine with trityl modification to side chain
<220>
   <221> MOD_RES
   <222> (4)...(4)
   <223> acetaminomethyl modification to side chain
<400> 18
   Cys Pro Gly Cys
    1
<210> 19
   <211> 4
   <212> PRT
   <213> Artificial Sequence
<220>
   <223> Synthetic N-terminal peptide fragment of a lantibiotic
<220>
   <221> MOD_RES
   <222> (1)...(1)
   <223> Xaa = D-Abu
<400> 19
   Xaa Pro Gly Ala
    1
<210> 20
   <211> 5
   <212> PRT
   <213> Artificial Sequence
<220>
   <223> Synthetic N-terminal peptide fragment of a lantibiotic
<220>
   <221> MOD_RES
   <222> (1)...(1)
   <223> Xaa = D-Abu
<220>
   <221> MOD_RES
   <222> (5)...(5)
   <223> carbobenzyloxy modification to side chain
<400> 20
   Xaa Pro Gly Ala Lys
    1               5
<210> 21
   <211> 5
   <212> PRT
   <213> Artificial Sequence
<220>
   <223> Synthetic N-terminal peptide fragment of a lantibiotic
<220>
   <221> MOD_RES
   <222> (1)...(1)
   <223> D-Alanine
<400> 21
   Ala Ile Ala Leu Ala
    1               5
<210> 22
   <211> 7
   <212> PRT
   <213> Artificial Sequence
<220>
   <223> Synthetic N-terminal peptide fragment of a lantibiotic
<220>
   <221> MOD_RES
   <222> (2)...(2)
   <223> Xaa = Abu
<220>
   <221> MOD_RES
   <222> (3)...(3)
   <223> D-Alanine
<400> 22
   Ile Xaa Ala Ile Ala Leu Ala
    1               5
<210> 23
   <211> 4
   <212> PRT
   <213> Artificial Sequence
<220>
   <223> Synthetic N-terminal peptide fragment of a lantibiotic
<220>
   <221> MOD_RES
   <222> (0)...(0)
   <223> acetamidomethyl modification to side chain
<400> 23
   Ile Ala Leu Cys
    1
<210> 24
   <211> 5
   <212> PRT
   <213> Artificial Sequence
<220>
   <223> Synthetic N-terminal peptide fragment of a lantibiotic
<220>
   <221> MOD_RES
   <222> (1)...(1)
   <223> D-Cysteine with trityl modification to side chain
<220>
   <221> MOD_RES
   <222> (5)...(5)
   <223> acetamidomethyl modification to side chain
<400> 24
   Cys Ile Ala Leu Cys
    1               5